(19) Europäisches Patentamt
European Patent Office
Office européen des brevets

(11) Veröffentlichungsnummer : **0 119 955**
**B1**

(12) **EUROPÄISCHE PATENTSCHRIFT**

(45) Veröffentlichungstag der Patentschrift : **13.12.89**

(51) Int. Cl.[4] : **C 07 D223/16**, A 61 K 37/02, A 61 K 37/64

(21) Anmeldenummer : **84810072.3**

(22) Anmeldetag : **06.02.84**

(54) **Gewisse 3-(5-Aminopentyl)-amino-1-benzazepin-2-on-1-alkansäuren, Verfahren zu ihrer Herstellung, pharmazeutische Präparate enthaltend diese Verbindungen, sowie ihre therapeutische Verwendung.**

(30) Priorität : **10.02.83 US 465695**

(43) Veröffentlichungstag der Anmeldung : **26.09.84 Patentblatt 84/39**

(45) Bekanntmachung des Hinweises auf die Patenterteilung : **13.12.89 Patentblatt 89/50**

(84) Benannte Vertragsstaaten : **AT BE CH DE FR GB IT LI LU NL SE**

(56) Entgegenhaltungen :
**EP-A- 0 046 291**
**EP-A- 0 072 352**
**EP-A- 0 107 095**
**CHEMICAL ABSTRACTS, Band 100, 1984, Seite 19, ref. nr. 167718k, Columbus, Ohio, US. W.H. PARSONS et al.: "Benzolactams. A new class of converting enzyme inhibitors", & Biochem. Biophys. Res. Commun. 1983, 117(1), 108-13**

(73) Patentinhaber : **CIBA-GEIGY AG**
**Klybeckstrasse 141**
**CH-4002 Basel (CH)**

(72) Erfinder : **Watthey, Jeffrey W.H., Dr.**
**61 Deepwood Drive**
**Chappaqua New York 10514 (US)**
Erfinder : **Boyer, Stephen K.**
**Rd. 1, Box 204**
**Far Hills, New Jersey 07931 (US)**
Erfinder : **Bach, Joseph**
**Argyle Court**
**Parsippany, New Jersey 07054 (US)**
Erfinder : **Sedelmeier, Gottfried, Dr.**
**Erlenweg 11**
**D-7801 Schallstadt (DE)**

**EP 0 119 955 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft neue 3-(5-Aminopentyl)-amino-1-benzazepin-2-on-1-alkansäuren der allgemeinen Formel

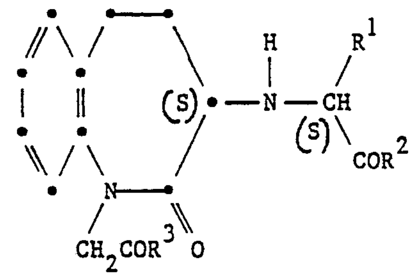

(I)

worin $R^1$ 4-Aminobutyl bedeutet, $R^2$ und $R^3$, unabhängig voneinander, Hydroxy oder Niederalkoxy bedeuten und S die Chiralität bedeutet, Salze, insbesondere pharmazeutisch annehmbare Salze davon, Verfahren zur Herstellung dieser Verbindungen, pharmazeutische Präparate enthaltend diese Verbindungen und ihre therapeutische Verwendung.

Die vorliegend verwendeten allgemeinen Definitionen besitzen im Bereich der Erfindung die folgenden Bedeutungen :

Die Bezeichnung « nieder », die vorstehend und nachfolgend in Verbindung mit organischen Resten bzw. Verbindungen verwendet wird, definiert solche mit bis zu und einschliesslich 7, vorzugsweise bis zu und einschliesslich 4 und insbesondere 1 oder 2 Kohlenstoffatomen.

Niederalkyl enthält 1-7 Kohlenstoffatome, vorzugsweise 1 bis 4 Kohlenstoffatome und bedeutet z. B. Ethyl, Propyl, Butyl oder insbesondere Methyl.

Aryl bedeutet einen carbocyclischen oder heterocyclischen aromatischen Rest, vorzugsweise unsubstituiertes oder durch Niederalkyl, Niederalkoxy, Niederalkylendioxy, Niederalkanoyloxy, Hydroxy, Halogen oder Trifluormethyl mono- oder disubstituiertes Phenyl.

Cycloalkyl bedeutet einen gesättigten cyclischen Kohlenwasserstoffrest, der vorzugsweise 3 bis 8 Kohlenstoffatome enthält und beispielsweise Cyclopentyl oder Cyclohexyl ist.

Arylniederalkyl bedeutet vorzugsweise Benzyl, 1- oder 2-Phenylethyl, 1-, 2- oder 3-Phenylpropyl, 1-, 2-, 3- oder 4-Phenylbutyl, worin der Phenylring unsubstituiert oder durch Niederalkyl, Hydroxy, Niederalkoxy, Niederalkylendioxy, Niederalkanoyloxy, Halogen oder Trifluormethyl mono- oder disubstituiert ist.

Niederalkoxy enthält vorzugsweise 1 bis 4 Kohlenstoffatome und ist beispielsweise Methoxy, Propoxy, Isopropoxy oder insbesondere Ethoxy.

Niederalkanoyloxy bedeutet vorzugsweise Acetoxy, Propionyloxy oder Pivaloyloxy.

Niederalkylendioxy bedeutet vorzugsweise Ethylendioxy und insbesondere Methylendioxy.

Halogen bedeutet vorzugsweise Chlor, kann jedoch auch Brom, Fluor oder Iod sein.

Acyliertes Hydroxy bedeutet vorzugsweise Niederalkanoyloxy, z. B. Acetyloxy, Benzoyloxy, am Phenylring durch Niederalkyl, Halogen oder Niederalkoxy, wie durch Methyl, Chlor bzw. Methoxy substituiertes Benzoyloxy, oder Nicotinoyloxy.

Verethertes Hydroxy bedeutet vorzugsweise Niederalkoxy, z. B. Methoxy, Ethoxy oder t-Butoxy, oder Benzyloxy.

Trialkoxymethyl bedeutet vorzugsweise Tri-niederalkoxy-methyl, insbesondere Triethoxy- oder Trimethoxymethyl.

Verethertes Hydroxymethyl bedeutet vorzugsweise tertiäres Niederalkoxymethyl, Niederalkoxy-niederalkoxymethyl wie Methoxymethoxymethyl, 2-Oxa- oder 2-Thiacycloalkoxymethyl, insbesondere 2-Tetrahydropyranyloxymethyl.

Die Salze von Verbindungen der Formel I sind von solchen Verbindungen abgeleitet, die salzbildende Eigenschaften haben. Bevorzugt sind die pharmazeutisch verträglichen Salze.

Pharmazeutisch verträgliche Salze sind vorzugsweise Metall- oder Ammoniumsalze der Verbindungen der Formel I, worin $COR^2$ und/oder $COR^3$ Carboxy bedeuten, insbesondere Alkali- oder Erdalkalimetallsalze, z. B. Natrium-, Kalium-, Magnesium- oder Calciumsalze ; oder vorteilhafterweise leicht kristallisierende Ammoniumsalze, abgeleitet von Ammoniak oder organischen Aminen, wie Mono-, Di- oder Tri-nieder(alkyl, cycloalkyl oder hydroxyalkyl)aminen, Niederalkylendiaminen oder (Hydroxyniederalkyl oder Arylniederalkyl)alkylammonium-Basen, z. B. Methylamin, Diethylamin, Triethylamin, Dicyclohexylamin, Triethanolamin, Ethylendiamin, Tris-(hydroxymethyl)aminomethan oder Benzyltrimethylammoniumhydroxid. Diese Verbindungen der Formel I bilden Säureadditionssalze, bei denen es sich vorzugsweise um solche von therapeutisch verträglichen anorganischen oder organischen Säuren handelt, wie starken Mineralsäuren, z. B. Halogenwasserstoffsäuren, z. B. Chlorwasserstoff- oder Bromwasserstoffsäure ; Schwefel-, Phosphor-, Salpeter- oder Perchlorsäure ; aliphatischen oder aromatischen Carbon- oder Sulfonsäuren, z. B. Ameisen-, Essig-, Propion-, Bernstein-, Glykol-, Milch-, Aepfel-, Wein-, Glucon-, Citronen-, Ascorbin-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoa-, Nicotin-, Methansulfon-, Ethansulfon-, Hydroxyethansulfon-, Benzolsulfon-, p-Toluolsulfon-, Naphthalinsulfon-, Sulfanil- oder Cyclohexylsulfaminsäure.

Die Verbindungen der Formel I zeigen wertvolle pharmakologische Eigenschaften, z. B. kardiovaskuläre Effekte, welche sie unter anderem aufgrund ihrer Hemmwirkung auf die Bildung von Angiotensin II hervorrufen. Diese Wirkung entsteht durch selektive Hemmung des Enzyms, welches das Angiotensin in Säugern umwandelt. Die Verbindungen sind daher nützlich für die Behandlung von Krankheiten, welche auf die Hemmung des Enzyms, das Angiotensin in Säugern, einschliesslich Menschen, umwandelt, ansprechen.

Die Verbindungen der Erfindung zeigen in erster Linie hypotensive/antihypertensive Wirkungen und beeinflussen die Herztätigkeit, u. a. aufgrund ihrer hemmenden Wirkung auf das Enzym, welches das Angiotensin umwandelt. Diese Eigenschaften können durch in-vitro- oder in-vivo-Tierversuche, vorzugsweise an Säugetieren, z. B. Ratten, Katzen, Hunden oder deren isolierten Organen, als Testobjekte nachgewiesen werden. Die Tiere können entweder normotensiv oder hypertensiv, z. B. genetisch spontan hypertensive Ratten, oder renal hypertensive Ratten oder Hunde, und Hunde, denen Natrium entzogen ist, sein. Die Verbindungen können den Versuchstieren enteral oder parenteral, vorzugsweise oral oder intravenös, z. B. in Gelatine-Kapseln oder in Form von Stärke enthaltenden Suspensionen bzw. wässrigen Lösungen, verabreicht werden. Die verwendete Dosis kann in einem Bereich von ungefähr zwischen 0,001 und 30 mg/kg/Tag, vorzugsweise zwischen ungefähr 0,01 und 10 mg/kg/Tag liegen.

Die blutdrucksenkende Wirkung in vivo wird entweder direkt mit einem Katheter, der in die femurale Arterie des Versuchstieres eingeführt ist, oder indirekt durch Sphygmomanometrie am Rattenschwanz, und einem Uebertragungsinstrument registriert. Der Blutdruck wird vor und nach der Verabreichung des Wirkstoffes in mm Hg bestimmt.

So sind die antihypertensiven Wirkungen in spontan hypertensiven Ratten durch indirekte Messung des systolischen Blutdrucks nachweisbar. Wache Ratten werden einzeln in sehr engen Käfigen in eine leicht geheizte Kammer gebracht. Ein Puls-Sensor wird innerhalb einer aufblasbaren Manschette am Schwanz jeder Ratte angebracht. Die Manschette wird periodisch aufgeblasen, um die Schwanz-Arterie zu schliessen. Der Druck in der Manschette wird kontinuierlich reduziert, und der systolische Druck entspricht demjenigen Druck in der Manschette, bei dem die Pulswellen wieder auftreten. Nach der Registrierung von Kontrollwerten des Blutdrucks und der Herzfrequenz werden die Testverbindungen einmal täglich an vier aufeinander folgenden Tagen oral verabreicht. Zusätzliche Blutdruckmessungen werden üblicherweise 2,0, 4,0 und 23,5 Stunden nach einer täglichen Dosis vorgenommen. Die Werte werden mit denjenigen von Ratten, welche lediglich mit der Trägersubstanz behandelt worden sind, verglichen.

Die Verbindungen der Erfindung zeigen nach intravenöser oder oraler Verabreichung eine hemmende Wirkung gegen die durch Angiotensin I hervorgerufene Blutdrucksteigerung auch an normotensiven Ratten. Das Angiotensin I wird durch das genannte Umwandlungsenzym zu der stark blutdrucksteigernden Substanz Angiotensin II hydrolysiert. Die Hemmung des genannten Enzyms blockiert die Bildung von Angiotensin II aus Angiotensin I. Auf diese Weise wird die durch Angiotensin I hervorgerufene Blutdrucksteigerung abgeschwächt.

Der entsprechende Test in vivo wird mit männlichen normotensiven Ratten, welche mit dem Natriumsalz der 5-Ethyl-5-(1-methyl-propyl)-2-thiobarbitursäure narkotisiert sind, durchgeführt. Eine femurale Arterie und eine Wadenvene werden mit je einer Hohlnadel für die direkte Messung des Blutdrucks und für die intravenöse Verabreichung des Angiotensins I und einer erfindungsgemässen Verbindung versehen. Nach der Stabilisierung des basalen Blutdrucks wird die Druckänderung nach drei, in 5-minütigen Intervallen, i. v. durchgeführten Reizungen mit 333 ng/kg Angiotension I bestimmt.

Solche Druckänderungen werden wieder 5, 10, 15, 30 und 60 Minuten nach intravenöser Verabreichung oder 1, 2, 3 und 4 Stunden nach peroraler Verabreichung der zu prüfenden Verbindung bestimmt und mit den Anfangswerten verglichen. Jede durch die erfindungsgemässen Verbindungen hervorgerufene festgestellte Abnahme vom Ansprechen des Blutdrucks ist ein Hinweis auf die Hemmung des Enzyms, welches das Angiotensin I umwandelt.

Die Hemmung des Enzyms, welches das Angiotensin umwandelt, durch die Verbindungen dieser Erfindung in vitro kann analog zu Biochim. Biophys. Acta 293, 451 (1973) nachgewiesen werden. Gemäss dieser Methode werden die genannten Verbindungen in ungefähr 1 mMol-Konzentrationen in Phosphatpuffer gelöst. Zu 100 µl von Lösungen der Testverbindung in Phosphatpuffer, welche man auf die gewünschte Konzentration verdünnt, gibt man zuerst 100 µl von 5 millimolarem Hippuryl-histidyl-leucin in Phosphatpuffer und dann 50 µl des Angiotensin-umwandelnden Enzyms. Dieses Enzym wird aus Lungen von erwachsenen männlichen Kaninchen in Tris-Puffer, der Kalium- und Magnesiumchlorid und auch Rohrzucker enthält, hergestellt. Die genannten Lösungen werden 30 Minuten bei 37 °C inkubiert. Durch Hinzufügen von 0,75 ml einer 0,6-normalen wässrigen Natriumhydroxidlösung wird die Reaktion dann abgebrochen. Man gibt bei Zimmertemperatur 100 µl einer 0,2 %igen Lösung von o-Phthalaldehyd in Methanol und 10 Minuten danach 100 µl 6n-Chlorwasserstoffsäure dazu. Diese Proben werden in einem Spektrophotometer bei 360 nm mit Wasser verglichen, und ihre optische Dichte wird bestimmt. Diese Werte werden durch einen Konversionsfaktor einer Standard-Kurve angepasst, wobei man die während der genannten Inkubation von 30 Minuten gebildete Histidyl-leucin-Menge in Nanomolen erhält. Die

Ergebnisse werden, um dem $IC_{50}$-Wert zu bestimmen, gegenüber den Wirkstoffkonzentrationen graphisch als Kurve dargestellt. Der $IC_{50}$-Wert bedeutet diejenige Wirkstoff-Konzentration, welche die Hälfte der Aktivität einer Kontrollprobe, die keinen Wirkstoff enthält, ergibt.

Wegen der vorgenannten vorteilhaften Eigenschaften sind die erfindungsgemässen Verbindungen als spezifische therapeutische Mittel für Säuger, inklusive Menschen, sehr nützlich.

Dementsprechend sind die Verbindungen der Erfindung wertvolle antihypertensive Mittel, insbesondere für die Herabsetzung von hohem Blutdruck (ohne Rücksicht auf die Aetiologie) und/oder bei Herzerkrankungen wie Herzinsuffizienz, und/oder anderen Zuständen, die mit Oedemen oder Ascites einhergehen. Sie können auch zur Herstellung von anderen wertvollen Produkten, insbesondere von entsprechenden pharmazeutischen Präparaten verwendet werden.

Insbesondere betrifft die Erfindung Verbindungen der Formel I, worin $R^1$ 4-Aminobutyl bedeutet, und $R^2$ und $R^3$ unabhängig voneinander Hydroxy oder Niederalkoxy wie Methoxy, Ethoxy, Propoxy, Isopropoxy, Butoxy oder tert.-Butoxy bedeuten, und pharmazeutisch annehmbare Salze davon.

Bevorzugt sind Verbindungen der Formel I, worin $R^1$ 4-Aminobutyl bedeutet, und $R^2$ und $R^3$ unabhängig voneinander Hydroxy, Methoxy, Ethoxy oder tert.-Butoxy bedeuten, und pharmazeutisch annehmbare Salze davon.

Besonders bevorzugt sind Verbindungen der Formel I, worin $R^1$ 4-Aminobutyl bedeutet, $R^2$ Hydroxy, Methoxy oder tert.-Butoxy und $R^3$ Hydroxy oder Ethoxy bedeuten, und pharmazeutisch annehmbare Salze, insbesondere die Hydrohalogenide und Dihydrohalogenide davon.

Besonders bevorzugt sind Verbindungen der Formel I, worin $R^1$ 4-Aminobutyl bedeutet, $R^2$ Hydroxy, Methoxy oder tert.-Butoxy bedeutet, wenn $R^3$ Hydroxy ist, und $R^2$ Hydroxy oder Methoxy bedeutet, wenn $R^3$ Ethoxy ist und die Hydrochloride und die Dihydrochloride davon.

Herausragend sind 3-[(5-Amino-1-carboxy)-(1S)-pentylamino]-1-carboxymethyl-2,3,4,5-tetrahydro-1H-1-(3S)-benzazepin-2-on und seine pharmazeutisch annehmbaren Salze.

Die Verbindungen der Formel I gemäss der Erfindung können in an sich bekannter Weise hergestellt werden, indem man z. B.

a) eine Verbindung der Formel

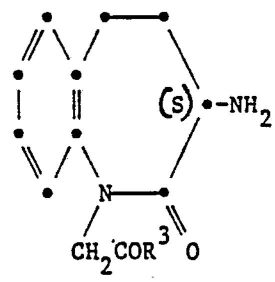

(II)

als S-Enantiomer oder Mischung von Stereoisomeren, die dieses Enantiomer enthält, worin $R^3$ die vorstehend angegebenen Bedeutungen besitzt, mit einer Verbindung der Formel

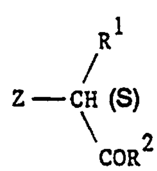

(IIIA)

als S-Enantiomer oder Mischung von Stereoisomeren, die dieses Enantiomer enthält, worin Z eine reaktive veresterte Hydroxygruppe bedeutet und $R^1$ und $R^2$ die vorstehend angegebenen Bedeutungen besitzen, oder mit einer Verbindung der Formel

$$R^1—CO—COR^2 \quad (IV)$$

worin $R^1$ und $R^2$ die oben angegebenen Bedeutungen haben, in Gegenwart eines Reduktionsmittels, unter vorübergehendem Schutz der Aminogruppe und/oder von Hydroxygruppen, die in irgendeinem der Reste $R^1$, $R^2$ und $R^3$ anwesend sein können, alkyliert oder

b) eine Verbindung der Formel

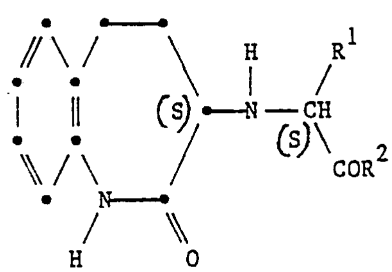

(V)

oder eine Mischung von Stereoisomeren, die diese Verbindung enthält, worin S, $R^1$ und $R^2$ die vorstehend angegebenen Bedeutungen besitzen, mit einer Verbindung der Formel

$$Z{-}CH_2COR^3 \qquad \text{(IIIB)},$$

worin Z eine reaktive veresterte Hydroxygruppe bedeutet und $R^3$ die vorstehend angegebenen Bedeutungen besitzt, alkyliert, wobei man vorzugsweise vorübergehend die Aminogruppe und/oder Hydroxygruppen, die in irgendeinem der Reaktanten anwesend sein können, schützt, oder

c) eine Verbindung der Formel

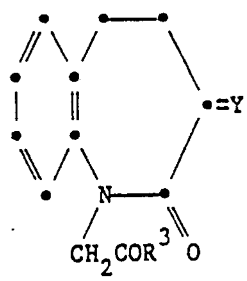

(VI)

worin Y Oxo, eine reaktive veresterte oder veretherte Hydroxygruppe Z gemeinsam mit Wasserstoff oder zwei reaktive veresterte oder veretherte Hydroxygruppen Z bedeutet und $R^3$ die vorstehend angegebenen Bedeutungen besitzt, mit einem Amin der Formel

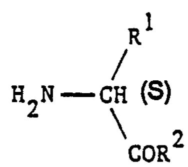

(VII)

als S-Enantiomer oder Mischung von Stereoisomeren, die dieses Enantiomer enthält, worin $R^1$ und $R^2$ die vorstehend angegebenen Bedeutungen besitzen, kondensiert, wobei, wenn Y Oxo oder zwei veresterte oder veretherte Hydroxygruppen bedeutet, die Kondensation in Gegenwart eines Reduktionsmittels durchgeführt wird, oder

d) in einer Verbindung der Formel

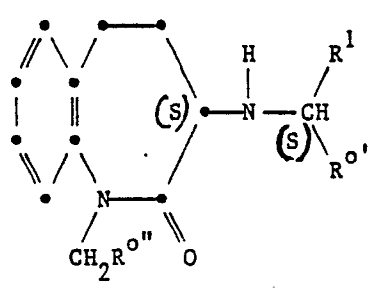

(VIII)

oder in einer Mischung von Stereoisomeren, die diese Verbindung enthält, worin S und $R^1$ die vorstehend angegebenen Bedeutungen besitzen, eines der Symbole $R^{0'}$ und $R^{0''}$ Cyano ist und das andere Cyano oder $COR^2$ bzw. $COR^3$ wie vorstehend definiert ist, die Cyanogruppe(n) einer Solvolyse unterzieht oder

e) eine Verbindung der Formel

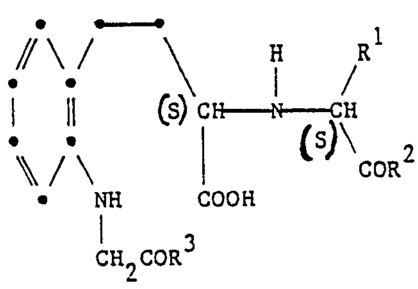

(IX)

oder eine Mischung von Stereoisomeren, die diese Verbindung enthält, worin S, $R^1$, $R^2$ und $R^3$ die vorstehend angegebenen Bedeutungen besitzen, oder einen reaktiven Ester hiervon cyclisiert oder

f) eine oder zwei Doppelbindungen in einer Verbindung der Formel

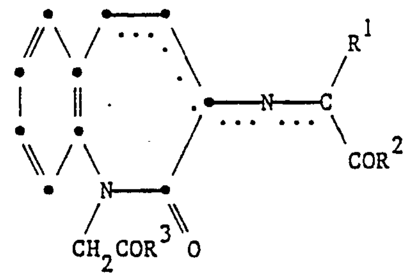

(X)

worin diese Doppelbindung(en) sich zwischen C3 und C4, C4 und C5 und/oder in der Seitenkette zwischen dem Stickstoffatom und einem benachbarten Kohlenstoffatom befinden, und worin $R^1$, $R^2$ und $R^3$ die vorstehend angegebenen Bedeutungen besitzen, durch Behandlung mit einem Reduktionsmittel sättigt oder

g) das monocyclische Lactam in einer Verbindung der Formel

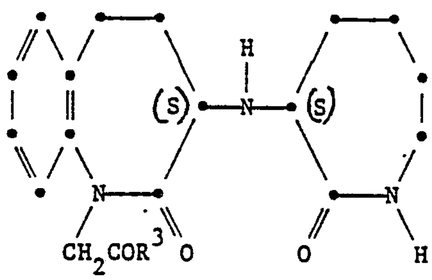

(XI)

oder in einer Mischung von Stereoisomeren, die diese Verbindung enthält, worin S und $R^3$ die vorstehend angegebenen Bedeutungen besitzen, zu einer Verbindung der Formel I, worin $R^2$ und $R^3$ Hydroxy bedeuten, ringöffnet oder

h) eine Verbindung der Formel

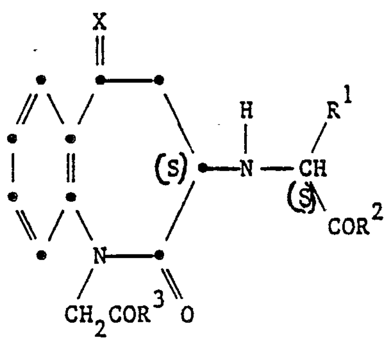

(XII)

oder eine Mischung von Stereoisomeren, die diese Verbindung enthält, worin X Oxo, geschütztes Oxo oder zwei veresterte oder veretherte Hydroxygruppen bedeutet, oder worin X Hydroxy oder verestertes oder verethertes Hydroxy zusammen mit einem Wasserstoffatom bedeutet, und worin S, $R^1$, $R^2$ und $R^3$ die vorstehend angegebenen Bedeutungen haben, hydrogenolysiert oder reduziert oder

i) eine Verbindung der Formel

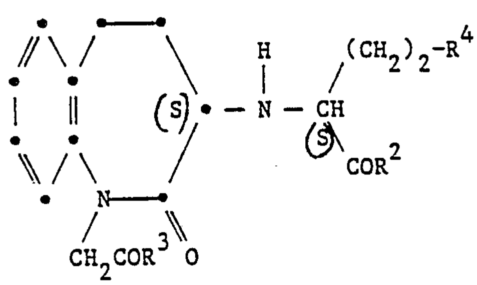

(XIII)

oder eine Mischung von Stereoisomeren, die diese Verbindung enthält, worin $R^4$ eine in 2-Aminoethyl umwandelbare Gruppe bedeutet, und $R^2$ und $R^3$ die vorstehend angegebenen Bedeutungen besitzen, in eine Verbindung der Formel I umwandelt oder

j) eine Verbindung der Formel

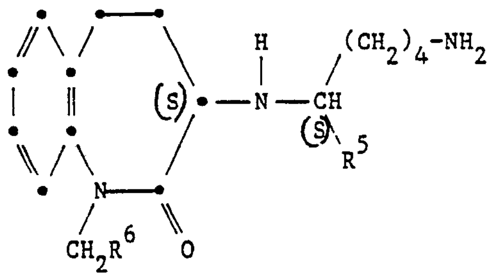

(XIV)

oder eine Mischung von Isomeren, die diese Verbindung enthält, worin $R^5$ oder $R^6$ eine in $COR^2$ bzw. $COR^3$ umwandelbare Gruppe bedeutet, und die andere $COR^2$ bzw. $COR^3$ bedeutet, oder worin $R^5$ und $R^6$ in $COR^2$ und $COR^3$ umwandelbare Gruppen bedeuten, und worin S die vorstehend angegebene Bedeutung hat, in eine Verbindung der Formel I umwandelt oder

k) in einer Verbindung der Formel

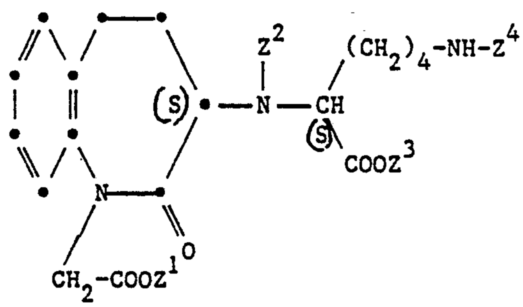

(XV)

oder einer Mischung von Stereoisomeren, die diese Verbindung enthält, worin zumindest eine der Gruppen $Z^1$ bis $Z^4$ eine Schutzgruppe, verbleibende Gruppen $Z^2$ und $Z^4$ Wasserstoff und verbleibende Gruppen $Z^1$ und $Z^3$ Wasserstoff oder Niederalkyl bedeuten, mit der Maßgabe, daß mindestens eine Gruppe von verbleibenden Gruppen $Z^1$ und $Z^3$ Wasserstoff bedeutet, und worin S die vorstehend angegebene Bedeutung hat, um eine Verbindung der Formel I zu erhalten, worin einer oder beide Rest $R^2$ und $R^3$ Hydroxy bedeuten, die Schutzgruppe(n) entfernt, und gewünschtenfalls eine erhaltene Verbindung der Formel I in eine andere Verbindung der Formel I umwandelt und/oder gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt und/oder gewünschtenfalls das optische Isomere der Formel I, das die S,S-Konfiguration im Hinblick auf die beiden Chiralitätszentren besitzt, aus einer Mischung der stereoisomeren Formen, die eine Verbindung der Formel I enthält, isoliert.

Alle Verfahren zur Herstellung einer Verbindung der Formel I werden vorteilhafterweise unter vorübergehendem Schutz von reaktiven funktionellen Gruppen durchgeführt, jenachdem wie es sich für einen Fachmann nach den jeweiligen Umständen bei einem beliebigen Verfahrensschritt als notwendig erweist.

Eine reaktive veresterte Hydroxygruppe Z ist eine mit einer starken organischen Säure veresterte Hydroxygruppe, z. B. einer aliphatischen oder aromatischen Sulfonsäure (wie einer Niederalkansulfonsäure, insbesondere Methansulfonsäure, Trifluormethansulfonsäure, insbesondere Benzolsulfonsäure, p-Toluolsulfonsäure, p-Brombenzolsulfonsäure und p-Nitrobenzolsulfonsäure) oder mit einer starken anorganischen Säure, wie insbesondere Schwefelsäure, oder einer Halogenwasserstoffsäure, wie Chlorwasserstoffsäure oder am meisten bevorzugt lodwasserstoffsäure oder Bromwasserstoffsäure, veresterte Hydroxygruppe.

Eine substitutive Alkylierung gemäss der vorliegenden Erfindung wird unter herkömmlichen allgemeinen Bedingungen bei Temperaturen im Bereich von etwa 0 °C bis zur Siedetemperatur der Reaktionsmischung, vorzugsweise bei Temperaturen zwischen Raumtemperatur und etwa 100 °C, durchgeführt. Die Umsetzung findet vorteilhaft in Gegenwart eines Lösungsmittels, das im Hinnblick auf die Reaktanten inert ist, statt, wie in Gegenwart eines chlorierten Niederalkans (z. B. Chloroform oder Methylenchlorid), eines acyclischen oder cyclischen Ethers (z. B. Diethylether, 1,2-Dimethoxyethan, Dioxan oder Tetrahydrofuran) und insbesondere eines tertiären Amids mit niedrigem Molekulargewicht (z. B. N,N-Dimethylformamid, N,N-Dimethylacetamid, N-Methylpyrrolidon, N-Ethylpiperidon und Hexamethylphosphorsäuretrisamid). Vorteilhafterweise wird die während der Reaktion freigesetzte starke

Säure HZ durch Zugabe eines säurebindenden Mittels gebunden, wie vorzugsweise eines anorganischen Säurefängers, wie eines Alkalimetallbicarbonats, -carbonats oder -hydroxids, eines organischen quaternären Ammoniumsalzes (z. B. eines Tetrabutylammoniumsalzes) oder einer organischen tertiären Base, wie Triethylamin, N-Ethylpiperidin, Pyridin oder Chinolin.

Eine erfindungsgemässe Alkylierung kann auch unter an sich bekannten und im Stand der Technik verwendeten Bedingungen einer reduktiven Alkylierung durchgeführt werden. Bei Durchführung dieser Alkylierung werden die Ausgansstoffe gleichzeitig oder in einem nachfolgenden Schritt mit einem Reduktionsmittel umgesetzt. Unter Reduktionsmitteln, die gleichzeitig mit dem Alkylierungsmittel verwendet werden, sollen Ameisensäure und komplexe Metallhydride, wie Natriumcyanborhydrid, erwähnt werden; unter den Reduktionsmitteln, die überwiegend in einer getrennten nachfolgenden Arbeitsstufe, d. h. zur Reduktion eines vorher gebildeten Imins (Schiff'sche Base) verwendet werden, sollen Diboran und komplexe Metallhydride, wie Natriumborhydrid und Natriumcyanborhydrid, erwähnt werden, die vorteilhaft dem primären Reaktionsgemisch ohne Isolierung eines Zwischenprodukts, z. B. des Imins, zugegeben werden. In diesem Fall wird die Alkylierung vorteilhaft in einem gegenüber dem Reduktionsmittel inerten organischen Lösungsmittel durchgeführt, wie in einem aliphatischen oder cyclischen Ether (wie Diethylether, Diisopropylether, 1,2-Dimethoxyethan, Dioxan oder Tetrahydrofuran) oder in einem aliphatischen Alkohol (wie Methanol, Ethanol, Isopropylalkohol, Glykol, Glykolmonomethylether oder Diethylenglykol), vorzugsweise bei etwa 0 bis 80 °C. Ein wesentliches Reduktionsmittel, das sowohl gleichzeitig als auch anschliessend verwendet werden kann, ist Wasserstoff, insbesondere katalytisch aktivierter Wasserstoff. Die Katalysatoren sind diejenigen, die herkömmlich als Hydrierungskatalysatoren eingesetzt werden, vorzugsweise diejenigen der Klasse der Edelmetalle (wie Palladium, Platin und Rhodium) auf einem Träger (wie Calciumcarbonat, Aluminiumoxid oder Bariumsulfat), in feindisperser Suspension ohne Träger oder in Form von Komplexen in homogener Phase. Auch feindispergierte Uebergangsmettale, z. B. Raney-Metalle, insbesondere Raney-Nickel, sind sehr geeignete Katalysatoren für die reduktive Alkylierung. Die speziellen Reaktionsbedingungen hängen in grossem Ausmass von dem jeweiligen Hydrierungskatalysator und dessen exakter Aktivität ab und weichen von den allgemein für die Hydrierung bekannten nicht ab. Temperaturen im Bereich von Raumtemperatur bis zu etwa 150 °C und Wasserstoffdrucke im Bereich von Atmosphärendruck bis zu etwa 300 Atmosphären sind gemäss den Standardverfahren des Standes der Technik anwendbar. Zusätzlich zu den vorstehend im Zusammenhang mit der Hydridreduktion erwähnten inerten Lösungsmitteln können auch niedermolekulare Amide, insbesondere tertiäre Amide (wie N,N-Dimethylformamid, N,N-Dimethylacetamid, N-Methylpyrrolidon, N-Ethylpiperidon, Hexamethylphosphorsäure-trisamid), und auch Formamid und Acetamid als geeignete Lösungsmittel verwendet werden.

Die oben genannten im voraus gebildeten Imine werden vorzugsweise durch Kondensation entsprechender Ausgangsstoffe in einem inerten Lösungsmittel, z. B. Toluol oder Methylenchlorid, in erster Linie in Gegenwart von Dehydratisierungskatalysatoren, z. B. Bortrifluoridetherat, p-Toluolsulfonsäure oder Molekularsieben, hergestellt.

In jedem der Alkylierungsverfahren müssen primäre und sekundäre Aminogruppen in den Ausgangsmaterialien, mit Ausnahme der zu alkylierenden Aminogruppe, in einer vorübergehend geschützten Form während der Alkylierung vorliegen. Geeignete Schutzgruppen sowie Verfahren zu deren Einführung und Abspaltung sind aus dem Stand der Technik, der bis in die Einzelheiten ausgearbeitet ist, insbesondere als allgemeine Methoden für die Synthese von Peptiden, bekannt, vergl. Houben-Weyl: Methoden der Organischen Chemie, 4. Ausgabe, Band 15/I und II, E. Wünsch (Herausgeber): Synthese von Peptiden (Georg Thieme-Verlag, Stuttgart, 1974). Die nähere Auswahl der Schutzgruppen hängt von dem speziellen Zweck ab, wobei es erforderlich ist, insbesondere die speziellen Eigenschaften der jeweiligen Ausgangsmaterialien und die Reaktionsbedingungen des jeweiligen Verfahrens mit zu berücksichtigen. Im Fall von verschiedenen zu schützenden funktionellen Gruppen können vorteilhafte Kombinationen ausgewählt werden. Vorzugsweise werden z. B. ähnliche Amino- und Carboxyschutzgruppen in den Resten $COR^2$ und $COR^3$ beziehungsweise im Rest $R^1$ verwendet und gleichzeitig im Anschluss an die Alkylierung abgespalten.

Geeignet als Aminoschutzgruppen sind insbesondere Aminoschutzgruppen, die durch Reduktion entfernt werden können, wie z. B. insbesondere diejenigen vom Benzyloxycarbonyl-Typ, bei denen die Benzyloxycarbonylgruppe an dem aromatischen Teil durch Halogenatome, Niederalkoxygruppen und/oder Niederalkylreste und insbesondere durch Nitrogruppen, substituiert sein kann, wie die p-Chlor- und p-Brombenzyloxycarbonyl-, p-Methoxybenzyloxycarbonyl-, p-Methylbenzyloxycarbonyl- und insbesondere p-Nitrobenzyloxycarbonylgruppe, oder alternativ die Isonicotinyloxycarbonylgruppe. Eine vorteilhafte Aminoschutzgruppe ist eine Ethoxycarbonylgruppe, die in der β-Stellung eine durch drei Kohlenwasserstoffreste substituierte Silylgruppe enthält, wie Triphenylsilyl, Dimethyl-tert.-butylsilyl oder insbesondere Trimethylsilyl. Eine β-(Trihydrocarbylsilyl)-ethoxycarbonylgruppe dieses Typs, wie eine β-(Tri-niederalkylsilyl)-ethoxycarbonylgruppe, z. B. insbesondere β-(Trimethylsilyl)-ethoxycarbonyl, bildet mit der zu schützenden Aminogruppe eine entsprechende β-Trihydrocarbylsilylethoxycarbonylaminogruppe (z. B. die β-Trimethylsilylethoxycarbonylaminogruppe), die unter sehr speziellen, sehr milden Bedingungen durch Einwirken von Fluorid-Ionen abgespalten werden kann.

Es ist auch möglich, Gruppen zu verwenden, die durch Acidolyse abgespalten werden können, wie die tert.-Butoxycarbonylgruppen und analoge Gruppen, ebenso diejenigen des Aralkyl-Typs, wie

Benzhydryl, Di-(4-Methoxy)-benzhydryl und Triphenylmethyl (Trityl), oder bestimmte Aralkoxycarbonylgruppen von 2-(p-Biphenylyl)-2-propoxycarbonyl-Typ, die in der CH-PS-509 266 beschrieben werden. Es sei bemerkt, dass sich von Estern der Kohlensäure ableitende Schutzgruppen in den meisten Fällen auch durch basische Hydrolyse abspaltbar sind.

Für den fakultativen vorübergehenden Schutz der Hydroxygruppen können mit Vorteil Schutzgruppen verwendet werden, die durch Reduktion abgespalten werden können, vgl. den vorstehend zitierten Text (Houben-Weyl), und auch Gruppen, die durch Acidolyse entfernt werden können, wie 2-Tetrahydropyranyl, tert.-Butoxycarbonyl und tert.-Butyl. Bevorzugte Hydroxyschutzgruppen, die durch Reduktion abgespalten werden können, sind beispielsweise Benzylgruppen, die an dem aromatischen Teil durch Halogen, Niederalkyl, Niederalkoxy und/oder insbesondere Nitro, -insbesondere die 4-Nitrobenzylgruppe-, substituiert sein können. Es ist auch möglich, Acylgruppen, die unter schwach basischen Bedingungen abgespalten werden können, wie Formyl oder Trifluoracetyl, zu verwenden.

Für den fakultativen Schutz der Oxogruppen werden diese vorzugsweise als Ketale, insbesondere als Ketale abgeleitet von Niederalkanolen, wie Methanol oder Ethanol, oder vorteilhaft von Ethylenglykol, oder als entsprechende Thioketale, vorzugsweise als solche von 1,2-Ethandithiol, geschützt. Alle diese Gruppen können unter den nachstehend angegebenen Bedingungen Oxogruppen freisetzen.

Die anschliessende Abspaltung der Schutzgruppen gemäss der Erfindung hängt von deren Natur ab und wird in jedem Fall in an sich bekannter herkömmlicher Weise durchgeführt, wobei die allgemeinen Eigenschaften des sich ergebenden Produkts in Betracht gezogen werden. Wenn die Schutzgruppen für Amino und Carboxy so ausgewählt worden sind, dass sie unter ähnlichen Bedingungen abgespalten werden können, werden alle diese Schutzgruppen mit Vorteil in einem einzigen Arbeitsgang abgespalten ; in speziellen Fällen ist est jedoch möglich, verschiedene Typen von Gruppen zu verwenden und jede von Ihnen einzeln abzuspalten.

Die Gruppen, die durch Reduktion abgespalten werden können, insbesondere diejenigen, die halogenierte Niederalkylreste (z. B. 2,2,2-Trichlorethylreste), Isonicotinylreste (z. B. Isonicotinyloxycarbonyl), und gegebenenfalls substituierte Benzylrest, vor allem 4-Nitrobenzylreste jeglicher Art enthalten, werden vorzugsweise durch Reduktion mit Zink, gewöhnlich in Gegenwart einer Säure, vorzugsweise Essigsäure, und mit oder ohne Zugabe eines inerten organischen Lösungsmittels, gewöhnlich bei Raumtemperatur abgespalten. Die Abspaltung einer Schutzgruppe durch saure Hydrolyse (Acidolyse) wird im Fall von Gruppen des tert.-Butyl-Typs mit Hilfe von Chlorwasserstoff, Fluorwasserstoff oder Trifluoressigsäure und im Fall von säureempfindlichen Schutzgruppen hauptsächlich mit Hilfe einer niederen aliphatischen Carbonsäure, wie Ameisensäure und/oder Essigsäure, in Gegenwart von Wasser und gegebenenfalls eines polyhalogenierten Niederalkanols oder Niederalkanons, wie 1,1,1,3,3,3-Hexafluorpropan-2-ol oder Hexafluoraceton, durchgeführt. Auf diese Weise ist es beispielsweise möglich, eine N-Tritylgruppe durch eine organische Säure, wie Ameisensäure, Essigsäure, Chloressigsäure oder Trifluoressigsäure, in wässrigem oder absolutem Trifluorethanol als Lösungsmittel (vgl. DE-OS-2 346 147) oder durch wässrige Essigsäure zu spalten, die tert.-Butoxycarbonylgruppe durch Trifluoressigsäure oder Chlorwasserstoffsäure abzuspalten und die 2-(p-Biphenylyl)-isopropoxycarbonylgruppe durch wässrige Essigsäure oder beispielsweise durch ein Gemisch von Eisessig, Ameisensäure (Konzentration von 82,8 %) und Wasser (7 : 1 : 2) oder gemäss dem Verfahren in der DE-OS-2 346 147 zu entfernen. Die β-Silylethylestergruppen werden vorzugsweise durch Fluorid-Ionen-abgebende Reagentien, beispielsweise Fluoride von quaternären organischen Basen, wie Tetraethylammoniumfluorid, abgespalten.

Ketalisierte und thioketalisierte Oxogruppen werden durch Acidolyse mit üblichen starken anorganischen Säuren oder mit Oxalsäure in Gegenwart von Wasser, die letztgenannten vorteilhaft durch Behandlung mit einem schwefelbindenden Mittel, z. B. einem Quecksilber(II)-Salz und/oder Cadmiumcarbonat in freie Oxogruppen übergeführt. Schutzgruppen, die gegenüber basischen Bedingungen instabil sind, beispielsweise Formyl, Trifluoracetyl und Kohlensäureestergruppen, können vorsichtig durch Einwirken einer wässrigen Natrium- oder Kaliumbicarbonat- oder -carbonatlösung oder auch durch Einwirken von wässrigem Ammoniak in einem organischen Lösungsmittel, gewöhnlich bei Raumtemperatur, entfernt werden. Die Schutzgruppen werden vorzugsweise unter den Reaktionsbedingungen der Beispiele oder unter analogen Bedingungen abgespalten.

Im Fall der Ausgangsmaterialien der Formeln IIIA, IIIB, IV und VII, worin $R^2$ oder $R^3$ Hydroxy bedeutet, wird vor der nachfolgend im Einzelnen beschriebenen Kondensation mit der jeweiligen Reaktionskomponente, ein geeignetes Carbonsäuresalz, vorzugsweise in situ, hergestellt.

Verfahren a) Die Kondensation der Amine der Formel II mit den bekannten α-Ketosäure-Derivaten der Formel IV durch reduktive N-Alkylierung wird unter aus dem Stand der Technik bekannten Bedingungen, z. B. durch katalytische Hydrierung mit Wasserstoff in Gegenwart von Platin-, Palladium- oder Nickel-Katalysatoren oder mit chemischen Reduktionsmitteln, wie einfachen oder komplexen Leichtmetallhydriden, vorteilhaft einem Alkalimetallcyanborhydrid, wie Natriumcyanborhydrid, durchgeführt. Die reduktive Aminierung mit einem Alkalimetallcyanborhydrid wird vorzugsweise in einem inerten Lösungsmittel, z. B. Methanol oder Acetonitril, vorteilhaft in Gegenwart einer Säure, z. B. Chlorwasserstoffsäure oder Essigsäure, bei einer Temperatur zwischen etwa 0 und 50 °C, vorzugsweise Raumtemperatur, durchgeführt.

Die Alkylierung der Amine der Formel II mit einem Reagens der Formel IIIA, wie es aus dem Stand der

Technik gut bekannt ist, wird mit oder ohne basische Katalysatoren, wie Triethylamin oder Kaliumcarbonat in einem inerten Lösungsmittel durchgeführt.

Die Ausgangsmaterialien der Formeln IIIA und IV sind bekannt, oder wenn sie unbekannt sind, können sie auf einfache Weise nach herkömmlichen Syntheseverfahren hergestellt werden. Die Ausgangsmaterialien der Formel II können nach herkömmlichen Syntheseverfahren erhalten werden und mit Vorteil in der Weise, die nachstehend eingehender für die speziellen Zwischenprodukte beschrieben und veranschaulicht ist.

Verbindungen der Formel II können durch Kondensation, unter basischer Katalyse, einer Verbindung der Formel

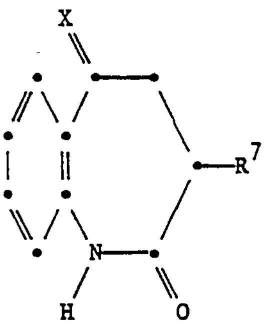

(XVI)

worin X zwei Wasserstoffatome, ein Wasserstoffatom und eine veresterte oder veretherte Hydroxygruppe, Oxo oder als Ketal oder Thioketal geschütztes Oxo bedeutet und $R^7$ Amino, Azido oder Acylamino, z. B. Niederalkanoylamino oder -alkoxycarbonylamino, bedeutet, mit einer Verbindung der Formel

$$Z—CH_2COR^3 \qquad (IIIB),$$

worin $R^3$ Hydroxy, Diniederalkylamino, Niederalkoxy, Arylniederalkoxy, Niederalkanoyloxymethoxy oder Niederalkoxycarbonylniederalkoxy bedeutet und Z eine reaktive veresterte Hydroxygruppe bedeutet, und gegebenenfalls Reduktion, Hydrogenolyse, Hydrolyse oder Alkylierung des erhaltenen Zwischenprodukts, erhalten werden.

Verbindungen der Formel XVI werden aus den entsprechenden gegebenenfalls substituierten und/oder derivatisierten 2,3,4,5-Tetrahydro-1H-1-benzazepin-2-onen [J. Chem. Soc. 1937, 456; British Patent 1,359,285, Liebigs Ann. Chem. 574, 171 (1951)] erhalten. Neue, in geeigneter Weise derivatisierte 1-Benzazepin-2-on-Ausgangsverbindungen werden vorteilhaft durch Beckmann-Umlagerung der entsprechend derivatisierten Naphthalen-1-one unter Verwendung von wohlbekannten und hierin beschriebenen Verfahren hergestellt.

Solche Tetrahydro-1-benzazepin-2-one werden in die 3-Halogen-, z. B. die 3-Chlor-2,3,4,5-tetrahydro-1H-1-benzazepin-2-one übergeführt, wobei hierin beschriebene Verfahren angewendet werden, z. B. Behandlung mit Phosphorpentachlorid, wobei man zuerst das 3,3-Dichlorderivat erhält, mit anschliessender Hydrierung. Substitution solcher Halogenderivate mit Metallazid, z. B. Natriumazid, und gegebenenfalls Reduktion, oder Substitution mit Ammoniak und gegebenenfalls Acylierung .ergibt racemische Verbindungen der Formel XVI, in denen $R^7$ Amino oder Acylamino bedeutet.

Alternativ können Verbindungen der Formel XVI, worin $R^7$ Amino oder Acylamino bedeutet, durch Reduktion und Cyclisierung einer in geeigneter Weise substituierten und/oder derivatisierten 4-(2-Nitrophenyl)-2-aminobuttersäure und gegebenenfalls nachfolgende N-Alkylierung oder N-Acylierung hergestellt werden.

Die Auftrennung einer Verbindung der Formel XVI, in der $R^7$ Amino bedeutet gemäss Verfahren, die zum Stand der Technik gehören, ergibt das 3-(S)-Enantiomere.

Eine alternative Synthese für die optisch aktiven Verbindungen dieser Erfindung geht aus der von der natürlichen Aminosäure Tryptophan. So wird L-4-(2-Aminophenyl)-4-oxo-2-aminobuttersäure (L-Kynurenin, J. Am. Chem. Soc. 76, 1708 (1954), erhalten aus L-Tryptophan) in ein optisch aktives Ausgangsmaterial der Formel XVI überführt, worin $R^7$ Acylamino bedeutet, z. B. 3-(S)-t-Butoxycarbonylamino-2,3,4,5-tetrahydro-1H-1-benzazepin-2,5-dion, wie in Australian Journal of Chemistry 33, 633-40 (1980) beschrieben. Die Lactam-Alkylierung einer Verbindung der Formel XVI mit einem Reagens der Formel IIIB, wird auf bekannte Weise, vorzugsweise in Gegenwart von Basen, wie Alkalimetallhydriden, z. B. Natrium- oder Kaliumhydrid, Alkalimetallalkoxiden, wie Kalium-tert.-butylat oder Natriummethanolat, organo-metallischen Reagentien, z. B. Lithiumdiisopropylamid, oder unter Bedingungen der Phasen-Transfer-Katalyse, z. B. in Gegenwart eines Tetrabutylammoniumsalzes, vorzugsweise in einem Lösungsmittel, z. B. Tetrahydrofuran, Dimethylformamid, bei einer Temperatur vorzugsweise zwischen 0° und 75 °C, durchgeführt.

Das Verfahren b) wird in üblicher Weise, unter den Bedingungen der vorher beschriebenen substitutiven Alkylierung, vorzugsweise in Gegenwart von sehr starken Basen, wie Alkalimetallhydriden (z. B. Natrium- oder Kaliumhydrid), -alkoholaten (z. B. Natrium-methylat oder -ethylat, Kalium-tert.-

butylat) oder -amiden (z. B. Lithiumdiisopropylamid) durchgeführt, wobei die vorstehend erwähnten Ether und Amide als Lösungsmittel bevorzugt sind.

Die Ausgangsmaterialien der Formel IIIB sind bekannt, oder wenn sie unbekannt sind, können sie auf einfache Weise nach herkömmlichen Syntheseverfahren hergestellt werden. Die Ausgangsmaterialien der Formel V können nach herkömmlichen Syntheseverfahren erhalten werden und mit Vorteil in der Weise, die nachstehend eingehender für die speziellen Zwischenprodukte beschrieben und veranschaulicht ist.

Verbindungen der Formel V können durch Kondensation unter den Bedingungen der reduktiven Alkylierung einer Verbindung der Formel

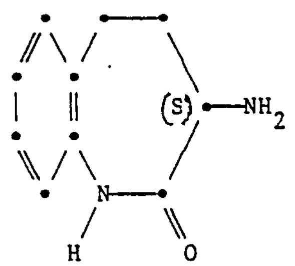

(XVII)

mit einer Verbindung der Formel

$$R^1—CO—COR^2 \quad (IV),$$

worin S, $R^1$ und $R^2$ die vorher definierten Bedeutungen haben, oder unter Alkylierungsbedingungen mit einer Verbindung der Formel

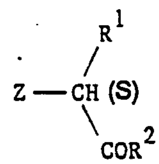

(IIIA)

worin S, $R^1$, $R^2$ und Z die vorher angegebenen Bedeutungen haben, erhalten werden.

Das Verfahren c), das auch eine Alkylierungsreaktion darstellt, wird entsprechend den gleichen allgemeinen Gesichtspunkten und unter den gleichen experimentellen Bedingungen durchgeführt, wie im einzelnen vorstehend beschrieben (substitutive oder reduktive Alkylierung). Die Ausgangsmaterialien der Formel VI können mit Hilfe an sich bekannter herkömmlicher Verfahren hergestellt werden, z. B. in der nachstehend eingehender beschriebenen Weise.

Die Lysinverbindungen der Formel VII sind bekannt, oder können, auch wenn sie unbekannt sind, nach herkömmlichen Syntheseverfahren leicht hergestellt werden.

Die Ausgangsverbindungen der Formel VI, worin Y Oxo bedeutet, können durch Behandlung einer Verbindung der Formel II mit einem Niederalkylnitrit, z. B. tert.-Butylnitrit, gefolgt von einer Persäure, z. B. m-Chlorperbenzoesäure, in einem inerten Lösungsmittel, wie Chloroform oder Methylenchlorid, vorzugsweise bei Raumtemperatur, hergestellt werden. Die Ausgangsverbindungen der Formel VI, worin Y zwei reaktive veresterte Hydroxygruppen (wie Dihalogen, z. B. Dichlor) bedeutet, können hergestellt werden, indem ein 2,3,4,5-Tetrahydro-1H-1-benzazepin-2-on zuerst mit einem Halogenierungsmittel wie Phosphorpentachlorid behandelt wird, um z. B. die 3,3-Dichlorverbindung zu erhalten, daraufhin mit einer Verbindung der Formel IIIB alkyliert wird.

Eine Verbindung der Formel VI, worin Y eine reaktive veresterte Hydroxygruppe und ein Wasserstoffatom bedeutet, kann durch Hydrierung der entsprechenden Verbindung, worin Y z. B. zwei reaktive veresterte Hydroxygruppen, wie Dichlor, bedeutet, hergestellt werden.

Das Verfahren d) wird auch in herkömmlicher Weise unter den allgemeinen Bedingungen der Solvolyse durchgeführt, die dafür bekannt sind, dass sie Cyanide (Nitrile) in die freien Carbonsäuren, ihre Salze oder Ester überführen. Für die Umwandlung in eine freie Säure wird die Hydrolyse mit Wasser vorteilhaft in einem inerten organischen Lösungsmittel, das zumindest teilweise mit Wasser mischbar ist, wie Ether (z. B. Diethyl- oder Diisopropylether, 1,2-Dimethoxyethan oder insbesondere Dioxan oder Tetrahydrofuran), oder einem Niederalkanol (z. B. Methanol, Ethanol, Isopropylalkohol, einem Butylalkohol, insbesondere tert.-Butylalkohol) durchgeführt, wobei eine grössere Menge Wasser in den letztgenannten Fällen erforderlich ist, um eine Alkoholyse zu verhindern. Die Hydrolyse kann sowohl durch starke Säuren, insbesondere anorganische Säuren, wie Schwefelsäure, oder vorzugsweise Halogenwasserstoffsäuren (z. B. Bromwasserstoffsäure oder als erste Wahl Chlorwasserstoffsäure), als auch durch Basen, insbesondere anorganische Basen, wie Hydroxide und Carbonate der Alkalimetalle, z. B. Natrium- und Kaliumhydroxid, katalysiert werden. Die Basen werden gewöhnlich in zumindest stöchiometrischen

Anteilen verwendet, wobei Carbonsäuresalze als primäre Produkte entstehen. Die sauren Katalysatoren werden zur Erzielung des besten Resultats vorteilhaft in Form einer verdünnten wässrigen Lösung angewandt. Die Endprodukte der Formel I, worin $COR^2$ und/oder $COR^3$ eine mit einem Niederalkanol veresterte Carboxylgruppe bedeutet, können erhalten werden, indem man die Solvolyse des Nitrils mit dem entsprechenden Alkohol (Alkoholyse) in Gegenwart einer katalytische Menge einer wasserfreien starken Säure, vorteilhaft von gasförmigem Chlorwasserstoff, durchführt. Gewöhnlich wird ein Ueberschuss an Alkohol als Lösungsmittel verwendet, jedoch können inerte organische Lösungsmittel, wie acyclische und cyclische Ether (insbesondere die vorstehend erwähnten), und/oder halogenierte Niederalkane (insbesondere Chloroform und Dichlormethan) zugegeben werden. Wird die Alkoholyse unter streng wasserfreien Bedingungen durchgeführt, muss das primäre Produkt (Imidoester), vorteilhaft durch Zugabe von Wasser, hydrolysiert werden. Andererseits wird, wenn man die Alkoholyse in Gegenwart eines annähernd stöchiometrischen Aequivalents von Wasser durchführt, der gewünschte Ester direkt erhalten.

Die Ausgangsmaterialien der Formel VIII können mit Hilfe an sich bekannter herkömmlicher Methoden, z. B. durch eine Kondensation analog derjenigen des Verfahrens c), erhalten werden, wobei ein Ausgangsmaterial der Formel VI mit einem Amin der Formel

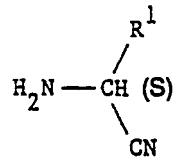

(VII')

worin S und $R^1$ die vorstehend angegebenen Bedeutungen besitzen, behandelt wird. Auch die Verfahren a) und b) können in analoger Weise zur Herstellung von Nitrilen der Formel VIII verwendet werden.

Die Cyclisierung entsprechend der Verfahrensvariante e) kann auch in an sich bekanter Weise, z. B. durch Dehydratation, durchgeführt werden. Besonders geeignete allgemeine Methoden für diesen Zweck sind die im Zusammenhang mit der Bildung der Amidbindung in Peptiden entwickelten, wie sie in Sammelwerken, z. B. Houben-Weyl, Band 15/I und Band 15/II, wie vorstehend zitiert, angegeben werden. Gemäss einer bevorzugten Abwandlung wird die zu cyclisierende Aminogruppe durch Protonierung (d. h. in Form eines Säureadditionssalzes) inaktiv gemacht und die Carboxylgruppe in einen aktivierten Ester, wie einen solchen mit 2,4,5-Trichlorphenol, Pentachlorphenol, Pentafluorphenol, 2-Nitrophenol oder insbesondere 4-Nitrophenol, oder mit einer N-Hydroxyverbindung, wie N-Hydroxysuccinimid, 1-Hydroxybenztriazol oder N-Hydroxypiperidin, oder alternativ mit einem N,N'-disubstituierten Isoharnstoff, wie insbesondere N,N'-Dicyclohexylisoharnstoff, oder einem analogen allgemein bekannten aktivierenden Mittel, übergeführt. Die Cyclisierung wird durchgeführt, indem man vorzugsweise durch Zugabe einer organischen Base, z. B. eines quaternären Ammoniumsalzes oder insbesondere eines tertiären Amins, wie Triethylamin, N-Ethylmorpholin oder N-Methylpiperidin, basisch macht, um die zu cyclisierende Aminogruppe durch Umwandlung in die unprotonierte Form zu reaktivieren. Die Reaktionstemperatur beträgt gewöhnlich von —20 bis +50 °C, vorzugsweise annähernd Raumtemperatur, und es werden übliche Lösungsmittel verwendet, z. B. Dioxan, Tetrahydrofuran, Acetonitril, Pyridin, Dimethylformamid, Dimethylacetamid, Dimethylsulfoxid, N-Methylpyrrolidon, Hexamethylphosphorsäuretrisamid, sowie Chloroform und Methylenchlorid und bewährte Mischungen derselben. Bei einer speziellen Variante des Verfahrens kann die Carboxygruppe direkt in situ durch Einwirken der freien Säure mit einem Carbodiimid, wie N,N'-Dicyclohexylcarbodiimid (gegebenenfalls unter Zusatz von N-Hydroxysuccinimid, eines unsubstituierten oder beispielsweise Halogen-, Methyl- oder Methoxy-substituierten 1-Hydroxybenztriazols oder von 4-Hydroxybenzo-1,2,3-triazin-3-oxid oder N-Hydroxy-5-norbornen-2,3-dicarboximid) oder mit N,N'-Carbonyldiimidazol aktiviert werden.

Die Ausgangsmaterialien der Formel IX können nach an sich bekannten allgemeinen Methoden, z. B. wie sie nachstehend anhand spezieller Beispiele erörtert werden, hergestellt werden.

Auch die Reduktion entsprechend dem Verfahren f) kann in einer Weise durchgeführt werden, wie sie an sich allgemein für die Sättigung derartiger Doppelbindungen bekannt ist. Im Einzelnen kann eine Doppelbindung in einem ungesättigten Ausgangsmaterial der Formel X sich zwischen C3 und C4, zwischen C4 und C5, und/oder zwischen C3 und dem benachbarten Stickstoffatom und/oder zwischen diesem Stickstoffatom und dem benachbarten exocyclischen Kohlenstoffatom befinden. Die Sättigung derartiger Doppelbindungen wird vorteilhaft durch katalytische Hydrierung, beispielsweise unter den im einzelnen vorstehend erörterten bevorzugten Bedingungen, sowie auch durch Metallreduktion, wie Zinkreduktion, in neutralem oder saurem Medium oder insbesondere im Fall der C-N-Doppelbindung durch Diboran oder komplexe Hydride, wie Natriumborhydrid, wie vorstehend erwähnt, durchgeführt. Die ungesättigten Ausgangsmaterialien für diese Verfahrensvariante werden nach allgemein bekannten Methoden, z. B. den bei den Verfahren a) und c) und/oder in einer speziellen Form, wie nachstehend erörtert, erhalten.

Die Ausgangsverbindungen, die z. B. eine C4-C5 Doppelbindung aufweisen, werden von Ausgangsmaterialien ausgehend hergestellt, die in ihrer Struktur identisch sind mit Verbindungen der Formeln II, V und VI, ausser dass sie eine zusätzliche C4-C5 Doppelbindung aufweisen, auf zu den Verfahren a), b) oder c) analoge Weise. Diese wiederum werden aus den entsprechenden 5-Hydroxy- oder 5-veresterten oder -veretherten Hydroxyderivaten von Verbindungen der Formeln II, V und VI durch Eliminierungsverfahren hergestellt. Diese 5-substituierten Verbindungen werden z. B. ausgehend von L-Kynurenin, wie beschrieben, hergestellt.

Die Ringöffnung des Caprolactamringes in einer Verbindung der Formel XI nach Verfahren g) wird nach im Stand der Technik bekannten Methoden ausgeführt, z. B. durch Behandlung mit einer starken Mineralsäure, vorzugsweise bei erhöhter Temperatur. Man erhält die Verbindung der Formel I, worin $R^2$ und $R^3$ Hydroxy bedeuten.

Eine Ausgangsverbindung der Formel XI wird z. B. durch reduktive Alkylierung von 3-(S)-Amino-ε-caprolactam mit einer Verbindung der Formel VI nach dem Verfahren c) hergestellt.

Gemäss Verfahren h) können Verbindungen der Formel XII, worin X ein Wasserstoffatom und eine Hydroxygruppe bedeutet, in Verbindungen überführt werden, worin X zwei Wasserstoffatome bedeutet, z. B. durch katalytische Hydrierung des Adduktes mit einem Carbodiimid, z. B. des Adduktes, das durch Kondensation einer Verbindung, worin X ein Wasserstoff und eine Hydroxygruppe bedeutet, mit Dicyclohexylcarbodiimid in Gegenwart von Kupferchlorid, entsprechend der allgemeinen Methode, die in Chem. Ber. 107, 1353 (1974) beschrieben wird.

Alternativ können Verbindungen, worin X ein Wasserstoffatom und eine Hydroxygruppe bedeutet, zuerst in die entsprechende Verbindung übergeführt werden, worin X ein Wasserstoffatom und eine acylierte Hydroxygruppe, z. B. Acetoxy oder Chlor, bedeutet, und nachfolgend zu Verbindungen, worin X zwei Wasserstoffatome bedeutet, reduziert werden, z. B. durch katalytische Hydrogenierung in Anwesenheit eines Palladium-Katalysators.

Die Reduktion der Oxogruppe in einer Verbindung der Formel XII zu zwei Wasserstoffatomen kann vorteilhafterweise durch Behandlung mit amalgamiertem Zink und Chlorwasserstoffsäure oder durch Raney-Nickel Desulfurisation eines entsprechenden Dithioketals erreicht werden.

Verbindungen der Formel XII, worin X Oxo bedeutet, können zuerst durch Reduktion in die entsprechenden Verbindungen, worin X ein Wasserstoffatom und eine Hydroxygruppe bedeutet, überführt werden, z. B. durch katalytische Hydrogenierung, wie mit Wasserstoff in Gegenwart eines Platin-Katalysators oder mit einem Metallhydrid-Reduktionsmittel, wie Natriumborhydrid, oder nach der Methode von Meerwein-Ponndorf, oder einer Variante davon, wobei man ein Alkanol, insbesondere Isopropanol, sowohl als Lösungsmittel und Reduktionsmittel und ein Metallalkoxid, vorzugsweise eines, das dem reduzierenden Alkohol entspricht, wie Aluminiumisopropylat, als Katalysator verwendet.

Die Ausgangsverbindungen der Formel XII werden analog zu den bereits beschriebenen Verfahren a), b) oder c) hergestellt, wobei man ausgeht von z. B. L-Kynurenin, wie bereits beschrieben. Bei Verfahren, die einen reduktiven Alkylierungsschritt beinhalten, müssen für Ausgangsmaterialien, die leicht reduzierbare funktionelle Gruppen aufweisen, wie die 5-Oxogruppe, besondere Massnahmen beachtet werden. Um diese Gruppen zu erhalten, müssen sie entweder zwischenzeitlich geschützt werden, oder es müssen selektive Reduktionsbedingungen, wie sie im Stand der Technik bekannt sind, angewendet werden, oder, wenn eine gleichzeitige Reduktion dieser Gruppen erwünscht oder notwendig ist, müssen kräftige Reagenzien und/oder Reaktionsbedingungen entsprechend angewandt werden.

Im Verfahren i) bedeutet eine Gruppe $R^4$, die sich in 2-Aminoethyl umwandeln lässt, z. B. 2-Nitroethyl, Cyanmethyl, 2-Azidoethyl, 2-Carbamoylethyl, 2-Hydrazincarbonylethyl, 2-Azidocarbonylethyl oder 2-Acylaminoethyl (vorzugsweise 2-(Benzyloxycarbonylamino)-ethyl oder 2-Niederalkanoylaminoethyl), oder Vinyl, oder die Gruppe $(CH_2)_2Z^5$, worin $Z^5$ eine Gruppe bedeutet, die sich durch Substitution in Amino umwandeln lässt, wie eine reaktive veresterte Gruppe, z. B. Halogen (vorzugsweise Brom oder Iod), oder Acyloxy (vorzugsweise Mesyloxy).

Die Umwandlung einer Gruppe $R^4$ in 2-Aminoethyl wird nach Verfahren, die im Stand der Technik bekannt sind, ausgeführt. Z. B. wird 2-Nitroethyl, Cyanmethyl und 2-Azidoethyl durch Reduktion, wie katalytische Hydrogenierung in einem inerten Lösungsmittel, in 2-Aminoethyl übergeführt. Eine Gruppe $(CH_2)_2Z^5$ wird z. B. durch Kondensation mit Kaliumphthalimid und nachfolgende Freisetzung der Aminogruppe, wie es im Stand der Technik bekannt ist, oder mit Chloramin, in 2-Aminoethyl übergeführt. Eine Vinylgruppe kann z. B. durch Behandlung mit Diboran in einem inerten Lösungsmittel wie Tetrahydrofuran, gefolgt von einer Behandlung mit Chloramin, in 2-Aminoethyl übergeführt werden. 2-Acylaminoethyl wird in 2-Aminoethyl durch Methoden überführt, die bei der Entfernung von Aminoschutzgruppen beschrieben sind. 2-Carbamoylethyl, 2-Hydrazincarbonylethyl, 2-Azidocarbonylethyl werden in 2-Aminoethyl auf bekannte Weise (z. B. Curtius-Abbau über eine Isocyanatzwischenstufe) übergeführt.

Die Ausgangsverbindungen der Formel XIII werdem z. B. nach den Verfahren a), b), c), e) oder f) hergestellt, wobei man ein Ausgangsmaterial der Formel IIIA, IV, V, VII, IX oder X durch die entsprechende Verbindung ersetzt, worin $R^1$ $(CH_2)_2$—$R^4$ bedeutet.

Im Verfahren j) bedeuten $R^5$ und $R^6$ vorzugsweise Gruppen, die in Carboxy übergeführt werden können, z. B. veresterte Carboxygruppen, Carboxygruppen in Form ihrer Anhydride, inklusive entsprechende Gruppen von asymmetrischen und inneren Anhydriden, amidierte Carboxygruppen, Amidinogruppen, inklusive cyclische Amidinogruppen, Iminoethergruppen inklusive cyclische Iminoethergruppen, z. B. 2-Oxazolinyl oder Dihydro-2-oxazolinylgruppen, substituiert durch Niederalkyl, und auch Hydroxymethyl, verethertes Hydroxymethyl, verestertes Hydroxymethyl (wie Niederalkanoyloxymethyl), Trialkoxymethyl, Acetyl, Trihalogenacetyl, Halogenmethyl, Formyl, Diniederalkoxymethyl oder Vinyl.

Die Umwandlung von $R^5$ und/oder $R^6$ im Verfahren j) in Carboxygruppen wird auf an sich bekannte Weise durchgeführt und wie hier und in den Beispielen beschrieben, z. B. durch Solvolyse, wie Hydrolyse oder Acidolyse (z. B. für amidierte und veresterte Carboxygruppen), durch Reduktion (z. B. für veresterte Carboxygruppen). Z. B. können Trichlorethyl- oder 2-Iodethylester durch Reduktion in die Carbonsäuren übergeführt werden, z. B. mit Zink und einer Carbonsäure in Gegenwart von Wasser. Benzylester oder Nitrobenzylester können durch katalytische Hydrogenierung in die Carbonsäuregruppen übergeführt werden, die letzteren auch mit chemischen Reduktionsmitteln, z. B. Natriumdithionit oder mit Zink und einer Carbonsäure. Ein tert.-Butylester kann auch mit Trifluoressigsäure gespalten werden.

Weiterhin können Verbindungen der Formel XIV, worin $R^5$ und/oder $R^6$ Acetyl bedeuten, oxidativ zu den entsprechenden Verbindungen der Formel I, worin $R^2$ und $R^3$ Hydroxy bedeuten, gespalten werden, indem sie zuerst in eine Verbindung, worin $R^5$ und/oder $R^6$ Trihalogenacetyl bedeuten, z. B. Tribrom- oder Triiodacetyl, wie durch Behandlung mit Natriumhypobromit, übergeführt werden, gefolgt von Spaltung mit z. B. einer wässrigen Base, wie Natriumhydroxyd.

Verbindungen der Formel XIV, worin $R^5$ und/oder $R^6$ z. B. Hydroxymethyl, verestertes oder verethertes Hydroxymethyl, Formyl, Diniederalkoxymethyl oder Alkylendioxymethyl (Formyl geschützt in Form eines Acetals, z. B. des Dimethylacetals) bedeuten, werden mit einem geeigneten Oxidationsmittel zu den entsprechenden Verbindungen der Formel I, worin $R^2$ und/oder $R^3$ Hydroxy bedeuten, oxidiert.

Verbindungen der Formel XIV, worin $R^5$ und/oder $R^6$ Vinyl bedeuten, können in Verbindungen der Formel I übergeführt werden, worin $R^2$ und/oder $R^3$ Hydroxy bedeuten, indem sie z. B. zuerst zu Verbindungen der Formel XIV, worin $R^5$ und/oder $R^6$ Formyl bedeuten, oxidiert werden, die darauf zu Verbindungen der Formel I, worin $R^2$ und/oder $R^3$ Hydroxy bedeuten, oxidiert werden.

Die Hydrolyse von Zwischenstoffen der Formel XIV, worin $R^5$ und/oder $R^6$ Trialkoxymethyl bedeuten, zu Verbindungen der Formel I, worin $R^2$ und/oder $R^3$ Hydroxy bedeuten, wird vorzugsweise mit anorganischen Säuren, wie Halogenwasserstoffsäuren oder Schwefelsäure, durchgeführt.

Die Ausgangsverbindungen der Formel XIV werden analog zu den Verfahren a), b), c), e) oder f) durchgeführt, indem man ein Ausgangsmaterial der Formeln II, IIIA, IIIB, IV, V, VI, VII, IX oder X durch eine entsprechende Verbindung, worin $COR^2$ und/oder $COR^3$ $R^5$ und/oder $R^6$ bedeuten, ersetzt.

Die Entfernung von Amino und Carboxyschutzgruppen nach Verfahren k) wird nach bekannten Methoden durchgeführt.

Die Entfernung und Art der Aminoschutzgruppen $Z^2$ und $Z^4$ ist vorstehend bereits beschrieben. Die Carboxyschutzgruppen $Z^1$ und $Z^3$ bedeuten verschiedene Reste des Alkoholteils von Mono- oder Diestern der Dicarbonsäuren der Formel I, worin $R^2$ und $R^3$ Hydroxy bedeuten.

Die freie Carboxylgruppe kann aus einem veresterten Carboxyl in allgemein bekannter Weise, insbesondere durch Basen-katalysierte Hydrolyse, freigesetzt werden. Von speziellem Interesse sind jedoch Methoden, nach denen es möglich ist, selektiv eine spezielle durch die Symbole $—COR^2$ und $—COR^3$ wiedergegebene Carboxylgruppe freizusetzen. In einem derartigen Fall kann von einer geeigneten Kombination von Estergruppen Gebrauch gemacht werden, die im Stand der Technik insbesondere als Carboxylschutzgruppen bekannt und in grosser Vielfalt insbesondere für die Synthese von Peptiden entwickelt worden sind, vgl. Houben-Weyl, Band 15/I und Band 15/II wie vorstehend zitiert. Für die selektive Abspaltung unter Freisetzung der Carboxylgruppe geeignete Reste sind Ester, die sich z. B. von Alkoholen, die durch Acidolyse entfernbare Reste ergeben, ableiten, wie Cyanmethylalkohol, Benzoylmethylalkohol oder tert.-Butylalkohol, jedoch insbesondere von Alkoholen, die Reste ergeben, die abgespalten werden können durch Reduktion, wie 2,2,2-Trichlorethanol, Benzylalkohol, und insbesondere 4-Nitrobenzylalkohol, oder alternativ Isonicotinalkohol. Eine besonders bevorzugte Klasse von substituierten Alkanolen sind Ethylalkohole, die in β-Stellung eine trisubstituierte Silylgruppe enthalten, wie Triphenylsilyl, Dimethyl-tert.-butylsilyl oder insbesondere Trimethylsilyl. Wie beispielsweise in der BE-PS 851 576 beschrieben, sind diese Alkohole besonders zur selektiven Entfernung geeignet, da die entsprechenden β-Silylethylester, beispielsweise β-(Trimethylsilyl)-ethylester, die Stabilität herkömmlicher Alkylester besitzen, jedoch selektiv unter milden Bedingungen durch Einwirken von Fluorid-Ionen unter Erhalt anderer veresterter Carboxylgruppen, z. B. von Alkoxycarbonylgruppen, abgespalten werden können.

Die Abspaltung der veresternden Gruppen hängt von ihrer Natur ab und wird in jedem Fall in herkömmlicher, an sich bekannter Weise durchgeführt, wobei man die Eigenschaften der anderen beteiligten Reste mit berücksichtigt. Die Gruppen, die durch Reduktion abgespalten werden können, insbesondere diejenigen, die halogenierte Niederalkylreste (beispielsweise 2,2,2-Trichlorethylreste), Isonicotinylreste (z. B. Isonicotinyloxycarbonyl) und gegebenenfalls substituierte Benzylreste, vor allem 4-Nitrobenzylreste jeglicher Art enthalten, werden bevorzugt durch Reduktion mit Zink, gewöhnlich in Gegenwart einer Säure, vorzugsweise Essigsäure, und mit oder ohne Zusatz eines inerten organischen Lösungsmittels, gewöhnlich bei Raumtemperatur abgespalten, diejenigen des Benzyl-Typs, insbesondere unsubstituierte Benzylester, werden auch durch Hydrogenolyse-Techniken, wie sie üblicherweise für Benzylgruppen angewandt werden, entfernt.

Die Abspaltung von Estergruppen durch saure Hydrolyse (Acidolyse) wird insbesondere im Fall des tert.-Butyltyps vorteilhaft durchgeführt durch Hydrolyse mit anorganischen Säuren, wie Halogenwasserstoffsäuren, Trifluoressigsäure oder Schwefelsäure. Die β-Silylethylestergruppen werden vorzugsweise durch Fluorid-Ionen erzeugende Reagentien, z. B. Fluoride von quaternären organischen Basen, wie Tetraethylammoniumfluorid, abgespalten. Ester, die Basen-instabil sind, können vorsichtig durch rasches Einwirken einer wässrigen Natrium- oder Kaliumbicarbonatlösung oder vorzugsweise von wässrigem Ammoniak in einem organischen Lösungsmittel, gewöhnlich bei Raumtemperatur, entfernt werden. Die Estergruppen werden vorzugsweise unter den Reaktionsbedingungen der Beispiele oder unter analogen Bedingungen abgespalten.

Eine geeignete Kombination der Estergruppen kann bei den früheren Synthesestufen oder durch eine geeignete Wahl an Ausgangsmaterialien und Reaktanten ausgewählt werden, wobei z. B. eine selektiv abspaltbare Estergruppe mit einem Carboxyl eingeführt wird, das in der letzten Stufe freigesetzt wird.

Die Ausgangsmaterialien der Formel XV werden nach den vorstehend beschriebenen Verfahren für die Herstellung von Verbindungen der Formel I hergestellt, wobei man von Verbindungen ausgeht, worin die Amino und/oder Carboxygruppen durch $Z^1$ bis $Z^4$ geschützt sind.

Zur Veresterung kann eine Carbonsäure direkt mit einem Diazoalkan, insbesondere Diazomethan, oder mit einem entsprechenden Alkohol in Gegenwart eines stark sauren Katalysators (z. B. Schwefelsäure oder eine organische Sulfonsäure) und/oder eines dehydrierenden Mittels (z. B. Dicyclohexylcarbodiimid) umgesetzt werden. Alternativ kann die Carbonsäure in ein reaktives Derivat übergeführt werden, wie einen reaktiver Ester, der in Verbindung mit dem Verfahren e) erwähnt ist, oder in ein gemischtes Anhydrid, z. B. mit ein Säurehalogenid (z. B. insbesondere ein Säurechlorid), und dieser aktivierte Zwischenstoff wird mit dem gewünschten Alkohol umgesetzt.

Die Schutzgruppen werden vorzugsweise unter den Reaktionsbedingungen der Beispiele oder unter analogen Bedingungen abgespalten.

Zur Durchführung der Umwandlung eines Zwischenstoffs oder eines Endproduktes in einen anderen Zwischenstoff oder ein anderes Endprodukt, werden Umwandlungen wie die folgenden ausgeführt :

Eine Aminogruppe wird alkyliert, und/oder eine Oxogruppe wird in Hydroxy (plus Wasserstoff) oder in zwei Wasserstoffatome durch Reduktion überführt, und/oder Hydroxy wird durch Reduktion in Wasserstoff überführt, und/oder freies Carboxy wird aus seiner veresterten Form durch Hydrolyse oder Hydrogenolyse freigesetzt, und/oder eine Aminogruppe wird acyliert und/oder freies Carboxy wird verestert, und/oder Amino wird in Oxo übergeführt.

Umwandlung von Verbindungen der Formel I und/oder Zwischenstoffen, worin $R^2$ und/oder $R^3$ z. B. Niederalkoxy, Arylniederalkoxy bedeuten, zu Verbindungen der Formel I, worin $R^2$ und/oder $R^3$ Hydroxy bedeuten, wird vorzugsweise durch Hydrolyse mit anorganischen Säuren, wie Halogenwasserstoffsäuren oder Schwefelsäure, oder mit wässrigen Alkalien, vorzugsweise Alkalimetallhydroxiden, wie Lithium- oder Natriumhydroxid, durchgeführt.

Verbindungen der Formel I und/oder Zwischenstoffe, worin $R^2$ und $R^3$ verethertes Hydroxy bedeuten, können in Monocarbonsäuren der Formel I, worin $R^2$ oder $R^3$ Hydroxy bedeutet, übergeführt werden. Eine solche Umwandlung wird durch selektive hydrolytische oder hydrogenolytische Verfahren durchgeführt, die im Stand der Technik bekannt sind, und von dem chemischen Charakter der Substituenten $R^2$ und $R^3$ abhängen.

Freie Carbonsäuren der Formel I oder Zwischenstoffe, worin $R^2$ und/oder $R^3$ Hydroxy bedeuten, oder deren Salze, können mit den geeigneten Alkoholen oder entsprechenden Derivaten davon, nach bekannten Verfahren in die entsprechenden Mono- oder Bisester überführt werden, d. h. in Verbindungen der Formel I und/oder Zwischenstoffe, worin $R^2$ und/oder $R^3$ z. B. Niederalkoxy, Arylniederalkoxy, Niederalkanoyloxymethoxy oder Niederalkoxycarbonylniederalkoxy bedeuten.

Verbindungen der Formel I, worin $R^1$ 4-Aminobutyl debeutet, können in Verbindungen, worin $R^1$ 4-Acylaminobutyl bedeutet, übergeführt werden, oder vice versa, durch Verfahren, die zum Stand der Technik gehören und hierin im Zusammenhang mit Schutzgruppen beschrieben werden.

Die vorstehenden Reaktionen werden nach Standard-Methoden in Gegenwart oder Abwesenheit von Verdünnungsmitteln, vorzugsweise solchen, die gegenüber den Reagentien inert sind und deren Lösungsmittel darstellen, von Katalysatoren, Kondensationsmitteln bzw. den anderen Mitteln und/oder in inerter Atmosphäre, bei niedrigen Temperaturen, Raumtemperatur oder erhöhten Temperaturen, vorzugsweise beim Siedepunkt der verwendeten Lösungsmittel, bei atmosphärischem oder überatmosphärischem Druck, durchgeführt.

Die Erfindung umfasst weiterhin jegliche Variante der vorliegenden Verfahren, bei der ein bei irgendeiner Stufe derselben erhältliches Zwischenprodukt als Ausgangsmaterial verwendet wird und die verbliebenen Stufen ausgeführt werden, oder das Verfahren bei irgendeiner Stufe desselben unterbrochen wird oder bei der die Ausgangsmaterialien unter den Reaktionsbedingungen gebildet werden oder bei der die Reaktionskomponenten in Form ihrer Salze oder optisch reinen Antipoden verwendet werden. Hauptsächlich diejenigen Ausgangsmaterialien sollten bei diesen Reaktionen verwendet werden, die zur Bildung der vorstehend als besonders wertvoll angegebenen Verbindungen führen.

Die Erfindung betrifft auch neue Ausgangsmaterialien und Verfahren zu deren Herstellung.

In Abhängigkeit von der Wahl der Ausgangsmaterialien und Methoden können die neuen Verbindungen in Form eines der möglichen Isomeren oder von deren Gemischen vorliegen, beispielsweise in Abhängigkeit von der Anzahl der asymmetrischen Kohlenstoffatome, als reine optische Isomere, wie als

Antipoden, oder als Gemische von optischen Isomeren, wie als Racemate, oder als Gemische von Diastereoisomeren, aus denen der Antipode, der der Formel I entspricht, isoliert wird.

Erhaltene Gemische der Diastereoisomeren und Gemische der Racemate können wegen der physikochemischen Unterschiede der Bestandteile in bekannter Weise in die reinen Isomeren, Diastereoisomeren oder Racemate getrennt werden, z. B. durch Chromatographie und/oder fraktionierte Kristallisation.

Die erhaltenen Racemate (racemische Diastereoisomere) können weiterhin in die optischen Antipoden nach an sich bekannten Methoden, beispielsweise durch Umkristallisieren aus einem optisch aktiven Lösungsmittel, mit Hilfe von Mikroorganismen oder durch Umsetzung eines sauren Endprodukts mit einer optisch aktiven Base, die Salze mit der racemischen Säure bildet, und Trennung der auf diese Weise erhaltenen Salze, beispielsweise aufgrund ihrer unterschiedlichen Löslichkeiten, in die Diastereoisomeren, aus denen die Antipoden durch Einwirken geeigneter Mittel freigesetzt werden können, aufgetrennt werden. Basische racemische Produkte können gleichfalls in die Antipoden, z. B. durch Trennung von deren diastereoisomeren Salzen, z. B. durch fraktionierte Kristallisation von deren d- oder ℓ-Tartraten, getrennt werden. Irgendwelche racemischen Zwischenprodukte oder Ausgangsmaterialien können in ähnlicher Weise getrennt werden.

Es wird der Antipode isoliert, der zu dem S,S-Stereoisomeren der Formel I führt oder ihm entspricht.

Schliesslich werden die erfindungsgemässen Verbindungen entweder in freier Form oder in Form von deren Salzen erhalten. Irgendeine erhaltene Base kann in ein entsprechendes Säureadditionssalz, vorzugsweise unter Verwendung einer pharmazeutisch verträglichen Säure oder eines Anionenaustausch-Präparates, übergeführt werden, oder erhaltene Salze können in die entsprechenden freien Basen, beispielsweise unter Verwendung einer stärkeren Base, wie eines Metall- oder Ammoniumhydroxids oder eines basischen Salzes, z. B. eines Alkalimetallhydroxids oder -carbonats, oder eines Kationenaustausch-Präparats, übergeführt werden. Eine Verbindung der Formel I, worin $COR^2$ und/oder $COR^3$ Carboxy bedeuten, kann somit auch in die entsprechenden Metall-oder Ammoniumsalze übergeführt werden. Diese oder andere Salze, beispielsweise die Pikrate, können auch für die Reinigung der erhaltenen Basen verwendet werden. Die Basen werden in die Salze übergeführt, die Salze werden getrennt, und die Basen werden aus den Salzen freigesetzt. Im Hinblick auf die enge Verwandtschaft zwischen den freien Verbindungen und den Verbindungen in Form ihrer Salze ist, wenn immer in diesem Zusammenhang auf eine Verbindung Bezug genommen wird, auch ein entsprechendes Salz mit umfasst, vorausgesetzt, dass dies unter den gegebenen Umständen möglich oder sinnvoll ist. Verbindungen der Formel I, worin $COR^2$ und/oder $COR^3$ Carboxy bedeuten, können als innere Salze vorliegen.

Die Verbindungen einschliesslich ihrer Salze können auch in Form ihrer Hydrate erhalten werden oder andere für die Kristallisation verwendete Lösungsmittel enthalten.

Die erfindungsgemässen pharmazeutischen Präparate sind solche, die für die enterale, wie die orale oder rektale, und die parenterale Verabreichung an Säuger, einschliesslich des Menschen, zur Behandlung oder Verhinderung von Erkrankungen geeignet sind, die auf die Inhibierung des Angiotensin-umwandelnden Enzyms ansprechen, z. B. von kardiovaskulären Erkrankungen, wie der Hypertension, und von kongestiver Herzinsuffizienz und umfassen eine wirksame Menge einer pharmakologisch aktiven Verbindung der Formel I oder eines pharmazeutisch verträglichen Salzes derselben, allein oder in Kombination mit einem oder mehreren pharmazeutisch verträglichen Trägern.

Die pharmakologisch aktiven Verbindungen der Erfindung sind bei der Herstellung von pharmazeutischen Zusammensetzungen verwendbar, die eine wirksame Menge derselben in Verbindung oder Beimischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z. B. Lactose, Dextrose, Sucrose, Mannit, Sorbit, Cellulose und/oder Glycin, b) Gleitmitteln, z. B. Siliciumdioxid, Talk, Stearinsäure, deren Magnesium- oder Calciumsalz und/oder Polyethylenglykol, für Tabletten auch c) Bindemitteln, z. B. Magnesiumaluminiumsilikat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z. B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare Präparate sind vorzugsweise wässrige isotonische Lösungen oder Suspensionen, und Suppositorien werden vorteilhaft aus Fettemulsionen oder -suspensionen hergestellt. Diese Zusammensetzungen können sterilisiert werden und/oder Adjuvantien, wie Konservierungs-, Stabilisierungs-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier- bzw. Ueberzugsmethoden hergestellt und enthalten etwa 0,1 bis 75 %, vorzugsweise etwa 1 bis 50 %, des aktiven Bestandteils. Eine Einheitsdosis für einen Säuger von etwa 50 bis 70 kg kann zwischen etwa 1 und 100 mg aktiven Bestandteil enthalten.

Die folgenden Beispiele sollen die Erfindung erläutern und keine Einschränkungen darstellen. Die Temperaturen sind in Celsiusgraden angegeben, und sämtliche Teile sind in Form von Gewichtsteilen angegeben. Wenn nicht anders erwähnt, werden sämtliche Verdampfungen unter vermindertem Druck vorzugsweise zwischen etwa $20.10^{-3}$ und $133.10^{-3}$ bar (zwischen etwa 15 und 100 mg Hg) durchgeführt.

Im Fall der Verbindungen der Formel I und von Derivaten, in denen mehr als ein Asymmetrie-Zentrum

vorliegt, werden die diastereoisomeren Verbindungen der Formel I als Isomer B in den betreffenden Beispielen bezeichnet. Die jeweiligen diastereoisomeren Verbindungen werden durch die physikalischen Eigenschaften, z. B. den Schmelzpunkt, die relative Wanderung bei der Chromatographie sowie die spektralen Eigenschaften bei der Infrarot- oder kernmagnetischen Resonanz-Messung charakterisiert.

Die Symbole A und B wurden wie folgt den jeweiligen Isomeren auf Basis ihrer relativen Wanderung bei der Chromatographie zugeordnet. Aus Basis der Wanderung bei der Dünnschichtchromatographie und Normalphasen-Hochdruck-Flüssigkeits-Chromatographie unter Verwendung von Siliciumdioxidgel als stationäre Phase wird das sich schnell bewegende Isomere als Isomer A und das sich langsam bewegende Isomere als Isomer B bezeichnet. Auf Basis der Wanderung bei der Hochdruck-Flüssigkeits-Chromatographie mit reverser Phase wird das sich langsam bewegende Isomere als Isomer A und das sich schnell bewegende Isomere als Isomer B bezeichnet.

## Beispiel 1

Eine Lösung von 3,77 g 3-[(5-Benzyloxycarbonylamino-1-carboxy)-(1S)-pentylamino]-1-carboxymethyl-2,3,4,5-tetrahydro-1H-1-(3S)-benzazepin-2-on (Isomer B) in 800 ml 80 %igem wässrigen Ethanol, das 0,5 g 10 % Palladium-auf-Kohle enthält, wird 40,5 Stunden bei einem Druck von 3 Atmosphären hydriert. Der Katalysator wird durch Vakuumfiltration über Celite abfiltriert, und der Filterkuchen wird intensiv mit Wasser gewaschen. Das Filtrat wird im Vakuum eingeengt. Toluol wird zugesetzt, und die Mischung wird erneut im Vakuum eingeengt. Dieser Vorgang wird einige Male wiederholt. Der erhaltene Festkörper wird bei 80°/0,05 mm Hg getrocknet. Man erhält das 3-[(5-Amino-1-carboxy)-(1S)-pentylamino]-1-carboxymethyl-2,3,4,5-tetrahydro-1H-1-(3S)-benzazepin-2-on Monohydrat, Fp. 179° (Zers.), $[\alpha]_D$ = — 174,2° (c = 1,0 in Wasser).

Das Ausgangsmaterial wird folgendermassen hergestellt: Eine Lösung von 8 g 3-Amino-1-ethoxycarbonylmethyl-2,3,4,5-tetrahydro-1H-1-benzazepin-2-on, 0,4 ml Essigsäure und 4,5 ml t-Butylnitrit in 160 ml Chloroform wird 2 Stunden unter Rückfluss erhitzt und auf Raumtemperatur abgekühlt. 6,0 g m-Chlorperbenzoesäure werden portionsweise unter Rühren zugegeben, und es wird während 30 Minuten weitergerührt. Die Lösung wird mit 100 ml gesättigter wässriger Natriumbicarbonatlösung, 50 ml 2N Salzsäure und 50 ml Wasser gewaschen und über Magnesiumsulfat getrocknet. Das Lösungsmittel wird unter vermindertem Druck abgezogen und der Rückstand mit Essigester/Petrolether (Kp 60-80°) behandelt. Man erhält als gelben Festkörper das α-Ketolactam 1-Ethoxycarbonylmethyl-2,3,4,5-tetrahydro-1H-1-benzazepin-2,3-dion, Fp. 108-110°, das ohne weitere Reinigung in der nächsten Stufe verwendet wird.

Zu einer Lösung von 11 g 1-Ethoxycarbonylmethyl-2,3,4,5-tetrahydro-1H-1-benzazepin-2,3-dion, 14 g N-ε-Benzyloxycarbonyllysinmethylesterhydrochlorid, 6 ml Triethylamin und 0,7 g Dibutylzinndichlorid in 600 ml Methylenchlorid werden 50 g 4 Å Molekularsieb gegeben. Der Ansatz wird 40 Stunden gerührt und unter Rückfluss gekocht. Nach Abkühlen auf Raumtemperatur wird der Ansatz durch Celite filtriert und das Lösungsmittel unter vermindertem Druck abgezogen. Der Rückstand wird in 750 ml Methanol und 28 ml Essigsäure aufgenommen. Nach 5 Minuten werden 3,6 g Natriumcyanborhydrid zugegeben und der Ansatz wird 72 Stunden bei Raumtemperatur gerührt. Dann wird durch Zugabe von 10 ml konzentrierter Salzsäure angesäuert. Die Lösung wird zur Trockne eingeengt. Man erhält ein Oel, das durch Blitzchromatographie unter Verwendung von Essigester/-Toluol (9 : 1) aufgetrennt wird. Es werden zwei Fraktionen erhalten:

a) 3-[(5-Benzyloxycarbonylamino-1-methoxycarbonyl)-pentylamino]-1-ethoxycarbonylmethyl-2,3,4,5-tetrahydro-1H-1-benzazepin-2-on (Isomer A); $R_f$=0,7, NMR ($CDCl_3$) 1,47 (m,5H) 3,16 (m,4H).

b) 3-[(5-Benzyloxycarbonylamino-1-methoxycarbonyl)-pentylamino]-1-ethoxycarbonylmethyl-2,3,4,5-tetrahydro-1H-1-benzazepin-2-on (Isomer B); $R_f$=0,6, NMR ($CDCl_3$) 1,30 (m,5H), 3,06 (m,4H).

Eine Lösung von 22,6 g 3-[(5-Benzyloxycarbonylamino-1-methoxycarbonyl)-(1S)-pentylamino]-1-ethoxycarbonylmethyl-2,3,4,5-tetrahydro-1H-1-(3S)-benzazepin-2-on (Isomer B) und 84 ml 1N Natronlauge in 580 ml Methanol wird 19 Stunden unter Stickstoffatmosphäre bei Raumtemperatur gerührt. Die Lösung wird eingeengt und der Rückstand zwischen 300 ml Wasser und 300 ml Ether verteilt. Die organische Schicht wird abgetrennt und die wässrige Schicht einmal mit 300 ml Ether gewaschen. Die wässrige Schicht wird dann mit konzentrierter Salzsäure auf pH 2 eingestellt, wonach das Produkt langsam auskristallisiert. Dieser Festkörper wird durch Vakuumfiltration gesammelt, intensiv mit Wasser gewaschen und getrocknet. Man erhält 3-[(5-Benzyloxycarbonylamino-1-carboxy)-(1S)-pentylamino]-1-carboxymethyl-2,3,4,5-tetrahydro-1H-1-(3S)-benzazepin-2-on, Fp. 216-218° (Zersetzung).

## Beispiel 2

Eine Lösung von 0,5 g 3-[(5-Benzyloxycarbonylamino-1-carboxy)-pentyl-amino]-1-carboxymethyl-2,3,4,5-tetrahydro-1H-1-benzazepin-2-on-hydrochlorid (Isomer B) in 200 ml Ethanol wird über 0,5 g 10 %igem Palladium-auf-Kohle Katalysator bei Raumtemperatur und atmosphärischen Druck bis zum Aufhören der Wasserstoffaufnahme hydriert. Der Katalysator wird abfiltriert und mit Wasser gewaschen.

Die vereinigten Filtrate werden eingedampft und das feste Material wird aus Methanol/Ether umkristallisiert. Man erhält das 3-[(5-Amino-1-carboxy)-pentylamino]-1-carboxymethyl-2,3,4,5-tetrahydro-1H-1-benzazepin-2-on-hydrochlorid (Isomer B). Fp. 148-150° (Zersetzung), $[\alpha]_D$=—112° (c=0,75 in Wasser). Diese Verbindung weist die S,S-Stereochemie auf. (Die freie Verbindung wird in den folgenden Beispielen als « Produkt » bezeichnet).

Der Ausgangsstoff wird wie folgt hergestellt: Zu einer Lösung von 1,5 g 3-[(5-Benzyloxycarbonylamino-1-methoxycarbonyl)-pentylamino]-1-ethoxycarbonylmethyl-2,3,4,5-tetrahydro-1H-1-benzazepin-2-on (Isomer A) in 60 ml Methanol werden bei 0° 5 ml 5 %iger Natronlauge zugegeben, und der Ansatz wird bei Raumtemperatur 18 Stunden gerührt. Die Lösung wird mit 2N Salzsäure angesäuert und zur Trockne eingeengt. 25 ml Ethanol werden zugegeben und die Lösung wird zur Trockne eingeengt. Der zurückbleibende weisse Festkörper wird mit 30 ml Methylenchlorid gerührt, und der zurückbleibende Feststoff abfiltriert. Dieses Material wird mit 30 ml Methylenchlorid gerührt und abfiltriert. Die vereinigten Methylenchloridlösungen werden eingedampft, der zurückbleibende Festkörper mit Ether behandelt und filtriert. Man erhält das 3-[(5-Benzyloxycarbonylamino-1-carboxy)-pentylamino]-1-carboxymethyl-2,3,4,5-tetrahydro-1H-1-benzazepin-2-on Hydrochlorid (Isomer A), Fp. 123-126°, $[\alpha]_D$=106° (c=1, Methanol).

Auf ähnliche Weise ergibt 3-[(5-Benzyloxycarbonylamino-1-methoxycarbonyl)-pentylamino]-1-ethoxycarbonylmethyl-2,3,4,5-tetrahydro-1H-1-benzazepin-2-on (Isomer B) das 3-[(5-Benzyloxycarbonylamino-1-carboxy)-pentylamino]-1-carboxymethyl-2,3,4,5-tetrahydro-1H-1-benzazepin-2-on Hydrochlorid (Isomer B), Fp. 107-110°, $[\alpha]_D$=— 88° (c=1.26, Methanol), dem die S,S-Stereochemie zuzuordnen ist.

## Beispiel 3

Eine Lösung von 1,5 g 3-[(5-Amino-1-methoxycarbonyl)-(1S)-pentylamino]-1-ethoxycarbonylmethyl-2,3,4,5-tetrahydro-1H-1-(3S)-benzazepin-2-on in 100 ml 2-normaler Salzsäure wird unter Stickstoff 18 Stunden unter Rückfluss gekocht. Das Reaktionsgemisch wird unter vermindertem Druck zur Trockene eingedampft und der Rückstand mit 10 ml Essigsäureethylester behandelt. Man erhält das Produkt als Dihydrochlorid.

Der Ausgangsstoff wird wie folgt hergestellt: Eine Lösung von 1,5 g 3-[(5-Benzyloxycarbonylamino-1-methoxycarbonyl)-(1S)-pentylamino]-1-ethoxycarbonylmethyl-2,3,4,5-tetrahydro-1H-1-(3S)-benzazepin-2-on in 250 ml Ethanol wird über 0,5 g 10 %-igem Palladium-auf-Kohle Katalysator bei Raumtemperatur und atmosphärischem Druck vollständig hydriert. Der Katalysator wird abfiltriert und mit 100 ml Ethanol, 100 ml 50 %-igem wässrigem Ethanol und 100 ml Wasser gewaschen. Die vereinigten Filtrate werden unter vermindertem Druck eingedampft und der erhaltene Feststoff mit Ether behandelt. Man erhält den Ausgangsstoff.

## Beispiel 4

Eine Lösung von 2 g 3-[(5-Amino-1-carboxy)-(1S)-pentylamino]-1-ethoxycarbonylmethyl-2,3,4,5-tetrahydro-1H-1-(3S)-benzazepin-2-on Hydrochlorid in 150 ml 2-normaler Salzsäure wird unter Stickstoff 18 Stunden unter Rückfluss gekocht. Das Reaktionsgemisch wird unter vermindertem Druck zur Trockene eingedampft und der Rückstand mit 15 ml Essigsäureethylester behandelt. Man erhält das Produkt als Dihydrochlorid.

Das Ausgangsmaterial wird wie folgt hergestellt:

Das für die Herstellung verwendete 3-[(5-Benzyloxycarbonylamino-1-t-butoxycarbonyl)-(1S)-pentylamino]-1-ethoxycarbonylmethyl-2,3,4,5-tetrahydro-1H-1-(3S)-benzazepin-2-on wird entsprechend der in Beispiel 1 beschriebenen Methode durch Kondensation von 1-Ethoxycarbonylmethyl-2,3,4,5-tetrahydro-1H-1-benzazepin-2,3-dion und N-ε-Benzyloxycarbonyllysin-t-butylester hergestellt.

Das dazu verwendete 1-Ethoxycarbonylmethyl-2,3,4,5-tetrahydro-1H-1benzazepin-2,3-dion kann auch folgendermassen hergestellt werden:

5,0 kg 3-Azido-2,3,4,5-tetrahydro-1H-1-benzazepin-2-on werden portionsweise unter Stickstoff zu 3,03 kg Kaliumtert.-butylat in 50 Litern trockenem Tetrahydrofuran so zugegeben, dass die Temperatur unter 5° bleibt. Der Ansatz wird nach beendeter Zugabe noch eine Stunde gerührt. Eine Lösung von 4,38 kg Bromessigsäureethylester in 5 Litern Tetrahydrofuran wird dann langsam so zugegeben, dass die Temperatur unter 5° bleibt. Der Ansatz wird dann über Nacht bei Raumtemperatur gehalten. 1,5 kg Hiflo-Filteraid wird hinzugegeben und der Ansatz wird filtriert. Der Filterkuchen wird mit Tetrahydrofuran gewaschen, die vereinigten Tetrahydrofuranlösungen werden zur Trockne eingeengt. Man erhält 3-Azido-1-ethoxycarbonylmethyl-2,3,4,5-tetrahydro-1H-1-benzazepin-2-on, das ohne weitere Reinigung für die nächste Stufe eingesetzt wird.

Eine Mischung von 13,98 kg 3-Azido-1-ethoxycarbonylmethyl-2,3,4,5-tetrahydro-1H-1-benzazepin-2-on und 1,3 kg 5 % Palladium-auf-Kohle in 57 Litern wasserfreiem Ethanol wird Stunden bei einem Druck von 3 Atmosphären Wasserstoff hydriert. Das Druckgefäss wird stündlich belüftet, um angesammelten Stickstoff entweichen zu lassen. Der Katalysator wird abfiltriert und mit Ethanol gewaschen. Die Lösung wird zur Trockne eingeengt. Man erhält 3-Amino-1-ethoxycarbonylmethyl-2,3,4,5-tetrahydro-1H-1-benzazepin-2-on.

31 ml t-Butylnitrit werden unter Rühren zu einer Lösung von 55 g 3-Amino-1-ethoxycarbonylmethyl-2,3,4,5-tetrahydro-1H-1-benzazepin-2-on in 1 000 ml Chloroform und 2,8 ml Essigsäure gegeben. Der Ansatz wird unter Stickstoff 3,5 Stunden unter Rückfluss gekocht und dann auf 0° abgekühlt. Unter Rühren werden während einer halben Stunde 43,5 g m-Chlorperbenzoesäure in 5 Portionen zugegeben. Man lässt den Ansartz auf Raumtemperatur kommen und weitere 1,5 Stunden rühren. Der Ansatz wird mit 500 ml gesättigter wässriger Natriumbicarbonatlösung, 2×250 ml konzentrierter wässriger Ammoniaklösung und 250 ml gesättigter wässriger Kochsalzlösung gewaschen. Die organische Lösung wird über Natriumsulfat getrocknet, mit Aktivkohle behandelt und unter vermindertem Druck eingeengt. Man erhält ein Oel, das mit Ether behandelt wird und so 1-Ethoxycarbonylmethyl-2,3,4,5-tetrahydro-1H-1-benzazepin-2,3-dion Fp. 112-114° ergibt.

Trockenes Chlorwasserstoffgas wird durch eine Lösung von 2,8 g 3-[(5-Benzyloxycarbonylamino-1-t-butoxycarbonyl)-(1S)-pentylamino]-1-ethoxycarbonylmethyl-2,3,4,5-tetrahydro-1H-1-(3S)-benzazepin-2-on in 150 ml Essigester eingeleitet, bis die dünnschichtchromatographische Analyse zeigt, dass kein Ausgangsmaterial zurückgeblieben ist. Das Lösungsmittel wird unter vermindertem Druck abgezogen und der Rückstand mit Ether behandelt. Man erhält das 3-[(5-Benzyloxycarbonylamino-1-carboxy)-(1S)-pentylamino]-1-ethoxycarbonylmethyl-2,3,4,5-tetrahydro-1H-1-(3S)-benzazepin-2-on Hydrochlorid, Fp. 124-126° (Zersetzung), $[\alpha]_D = -116°$ (c=1, Methanol).

Eine Lösung von 0,9 g 3-[(5-Benzyloxycarbonylamino-1-carboxy)-(1S)-pentylamino]-1-ethoxycarbonylmethyl-2,3,4,5-tetrahydro-1H-1-(3S)-benzazepin-2-on Hydrochlorid in 100 ml Ethanol wird bei Raumtemperatur und atmosphärischem Druck mit 0,5 g 5 %igem Palladium-auf-Kohle als Katalysator vollständig hydriert. Der Katalysator wird abfiltriert, das Lösungsmittel unter vermindertem Druck abgezogen. Man erhält das 3-[(5-Amino-1-carboxy)-(1S)-pentylamino]-1-ethoxycarbonylmethyl-2,3,4,5-tetrahydro-1H-1-(3S)-benzazepin-2-on Hydrochlorid, Fp. 123-125°, $[\alpha]_D = -141°$ (c=1 in Methanol).

## Beispiel 5

Herstellung des Produkts als Dihydrochlorid ausgehend von 3-[(5-Amino-1-methoxycarbonyl)-(1S)-pentylamino]-1-carboxymethyl-2,3,4,5-tetrahydro-1H-1-(3S)-benzazepin-2-on Hydrochlorid, wobei man das im Beispiel 3 beschriebene Verfahren verwendet.

Das Ausgangsmaterial wird wie folgt hergestellt: 3-[(5-Benzyloxycarbonylamino-1-methoxycarbonyl)-(1S)-pentylamino]-1-benzyloxycarbonylmethyl-2,3,4,5-tetrahydro-1H-1-(3S)-benzazepin-2-on Hydrochlorid, Fp. 61-63°, $[\alpha]_D = -58°$ (c=1, Methanol) wird nach den in den vorangegangenen Beispielen beschriebenen Methoden durch Kondensation von N-ε-Benzyloxycarbonyllysinmethylester und 1-Benzyloxycarbonylmethyl-2,3,4,5-tetrahydro-1H-1-benzazepin-2,3-dion, Fp. 106-108° hergestellt. Das α-Ketolactam wird unter Anwendung von vorstehend beschriebenen Verfahren durch Oxidation von 3-Amino-1-benzyloxycarbonylmethyl-2,3,4,5-tetrahydro-1H-1-benzazepin-2-on hergestellt.

Hydrierung von 3-[(5-Benzyloxycarbonylamino-1-methoxycarbonyl)-(1S)-pentylamino]-1-benzyloxycarbonylmethyl-2,3,4,5-tetrahydro-1H-1-(3S)-benzazepin-2-on Hydrochlorid gemäss Beispiel 2 ergibt den Ausgangsstoff.

## Beispiel 6

Herstellung des Produkts als Hydrochlorid ausgehend von 3-[(5-Benzyloxycarbonylamino-1-benzyloxycarbonyl)-(1S)-pentylamino]-1-carboxymethyl-2,3,4,5-tetrahydro-1H-1-(3S)-benzazepin-2-on, wobei man das Verfahren gemäss Beispiel 1 verwendet. Der Ausgangsstoff wird durch Hydrolyse des entsprechenden Ethylesters hergestellt (s. Beispiel 3).

## Beispiel 7

Herstellung des Produkts als Hydrochlorid ausgehend von 3-[(5-Benzyloxycarbonylamino-1-carboxy)-(1S)-pentylamino]-1-benzyloxycarbonylmethyl-2,3,4,5-tetrahydro-1H-1-(3S)-benzazepin-2-on Hydrochlorid, wobei man das im Beispiel 2 beschriebene Verfahren verwendet. Der Ausgangsstoff wird durch Hydrolyse des entsprechenden Ethylesters hergestellt.

## Beispiel 8

Herstellung des Produkts ausgehend von 3-[(5-Benzyloxycarbonylamino-1-benzyloxycarbonyl)-(1S)-pentylamino]-1-benzyloxycarbonylmethyl-2,3,4,5-tetrahydro-1H-1-(3S)-benzazepin-2-on, wobei man das im Beispiel 1 beschriebene Verfahren verwendet. Der Ausgangsstoff wird gemäss Beispiel 1 hergestellt.

## Beispiel 9

Eine Lösung von 2,0 g 3-[(5-Benzyloxycarbonylamino-1-ethoxycarbonyl)-(1S)-pentylamino]-1-benzyloxycarbonylmethyl-2,3,4,5-tetrahydro-1H-1-(3S)-benzazepin-2-on Hydrochlorid in 150 ml absolutem Ethanol wird unter Verwendung von 1,0 g 10 %igem Palladium-auf-Kohle als Katalysator erschöpfend hydriert. Der Katalysator wird abfiltriert, die Lösungsmittel werden unter vermindertem Druck abgezogen. Der

Rückstand wird mit Ether behandelt. Man erhält 3-[(5-Amino-1-ethoxycarbonyl)-(1S)-pentylamino]-1-carboxymethyl-2,3,4,5-tetrahydro-1H-1-(3S)-benzazepin-2-on Hydrochlorid, Fp. 85-87°, $[\alpha]_D = -149°$ (c=1, Methanol).

Wenn die Hydrogenolyse in Gegenwart eines weiteren Mol-Aequivalent Chlorwasserstoff durchgeführt wird, wird das entsprechende Dihydrochlorid erhalten, Fp. 203-205°, $[\alpha]_D = -143°$ (c=1.3, Ethanol).

Beispiel 10

Das folgende Verfahren wird für die selektive Hydrolyse von Diestern zu der entsprechenden 1-Carboxymethylverbindung verwendet:

Der Diester wird in Ethanol gelöst und mit einem Mol-Aequivalent Kalilauge behandelt, der Ansatz wird bei Raumtemperatur 1 Stunde gerührt. Das Lösungsmittel wird unter vermindertem Druck abgezogen, und Wasser wird zu dem Rückstand gegeben. Die wässrige Lösung wird mit Ether gewaschen, mit Salzsäure angesäuert, um die freie Aminosäure herzustellen, die durch Extraktion isoliert und in das Hydrochlorid überführt wird.

Die folgenden Verbindungen werden hergestellt:

a) 3-[(5-Benzyloxycarbonylamino-1-methoxycarbonyl)-(1S)-pentylamino]-1-carboxymethyl-2,3,4,5-tetrahydro-1H-1-(3S)-benzazepin-2-on Hydrochlorid, Fp. 102-104°, $[\alpha]_D = -116°$ (c=1, Methanol) durch Hydrolyse des entsprechenden 1-Ethoxycarbonylmethylderivates.

b) 3-[(5-Benzyloxycarbonylamino-1-ethoxycarbonyl)-(1S)-pentylamino]-1-carboxymethyl-2,3,4,5-tetrahydro-1H-1-(3S)-benzazepin-2-on Hydrochlorid, Fp. 104-106°, $[\alpha]_D = -113°$ (c=1, Methanol), durch Hydrolyse des entsprechenden 1-Ethoxycarbonylmethylderivates.

Diese Verbindungen werden nach der Methode des Beispiels 2 in die entprechenden Verbindungen mit freier Aminopentylgruppe übergeführt.

Beispiel 11

Eine Lösung von 1,3 g 1-Ethoxycarbonylmethyl-3-(2-oxo-2,3,4,5,6,7-hexahydro-1H-(3S)-azepin-3-yl)-amino-2,3,4,5-tetrahydro-1H-1-(3S)-benzazepin-2-on in 150 ml 6-normaler Salzsäure wird 43 Stunden unter Rückfluss gekocht. Das Reaktionsgemisch wird unter vermindertem Druck zur Trockene eingedampft, und der Rückstand 5 Minuten mit 50 ml Essigsäureethylester gekocht. Nach Abkühlen auf Raumtemperatur wird der Feststoff abfiltriert und 5 Minuten mit 5 ml Essigsäureethylester gekocht. Der Feststoff wird abfiltriert. Man erhält das Produkt als Dihydrochlorid.

Der Ausgangsstoff wird durch Umsetzung von 1-Ethoxycarbonylmethyl-2,3,4,5-tetrahydro-1H-1-benzazepin-2,3-dion mit 3-(S)-Aminocaprolactam, wie im Beispiel 1 beschrieben, hergestellt.

Beispiel 12

In eine Lösung von 2,1 g 3-[(5-Amino-1-t-butoxycarbonyl)-(1S)-pentylamino]-1-carboxymethyl-2,3,4,5-tetrahydro-1H-1-(3S)-benzazepin-2-on in 75 ml Ethanol wird Chlorwasserstoffgas eingeleitet, wobei man die Lösung bei Raumtemperatur hält. Der erhaltene Feststoff wird abfiltriert, im Hochvakuum getrocknet und mit 10 ml Essigsäureethylester (10 ml) behandelt. Man erhält das Produkt als Dihydrochlorid.

Der Ausgangsstoff wird durch Umsetzung von 1-Benzyloxycarbonylmethyl-2,3,4,5-tetrahydro-1H-1-benzazepin-2,3-dion mit (S)-N-ε-Benzyloxycarbonyl-lysin-t-butylester und nachfolgende Hydrierung der erhaltenen Verbindung gemäss Beispiel 1 hergestellt.

Beispiel 13

Eine Lösung von 0,7 N-α-[1-Carboxy-3-(2-carboxymethylaminophenyl)-propyl]-(S)-lysin und 0,4 g Dicyclohexylcarbodiimid in 15 ml Dimethylformamid wird 18 Stunden bei Raumtemperatur gehalten. Das Reaktionsgemisch wird filtriert und das Filtrat unter vermindertem Druck zur Trockene eingedampft. Der Rückstand wird mit 10 ml Wasser gerührt und filtriert. Diese Prozedur wird mit 2 ×10 ml Wasser wiederholt. Die vereinigten wässrigen Lösungen werden mit 3×50 ml Essigsäureethylester extrahiert und dann mit überschüssiger 2-normaler Salzsäure angesäuert. Die Lösung wird unter vermindertem Druck eingedampft und der Rückstand mit 2×10 ml Essigsäureethylester behandelt. Man erhält das Produkt als Dihydrochlorid.

Der Ausgangsstoff wird gemäss der folgenden Reaktions-Sequenz hergestellt:

Eine Lösung von 33,2 g Acetylaminomalonsäurediethylester in 150 ml Ethanol wird zu einer Lösung von Natriumethanolat in Ethanol, die aus 3,9 g Natrium und 200 ml Ethanol hergestellt ist, gegeben. Der Ansatz wird 30 Minuten bei Raumtemperatur gerührt. Eine Lösung von 40,0 g 2-Nitrophenethylbromid [J. Med. Chem. 20, 1020 (1977)] in 100 ml Ethanol wird während 20 Minuten zugetropft. Nach beendeter Zugabe wird der Ansatz 18 Stunden unter Rückfluss gekocht, dann auf Raumtemperatur abgekühlt und unter vermindertem Druck eingeengt. Der Rückstand wird in 350 ml Wasser aufgenommen und die Lösung mit 2 ×350 ml Essigester extrahiert. Die vereinigten Essigesterextrakte werden mit 200 ml Wasser

gewaschen und über Magnesiumsulfat getrocknet. Nach Abziehen des Lösungsmittels unter vermindertem Druck erhält man den 2-Acetylamino-2-(2-nitrophenethyl)-malonsäurediethylester als niedrigschmelzenden Feststoff, der ohne weitere Reinigung für die nächste Stufe eingesetzt wird.

Eine Lösung von 80 g 2-Acetylamino-2-(2-nitrophenethyl)-malonsäurediethylester in 900 ml 3-normaler Salzsäure wird 12 Stunden unter Rückfluss gekocht. Die Lösung wird abgekühlt und mit 200 ml Essigester extrahiert. Die wässrige Lösung wird filtriert und unter vermindertem Druck zur Trockne eingeengt. Der Rückstand wird aus Ethanol/Ether umkristallisiert. Man erhält das 2-Amino-4-(2-nitrophenyl)-buttersäurehydrochlorid, Fp. 219-221° (Zersetzung).

Eine Lösung von 38,0 g 2-Amino-4-(2-nitrophenyl)-buttersäurehydrochlorid in 1 200 ml 10 %igem ethanolischen Chlorwasserstoff wird unter Rühren 18 Stunden unter Rückfluss gekocht. Der Ansatz wird unter vermindertem Druck zur Trockne eingeengt, 250 ml Wasser werden zugegeben, und die wässrige Lösung wird durch Zugabe von 2N Natronlauge basisch gemacht. Die Lösung wird mit 2×500 ml Dichlormethan extrahiert, und die vereinigten Dichlormethanlösungen werden mit 2×150 ml Wasser gewaschen und über wasserfreiem Magnesiumsulfat getrocknet. Einengen ergibt den 2-Amino-4-(2-nitrophenyl)-buttersäureethylester, der ohne weitere Reinigung für die nächste Stufe verwendet wird.

Eine Lösung von 17,4 g 2-Amino-4-(2-nitrophenyl)-buttersäureethylester in 130 ml 50 %igem wässrigem Dioxan wird mit 10,5 g Triethylamin und 18,7 g 2-(tert.-Butoxycarbonyloxyimino)-2-phenylacetonitril versetzt. Das Reaktionsgemisch wird bei Raumtemperatur 4 Stunden gerührt und dann mit 300 ml Wasser verdünnt. Das Gemisch wird mit 2×150 ml Ether extrahiert, die wässrige Phase mit eiskalter, 2-normaler Salzsäure angesäuert und mit 2×250 ml Essigsäureethylester extrahiert. Die Essigsäureethylester-Phasen werden vereinigt, mit 150 ml Wasser gewaschen und über Natriumsulfat getrocknet. Das Lösungsmittel wird unter vermindertem Druck abgedampft. Man erhält den 2-t-Butoxycarbonylamino-4-(2-nitrophenyl)-buttersäureethylester, welcher ohne weitere Reinigung verwendet wird.

Eine Lösung von 13,0 g 2-t-Butoxycarbonylamino-4-(2-nitrophenyl)-buttersäureethylester in 300 ml Ethanol wird bei Raumtemperatur und atmosphärischem Druck mit 1 g 10 %igem Palladium-auf-Kohle als Katalysator bis zum Abschluss der Wasserstoffaufnahme hydriert. Der Katalysator wird abfiltriert und das Lösungsmittel abgedampft. Man erhält den 2-t-Butoxycarbonylamino-4-(2-aminophenyl)-buttersäureethylester, der ohne weitere Reinigung im folgenden Schritt verwendet wird.

Eine Lösung von 10,0 g 2-t-Butoxycarbonylamino-4-(2-aminophenyl)-buttersäureethylester und 4,2 g Glyoxylsäureethylester in 120 ml Ethanol wird bei 80° und 3 Atmosphären 72 Stunden mit 3 g 10 %igem Palladium-auf-Kohle als Katalysator hydriert. Das Reaktionsgemisch wird auf Raumtemperatur abgekühlt und der Katalysator abfiltriert. Das Lösungsmittel wird unter vermindertem Druck abgedampft und der Rückstand zwischen 150 ml Essigsäureethylester und 75 ml Wasser verteilt. Die organische Phase wird über Natriumsulfat getrocknet und das Lösungsmittel unter vermindertem Druck abgedampft. Man erhält den 2-t-Butoxycarbonylamino-4-[2-(ethoxycarbonylmethylamino)-phenyl]-buttersäureethylester, der ohne weitere Reinigung im nächsten Schritt verwendet wird.

Man leitet Chlorwasserstoffgas 30 Minuten bei Raumtemperatur durch eine Lösung von 8,5 g 2-t-Butoxycarbonylamino-4-[2-(ethoxycarbonylmethylamino)-phenyl]-buttersäureethylester in 150 ml Essigsäureethylester ein. Die Lösung wird unter vermindertem Druck eingedampft und der Rückstand in 100 ml Essigsäureethylester gelöst. Die Lösung wird mit 3 ×100 ml Wasser gewaschen und über Natriumsulfat getrocknet. Das Lösungsmittel wird unter vermindertem Druck abgedampft. Man erhält den 2-Amino-4-[2-(ethoxycarbonylmethylamino)-phenyl]-buttersäureethylester, der ohne weitere Reinigung im nächsten Schritt verwendet wird.

Das erhaltene Produkt wird mit α-Oxo-ε-benzyloxycarbonylamino-hexansäuremethylester (Tetrahedron Letters 1982, 1875) nach dem in Beispiel 1 beschriebenen Verfahren umgesetzt. Hydrolyse der Estergruppe gemäss Beispiel 3, gefolgt von einer Hydrierung gemäss Beispiel 1 ergibt das Ausgangsmaterial für dieses Beispiel.

### Beispiel 14

Eine Lösung von 0,9 g 3-(4-Cyan-1-carboxy)-(1S)-butylamino]-1-carboxymethyl-2,3,4,5-tetrahydro-1H-1-(3S)-benzazepin-2-on Hydrochlorid in 75 ml 10 %igem ethanolischen Ammoniak wird in einem Faver-Apparat, über 1 g 5 %igem Rhodium-auf-Aluminiumoxid, bei 2,5 Atmosphären vollständig hydriert. Der Katalysator wird abfiltriert und das Filtrat unter vermindertem Druck zur Trockene eingedampft. Der Rückstand wird in 2-normaler Salzsäure gelöst, erneut eingedampft und dann mit 10 ml Essigsäureethylester behandelt. Man erhält das Produkt als Dihydrochlorid.

Der Ausgangsstoff wird wie folgt hergestellt: Zuerst wird γ-Cyan-L-α-aminovaleriansäure gemäss I. Mezo et al., Acta Chim. Acad. Sci. Hung. 85, 201 (1975) hergestellt. Diese Verbindung wird durch Umsetzung mit Ethyliodid in Gegenwart von Natriumbicarbonat in Dimethylacetamid in den Ethylester überführt. Dieses Material wird mit 1-Ethoxycarbonylmethyl-2,3,4,5-tetrahydro-1H-1-benzazepin-2,3-dion gemäss Beispiel 1 umgesetzt und gemäss Beispiel 2 zur Cyan-Disäure hydrolysiert.

### Beispiel 15

Eine Lösung von 1,3 g 3-[(1-Carboxy-5-nitro)-(1S)-pentylamino]-1-carboxymethyl-2,3,4,5-tetrahydro-1H-1-(3S)-benzazepin-2-on Hydrochlorid in 100 ml Ethanol wird über 0,5 g Platinoxid bei Raumtemperatur und atmosphärischem Druck vollständig hydriert. Der Katalysator wird abfiltriert und 25 ml 2-normale Salzsäure werden zugegeben. Die Lösung wird unter vermindertem Druck zur Trockne eingedampft und der erhaltene Feststoff mit 5 ml Essigsäureethylester behandelt. Man erhält das Produkt als Dihydrochlorid.

Der Ausgangsstoff wird wie folgt erhalten: Zuerst wird die L-6-Nitro-2-amino-hexansäure gemäss der Methode von E. Bayer und K. Schmidt, Tetrahedron Letters 2051 (1973) hergestellt. Diese Substanz wird in der gleichen Weise wie die Cyan-Disäure im Beispiel 14 weiter verarbeitet.

## Beispiel 16

Herstellung des Produkts als Dihydrochlorid ausgehend von 3-[(5-Acetylamino-1-carboxy)-(1S)-pentylamino]-1-carboxymethyl-2,3,4,5-tetrahydro-1H-1-(3S)-benzazepin-2-on, wobei man das im Beispiel 11 beschriebene Verfahren anwendet.

Der Ausgangsstoff wird durch vollständige Hydrierung einer Lösung von 3-[(1-Carboxy-4-cyan-(1S)-butylamino]-1-carboxymethyl-2,3,4,5-tetrahydro-1H-1-(3S)-benzazepin-2-on (Herstellung s. Beispiel 14) über Raney-Nickel [hergestellt gemäss F.E. Gould et al., J. Org. Chem. 25, 1658 (1960)], bei Raumtemperatur und 3,4 Atmosphären hergestellt.

## Beispiel 17

Herstellung des Produkts als Dihydrochlorid ausgehend von 3-[(5-Amino-1-carboxy)-(1S)-pentylamino]-1-cyanmethyl-2,3,4,5-tetrahydro-1H-1(3S)-benzazepin-2-on gemäss dem Verfahren des Beispiels 11.

Der Ausgangsstoff wird wie folgt erhalten: Zuerst wird das 3-Azido-2,3,4,5-tetrahydro-1H-1-benzazepin-2-on mit Bromacetonitril alkyliert und dann gemäss dem im Beispiel 4 beschrieben Verfahren zum 3-Amino-nitril reduziert. Die Oxidation zum 1-Cyanmethyl-2,3,4,5-tetrahydro-1H-1-benzazepin-2,3-dion wird gemäss dem im Beispiel 1 gegebenen Verfahren durchgeführt. Kondensation mit ε-Benzyloxycarbonyl-L-lysin-ethylester, Abspaltung der Benzyl-oxycarbonylgruppe und Hydrolyse des Esters werden gemäss den in den Beispielen 1 und 3 angegebenen Verfahren vorgenommen.

## Beispiel 18

In eine Lösung von 0,6 g 3-[(1-Carboxy)-(1S)-4-pentenylamino]-1-carboxymethyl-2,3,4,5-tetrahydro-1H-1-(3S)-benzazepin-2-on Hydrochlorid in 75 ml Tetrahydrofuran werden unter Rühren in einer trockenen Stickstoffatmosphäre 0,6 ml einer Lösung von Boran in Tetrahydrofuran (1,0 M) mit einer Spritze eingetragen. Das Reaktionsgemisch wird eine Stunde bei Raumtemperatur gehalten, dann mit 1 ml Wasser, und nachfolgend mit 3 ml 3-normaler Natronlauge und mit 4 ml frisch hergestellter 0,31-molarer Chloraminlösung versetzt. Das Gemisch wird eine Stunde bei Raumtemperatur gehalten und dann mit 2-normaler Salzsäure angesäuert. Der erhaltene Niederschlag wird abfiltriert, mit 3 × 10 ml Essigsäurethylester/-Tetrahydrofuran (1 : 1) gewaschen und mit Essigsäureethylester behandelt. Man erhält das Produkt als Dihydrochlorid.

Der Ausgangsstoff wird wie folgt hergestellt: 2-Amino-5-hexensäure wird gemäss der Methode von Smith und Drinkwater [J. Chem. Soc. 1305 (1971)] hergestellt. Diese Verbindung wird durch Umsetzung mit Ethyliodid in Gegenwart von Natriumbicarbonat in Dimethylacetamid verestert und gemäss der im Beispiel 1 beschriebenen Methode mit 1-Ethoxycarbonylmethyl-2,3,4,5-tetrahydro-1H-1-benzazepin-2,3-dion kondensiert. Die Hydrolyse des Ethylesters gemäss dem Verfahren des Beispiels 3 ergibt den Ausgangsstoff dieses Beispiels.

## Beispiel 19

Eine Lösung von 0,6 g 1-Ethoxycarbonylmethyl-3-(2-oxo-2,3,4,5,6,7-hexahydro-1H-(3S)-azepin-3-yl)-amino-2,3,4,5-tetrahydro-1H-1-(3S)-benzazepin-2-on in 20 ml 6N Salzsäure wird unter Rückfluss über Nacht erhitzt. Die Lösung wird abgekühlt und zur Trockne eingeengt. Man erhält das 3-[(5-Amino-1-carboxy)-(1S)-pentylamino]-1-carboxymethyl-2,3,4,5-tetrahydro-1H-1-(3S)-benzazepin-2-on Dihydrochlorid, $[\alpha]_D = -136{,}9^\circ$ (1 % in 1N Salzsäure).

Das Dihydrochlorid des Produktes wird in das Produkt überführt, indem man es in Ethanol über Nacht bei Raumtemperatur mit Propylenoxid behandelt.

Das Ausgangsmaterial wird wie folgt hergestellt:

Eine Lösung von 15,3 g 3-Brom-1-ethoxycarbonylmethyl-2,3,4,5-tetrahydro-1H-1-benzazepin-2-on und 30 g 3-(S)-ε-Aminocaprolactam in 400 ml Acetonitril wird unter Rückfluss 48 Stunden erhitzt. Der Ansatz wird auf Raumtemperatur abgekühlt, filtriert, und das Filtrat wird zur Trockne eingeengt. Der Rückstand wird in 200 ml Methylenchlorid aufgenommen, die Methylenchloridlösung mit 2×200 ml Wasser gewaschen und dann mit 2×100 ml 2N Salzsäure extrahiert. Der saure Extrakt wird durch Zugabe von festem Kaliumcarbonat auf pH 8 gebracht. Extraktion mit Methylenchlorid ergibt 1-Ethoxycarbonylmethyl-3-(2-oxo-2,3,4,5,6,7-hexahydro-1H-(3S)-azepin-3-yl)-amino-2,3,4,5-tetrahydro-1H-1-benzazepin-2-on

als Mischung von Diastereoisomeren. Das rohe Produkt wird aus Methanol umkristallisiert. Man erhält 1-Ethoxycarbonylmethyl-3-(2-oxo-2,3,4,5,6,7-hexahydro-1H-(3S)-azepin-3-yl)-amino-2,3,4,5-tetrahydro-1H-1-(1S)-benzazepin-2-on, Fp. 148-150°.

## Beispiel 20

Eine Mischung von 1,0 g 3-[(5-Amino-1-carboxy)-(1S)-pentylamino]-1-carboxymethyl-2,3-dihydro-1H-1-(3S)-benzazepin-2-on und 120 mg Palladium in 35 ml absolutem Ethanol wird bei 3 Atmosphären Druck hydriert, bis 1 Mol-Aequivalent Wasserstoff verbraucht ist. Der Katalysator wird entfernt, der Ansatz wird zur Trockne eingeengt. Man erhält 3-[(5-Amino-1-carboxy)-(1S)-pentylamino]-1-carboxymethyl-2,3,4,5-tetrahydro-1H-1-(3S)-benzazepin-2-on.

Das Ausgangsmaterial wird wie folgt hergestellt:

Eine Lösung von 0,4 g 3-(S)-t-Butoxycarbonylamino-2,3,4,5-tetrahydro-1H-1-benzazepin-2,5-dion [hergestellt aus L-Kynurenin gemäss Australian J. Chemistry 33, 633-640 (1980)] und 0,23 g Bromessigsäureethylester in 30 ml trockenem Tetrahydrofuran wird bei 0° in einer trockenen Stickstoffatmosphäre gerührt. 0,254 g Kalium-t-butoxid werden in einer Portion zugegeben. Nach 1 Stunde bei 0° werden weitere 0,23 g Bromessigsäureethylester zugegeben und bei 0° eine weitere Stunde gerührt. Das Reaktionsgemisch wird mit 100 ml Wasser versetzt und mit 2×50 ml Essigsäureethylester extrahiert. Die vereinigten Extrakte werden mit 100 ml Wasser gewaschen, über Magnesiumsulfat getrocknet und unter vermindertem Druck eingedampft. Man erhält ein gelbes gummiartiges Material, welches nach Behandeln mit Ether/Petrolether (Kp. 30-60°) 3-(S)-t-Butoxycarbonylamino-1-ethoxycarbonylmethyl-2,3,4,5-tetrahydro-1H-1-benzazepin-2,3-dion, Fp. 86-88°, $[\alpha]_D$=— 203° (c=1 in Dimethylformamid) ergibt.

Eine Lösung von 0,14 g 3-(S)-t-Butoxycarbonylamino-1-ethoxycarbonylmethyl-2,3,4,5-tetrahydro-1H-1-benzazepin-2,5-dion und 7 mg Natriumborhydrid (7 mg) in 10 ml Ethanol wird 18 Stunden bei Raumtemperatur gerührt. Das Ethanol wird unter vermindertem Druck abgedampft, und der Rückstand in 25 ml Dichlormethan gelöst. Die Lösung wird mit 2×20 ml 2-normaler Salzsäure und mit 20 ml gesättigter wässriger Natriumchloridlösung extrahiert und über Natriumsulfat getrocknet. Das Lösungsmittel wird unter vermindertem Druck abgedampft und der Rückstand mit Ether behandelt. Man erhält das 3-(S)-t-Butoxycarbonylamino-1-ethoxycarbonylmethyl-5-hydroxy-2,3,4,5-tetrahydro-1H-1-benzazepin-2-on, Fp. 167-169,5°, $[\alpha]_D$=— 193° (c=0,52 in Dimethylformamid). Diese Verbindung wird auch durch Hydrierung des Benzazepin-2,5-dion-Derivates mit $H_2$/Pt in Ethanol erhalten.

Eine Lösung von 0,75 g Methansulfonylchlorid in 2 ml Methylenchlorid wird bei —5 bis —10° zu einer Lösung von 3-(S)-t-Butoxycarbonylamino-1-ethoxycarbonylmethyl-5-hydroxy-2,3,4,5-tetrahydro-1H-1-benzazepin-2-on in 25 ml Methylenchlorid gegeben. Innerhalb von 10 Minuten wird bei 0-5° eine Lösung von 1,33 g Triethylamin in 8 ml Methylenchlorid zugegeben. Die erhaltene Lösung wird bei Raumtemperatur 3 Tage gerührt und dann mit 0,2N Salzsäure, Wasser und verdünnter Natriumbicarbonatlösung gewaschen. Die Methylenchloridlösung wird über Natriumsulfat getrocknet und zur Trockne eingeengt. Man erhält 3-(S)-t-Butoxycarbonylamino-1-ethoxycarbonylmethyl-2,3-dihydro-1H-1-benzazepin-2-on.

Während 1 Stunde wird Chlorwasserstoffgas durch eine Lösung des obigen Materials in Essigester geleitet. Danach wird für 30 Minuten Stickstoff durch diese Lösung geleitet. Die Essigesterlösung wird mit 30 ml Wasser und 30 ml 1N Salzsäure gewaschen. Die Essigesterphase wird abgetrennt und die wässrigen Phasen vereinigt. Die wässrige Phase wird mit verdünntem Ammoniumhydroxyd auf pH 9 eingestellt, mit Essigester extrahiert, die organischen Phasen werden vereint, über Natriumsulfat getrocknet und eingedampft. Man erhält nach Reinigung das 3-(S)-Amino-1-ethoxycarbonylmethyl-2,3-dihydro-1H-1-benzazepin-2-on.

Kondensation von 6-Benzyloxycarbonylamino-2-oxo-hexansäure in der Gegenwart von Natriumcyanborhydrid mit 3-(S)-Amino-1-ethoxycarbonylmethyl-2,3-dihydro-1H-1-benzazepin-2-on erbigt nach Isomerentrennung das 3-[(5-Benzyloxycarbonylamino-1-carboxy)-(1S)-pentylamino]-1-ethoxycarbonylmethyl-2,3-dihydro-1H-1-(3S)-benzazepin-2-on.

Behandlung mit Bromwasserstoffsäure in Essigsäure, Hydrolyse mit 3N Salzsäure und Umwandlung in die freie Aminosäure ergibt 1-Carboxymethyl-3-[(5-amino-1-carboxy)-(1S)-pentylamino]-2,3-dihydro-1H-1-(3S)-benzazepin-2-on.

## Beispiel 21

Eine Mischung von 1,0 g 3-[(5-Amino-1-carboxy)-(1S)-pentylamino]-1-carboxymethyl-5-chlor-2,3,4,5-tetrahydro-1H-1-(3S)-benzazepin-2-on und 150 mg Palladium in 35 ml absolutem Ethanol wird bei 3 Atmosphären Druck hydriert, bis 1 Mol-Aequivalent Wasserstoff verbraucht ist. Der Katalysator wird abfiltriert und die Lösung zur Trockne eingeengt. Man erhält das 3-[(5-Amino-1-carboxy)-(1S)-pentylamino]-1-carboxymethyl-2,3,4,5-tetrahydro-1H-1-(3S)-benzazepin-2-on Hydrochlorid.

Das Ausgangsmaterial wird wie folgt hergestellt:

Eine Lösung von 1,69 g Thionylchlorid in 5 ml Methylenchlorid wird zu einer Lösung von 4,8 g 3-(S)-t-Butoxycarbonylamino-1-ethoxycarbonylmethyl-5-hydroxy-2,3,4,5-tetrahydro-1H-1-benzazepin-2-on und 2,0 g Diisopropylethylamin in 45 ml Methylenchlorid bei —5 bis —10° innerhalb von 15 Minuten

gegeben. Der Ansatz wird eine Stunde bei —5° gerührt, mit 5 %iger Kaliumcarbonatlösung gewaschen, getrocknet und zur Trockne eingeengt. Man erhält die 5-Chlorverbindung.

Entsprechend den Verfahren, die in den vorstehenden Beispielen beschrieben sind, wird 3-(S)-t-Butoxycarbonylamino-1-ethoxycarboxylmethyl-5-chlor(2,3,4,5-tetrahydro-1H-1-benzazepin-2-on in 3-[(5-Amino-1-carboxy)-(1S)-pentylamino]-1-carboxymethyl-5-chlor-2,3,4,5-tetrahydro-1H-1-(3S)-benzazepin-2-on überführt.

Beispiel 22

Herstellung von 10,000 Kapseln mit einem Gehalt von je 10 mg der aktiven Substanz.

Zusammensetzung

| | |
|---|---|
| 3-[(5-Amino-1-carboxy)-(1S)-pentylamino]-1-carboxymethyl-2,3,4,5-tetrahydro-1H-1-(3S)-benzazepin-2-on | 100.00 g |
| Milchzucker | 1 900.0 g |

Verfahren

Die pulvrigen Bestandteile werden mit einem Sieb von 0,6 mm Maschenweite gesiebt. Dann wird der Wirkstoff in einen geeigneten Mixer gegeben und mit dem Milchzucker homogen vermischt. Kapseln Nr. 3 werden mit je 200 mg des Gemisches mit einer Füllmaschine gefüllt.

**Patentansprüche** (für die Vertragsstaaten : BE, CH, DE, FR, GB, IT, LI, LU, NL, SE)

1. Verbindungen der allgemeinen Formel

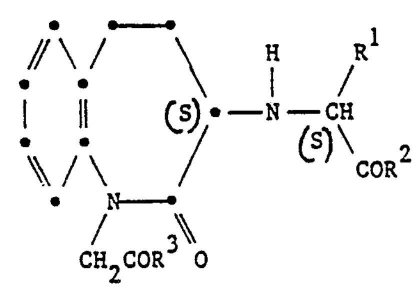

(I)

worin. $R^1$ 4-Aminobutyl bedeutet, $R^2$ und $R^3$, unabhängig voneinander, Hydroxy oder Niederalkoxy bedeuten und S die Chiralität bedeutet, oder deren Salze.

2. Verbindungen der im Anspruch 1 gezeigten Formel I, worin $R^1$ 4-Aminobutyl bedeutet, und $R^2$ und $R^3$ unabhängig voneinander Hydroxy, Methoxy, Ethoxy oder tert.-Butoxy bedeuten, oder Salze davon.

3. Verbindungen der im Anspruch 1 gezeigten Formel I, worin $R^1$ 4-Aminobutyl bedeutet, $R^2$ Hydroxy, Methoxy oder tert.-Butoxy und $R^3$ Hydroxy oder Ethoxy bedeuten, oder Salze davon.

4. 3-[(5-Amino-1-carboxy)-(1S)-pentylamino]-1-carboxymethyl-2,3,4,5-tetrahydro-1H-1-(3S)-benzazepin-2-on oder seine Salze.

5. Das Hydrochlorid und das Dihydrochlorid von 3-[(5-Amino-1-carboxy)-(1S)-pentylamino]-1-carboxymethyl-2,3,4,5-tetrahydro-1H-1-(3S)-benzazepin-2-on.

6. Pharmazeutische Präparate enthaltend eine Verbindung gemäß einem der Ansprüche 1 bis 5 oder ein pharmazeutisch annehmbares Salz davon zusammen mit einem pharmazeutisch geeigneten Trägermaterial.

7. Eine in einem der Ansprüche 1 bis 5 genannte Verbindung oder ein pharmazeutisch annehmbares Salz davon zur Anwendung in einem Verfahren zur therapeutischen Behandlung des menschlichen oder tierischen Körpers.

8. Eine in einem der Ansprüche 1 bis 5 genannte Verbindung oder ein pharmazeutisch annehmbares Salz davon des Angiotensin-umwandelnden Enzyms.

9. Verwendung einer der in den Ansprüchen 1 bis 5 genannten Verbindungen oder eines pharmazeutisch annehmbaren Salzes davon zur Herstellung von pharmazeutischen Präparaten.

10. Verfahren zur Herstellung einer in Anspruch 1 genannten Verbindung der Formel I, in der alle Symbole die in Anspruch 1 angegebenen Bedeutungen haben, oder eines Salzes davon, dadurch gekennzeichnet, dass man

a) eine Verbindung der Formel

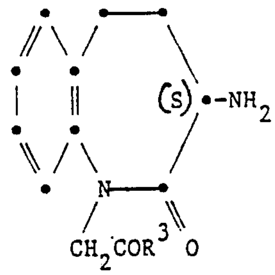

(II)

als S-Enantiomer oder Mischung von Stereoisomeren, die dieses Enantiomer enthält, worin $R^3$ die vorstehend angegebenen Bedeutungen besitzt, mit einer Verbindung der Formel

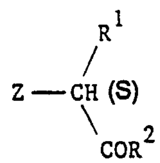

(IIIA)

als S-Enantiomer oder Mischung von Stereoisomeren, die dieses Enantiomer enthält, worin Z eine reaktive veresterte Hydroxygruppe bedeutet und $R^1$ und $R^2$ die vorstehend angegebenen Bedeutungen besitzen, oder mit einer Verbindung der Formel

$$R^1—CO—COR^2 \quad (IV)$$

worin $R^1$ und $R^2$ die oben angegebenen Bedeutungen haben, in Gegenwart eines Reduktionsmittels, unter vorübergehendem Schutz der Aminogruppe und/oder von Hydroxygruppen, die in irgendeinem der Reste $R^1$, $R^2$ und $R^3$ anwesend sein können, alkyliert oder

b) eine Verbindung der Formel

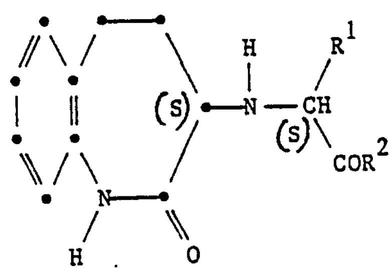

(V)

oder eine Mischung von Stereoisomeren, die diese Verbindung enthält, worin S, $R^1$ und $R^2$ die vorstehend angegebenen Bedeutungen besitzen, mit einer Verbindung der Formel

$$Z—CH_2COR^3 \quad (IIIB),$$

worin Z eine reaktive veresterte Hydroxygruppe bedeutet und $R^3$ die vorstehend angegebenen Bedeutungen besitzt, alkyliert, wobei man vorzugsweise vorübergehend die Aminogruppe und/oder Hydroxygruppen, die in irgendeinem der Reaktanten anwesend sein können, schützt, oder

c) eine Verbindung der Formel

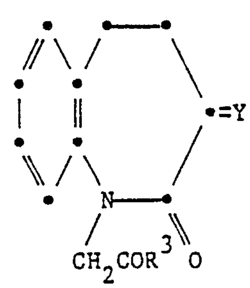

(VI)

worin Y Oxo, eine reaktive veresterte oder veretherte Hydroxygruppe Z gemeinsam mit Wasserstoff oder zwei reaktive veresterte oder veretherte Hydroxygruppen Z bedeutet und $R^3$ die vorstehend angegebenen Bedeutungen besitzt, mit einem Amin der Formel

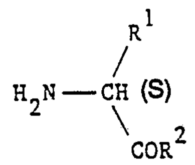

(VII)

als S-Enantiomer oder Mischung von Stereoisomeren, die dieses Enantiomer enthält, worin $R^1$ und $R^2$ die vorstehend angegebenen Bedeutungen besitzen, kondensiert, wobei, wenn Y Oxo oder zwei veresterte oder veretherte Hydroxygruppen bedeutet, die Kondensation in Gegenwart eines Reduktionsmittels durchgeführt wird, oder

d) in einer Verbindung der Formel

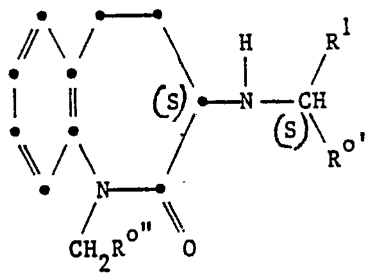

(VIII)

oder in einer Mischung von Stereoisomeren, die diese Verbindung enthält, worin S und $R^1$ die vorstehend angegebenen Bedeutungen besitzen, eines der Symbole $R^{0'}$ und $R^{0''}$ Cyano ist und das andere Cyano oder $COR^2$ bzw. $COR^3$ wie vorstehend definiert ist, die Cyanogruppe(n) einer Solvolyse unterzieht oder

e) eine Verbindung der Formel

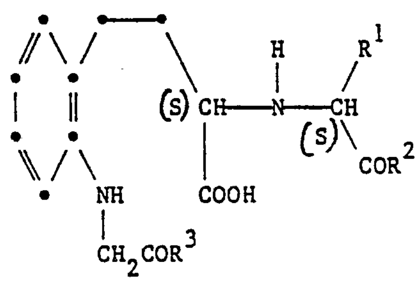

(IX)

oder eine Mischung von Stereoisomeren, die diese Verbindung Enthält, worin S, $R^1$, $R^2$ und $R^3$ die vorstehend angegebenen Bedeutungen bezitzen, oder einen reaktiven Ester hiervon cyclisiert oder

f) eine oder zwei Doppelbindungen in einer Verbindung der Formel

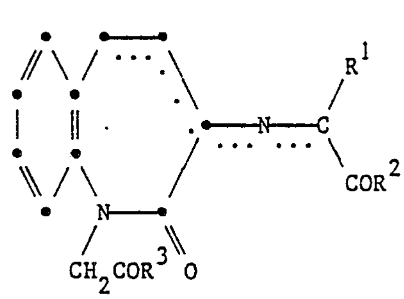

(X)

worin diese Doppelbindung(en) sich zwischen C3 und C4, C4 und C5 und/oder in der Seitenkette zwischen dem Stickstoffatom und einem benachbarten Kohlenstoffatom befinden, und worin $R^1$, $R^2$ und $R^3$ die vorstehend angegebenen Bedeutungen besitzen, durch Behandlung mit einem Reduktionsmittel sättigt oder

g) das monocyclische Lactam in einer Verbindung der Formel

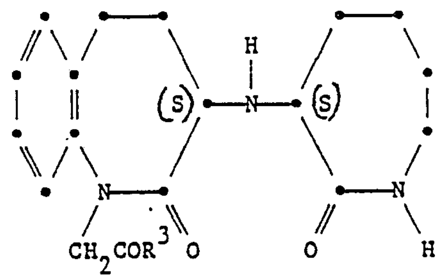

(XI)

oder in einer Mischung von Stereoisomeren, die diese Verbindung enthält, worin S und $R^3$ die vorstehend angegebenen Bedeutungen besitzen, zu einer Verbindung der Formel I, worin $R^2$ und $R^3$ Hydroxy bedeuten, ringöffnet oder

h) eine Verbindung der Formel

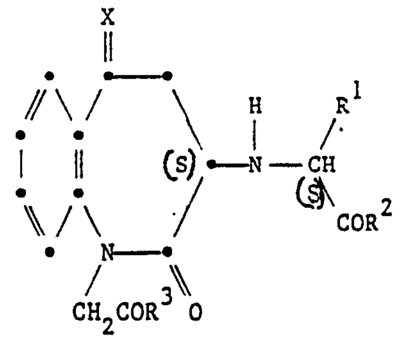

(XII)

oder eine Mischung von Stereoisomeren, die diese Verbindung enthält, worin X Oxo, geschütztes Oxo oder zwei veresterte oder veretherte Hydroxygruppen bedeutet, oder worin X Hydroxy oder verestertes oder verethertes Hydroxy zusammen mit einem Wasserstoffatom bedeutet, und worin S, $R^1$, $R^2$ und $R^3$ die vorstehend angegebenen Bedeutungen haben, hydrogenolysiert oder reduziert oder

i) eine Verbindung der Formel

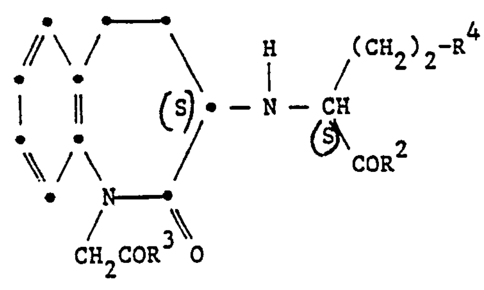

(XIII)

oder eine Mischung von Stereoisomeren, die diese Verbindung enthält, worin $R^4$ eine in 2-Aminoethyl umwandelbare Gruppe bedeutet, und $R^2$ und $R^3$ die vorstehend angegebenen Bedeutungen besitzen, in eine Verbindung der Formel I umwandelt oder

j) eine Verbindung der Formel

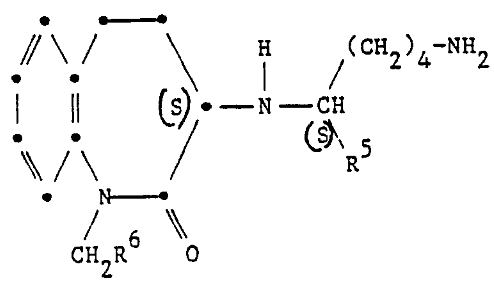

(XIV)

oder eine Mischung von Isomeren, die diese Verbindung enthält, worin $R^5$ oder $R^6$ eine in $COR^2$ bzw.

$COR^3$ umwandelbare Gruppe bedeutet, und die andere $COR^2$ bzw. $COR^3$ bedeutet, oder worin $R^5$ und $R^6$ in $COR^2$ und $COR^3$ umwandelbare Gruppen bedeuten, und worin S die vorstehend angegebene Bedeutung hat, in eine Verbindung der Formel I umwandelt oder

k) in einer Verbindung der Formel

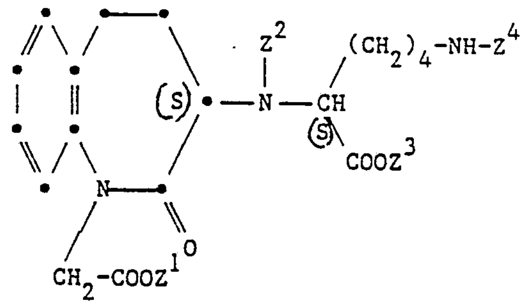

(XV)

oder einer Mischung von Stereoisomeren, die diese Verbindung enthält, worin zumindest eine der Gruppen $Z^1$ bis $Z^4$ eine Schutzgruppe, verbleibende Gruppen $Z^2$ und $Z^4$ Wasserstoff und verbleibende Gruppen $Z^1$ und $Z^3$ Wasserstoff oder Niederalkyl bedeuten, mit der Maßgabe, daß mindestens eine Gruppe von verbleibenden Gruppen $Z^1$ und $Z^3$ Wasserstoff bedeutet, und worin S die vorstehend angegebene Bedeutung hat, um eine Verbindung der Formel I zu erhalten, worin einer oder beide Reste $R^2$ und $R^3$ Hydroxy bedeuten, die Schutzgruppe(n) entfernt, und gewünschtenfalls eine erhaltene Verbindung der Formel I in eine andere Verbindung der Formel I umwandelt und/oder gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt und/oder gewünschtenfalls das optische Isomere der Formel I, das die S,S-Konfiguration im Hinblick auf die beiden Chiralitätszentren besitzt, aus einer Mischung der stereoisomeren Formen, die eine Verbindung der Formel I enthält, isoliert.

11. Die nach dem Verfahren des Anspruchs 10 erhältlichen Verbindungen.

**Patentansprüche** (für den Vertragsstaat AT)

1. Verfahren zur Herstellung einer 3-(5-Aminopentyl)-amino-1-benzazepin-2-on-1-alkansäure der allgemeinen Formel

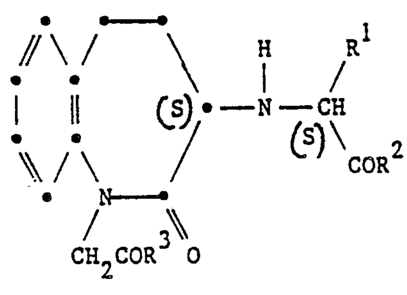

(I)

worin $R^1$ 4-Aminobutyl bedeutet, $R^2$ und $R^3$, unabhängig voneinander, Hydroxy oder Niederalkoxy bedeuten und S die Chiralität bedeutet, oder eines Salzes davon, dadurch gekennzeichnet, dass man

a) eine Verbindung der Formel

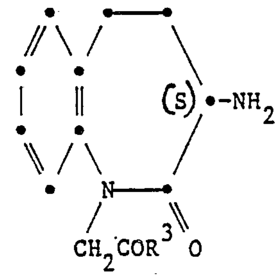

(II)

als S-Enantiomer oder Mischung von Stereoisomeren, die dieses Enantiomer enthält, worin $R^3$ die vorstehend angegebenen Bedeutungen besitzt, mit einer Verbindung der Formel

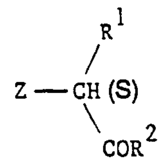

(IIIA)

als S-Enantiomer oder Mischung von Stereoisomeren, die dieses Enantiomer enthält, worin Z eine reaktive veresterte Hydroxygruppe bedeutet und $R^1$ und $R^2$ die vorstehend angegebenen Bedeutungen besitzen, oder mit einer Verbindung der Formel

$$R^1—CO—COR^2 \qquad (IV)$$

worin $R^1$ und $R^2$ die oben angegebenen Bedeutungen haben, in Gegenwart eines Reduktionsmittels, unter vorübergehendem Schutz der Aminogruppe und/oder von Hydroxygruppen, die in irgendeinem der Reste $R^1$, $R^2$ und $R^3$ anwesend sein können, alkyliert oder

b) eine Verbindung der Formel

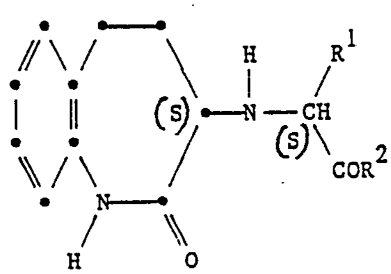

(V)

oder eine Mischung von Stereoisomeren, die diese Verbindung enthält, worin S, $R^1$ und $R^2$ die vorstehend angegebenen Bedeutungen besitzen, mit einer Verbindung der Formel

$$Z—CH_2COR^3 \qquad (IIIB),$$

worin Z eine reaktive veresterte Hydroxygruppe bedeutet und $R^3$ die vorstehend angegebenen Bedeutungen besitzt, alkyliert, wobei man vorzugsweise vorübergehend die Aminogruppe und/oder Hydroxygruppen, die in irgendeinem der Reaktanten anwesend sein können, schützt, oder

c) eine Verbindung der Formel

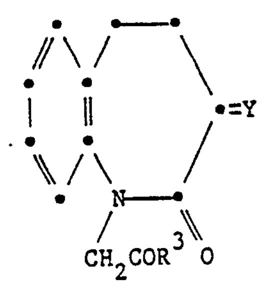

(VI)

worin Y Oxo, eine reaktive veresterte oder veretherte Hydroxygruppe Z gemeinsam mit Wasserstoff oder zwei reaktive veresterte oder veretherte Hydroxugruppen Z bedeutet und $R^3$ die vorstehend angegebenen Bedeutungen besitzt, mit einem Amin der Formel

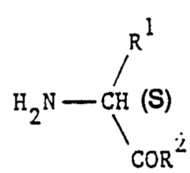

(VII)

als S-Enantiomer oder Mischung von Stereoisomeren, die dieses Enantiomer enthält, worin $R^1$ und $R^2$ die vorstehend angegebenen Bedeutungen besitzen, kondensiert, wobei, wenn Y Oxo oder zwei veresterte oder veretherte Hydroxygruppen bedeutet, die Kondensation in Gegenwart eines Reduktionsmittels durchgeführt wird, oder

d) in einer Verbindung der Formel

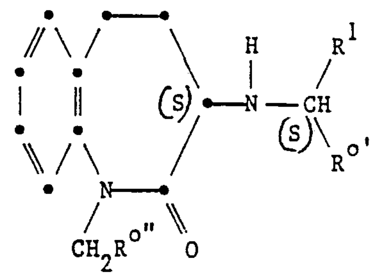

(VIII)

oder in einer Mischung von Stereoisomeren, die diese Verbindung enthält, worin S und $R^1$ die vorstehend angegebenen Bedeutungen besitzen, eines der Symbole $R^{0'}$ und $R^{0''}$ Cyano ist und das andere Cyano oder $COR^2$ bzw. $COR^3$ wie vorstehend definiert ist, die Cyanogruppe(n) einer Solvolyse unterzieht oder

e) eine Verbindung der Formel

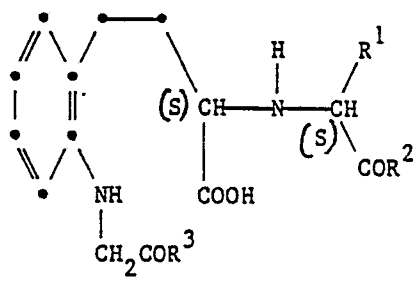

(IX)

oder eine Mischung von Stereoisomeren, die diese Verbindung enthält, worin S, $R^1$, $R^2$ und $R^3$ die vorstehend angegebenen Bedeutungen besitzen, oder einen reaktiven Ester hiervon cyclisiert oder

f) eine oder zwei Doppelbindungen in einer Verbindung der Formel

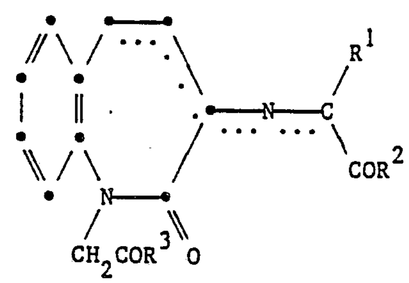

(X)

worin diese Doppelbindung(en) sich zwischen C3 und C4, C4 und C5 und/oder in der Seitenkette zwischen dem Stickstoffatom und einem benachbarten Kohlenstoffatom befinden, und worin $R^1$, $R^2$ und $R^3$ die vorstehend angegebenen Bedeutungen besitzen, durch Behandlung mit einem Reduktionsmittel sättigt oder

g) das monocyclische Lactam in einer Verbindung der Formel

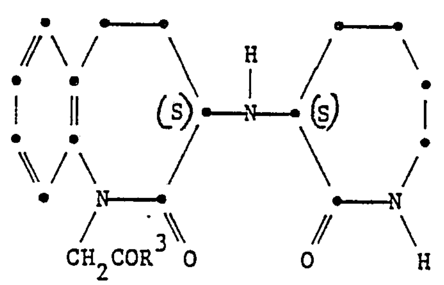

(XI)

oder in einer Mischung von Stereoisomeren, die diese Verbindung enthält, worin S und $R^3$ die vorstehend angegebenen Bedeutungen besitzen, zu einer Verbindung der Formel I, worin $R^2$ und $R^3$ Hydroxy bedeuten, ringöffnet oder

h) eine Verbindung der Formel

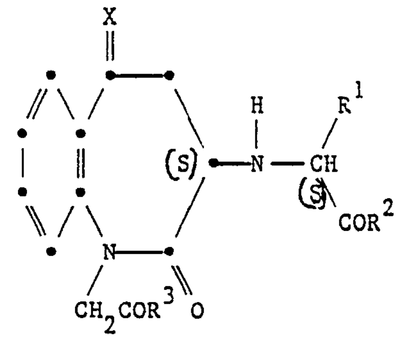

(XII)

oder eine Mischung von Stereoisomeren, die diese Verbindung enthält, worin X Oxo, geschütztes Oxo oder zwei veresterte oder veretherte Hydroxygruppen bedeutet, oder worin X Hydroxy oder verestertes oder verethertes Hydroxy zusammen mit einem Wasserstoffatom bedeutet, und worin S, $R^1$, $R^2$ und $R^3$ die vorstehend angegebenen Bedeutungen haben, hydrogenolysiert oder reduziert oder

i) eine Verbindung der Formel

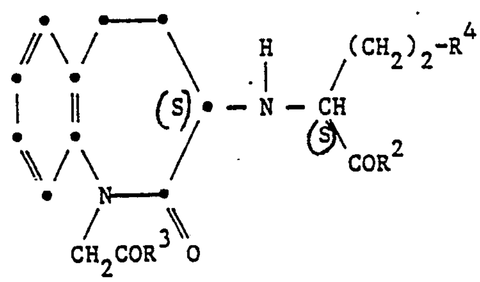

(XIII)

oder eine Mischung von Stereoisomeren, die diese Verbindung enthält, worin $R^4$ eine in 2-Aminoethyl unwandelbare Gruppe bedeutet, und $R^2$ und $R^3$ die vorstehend angegebenen Bedeutungen besitzen, in eine Verbindung der Formel I umwandelt oder

j) eine Verbindung der Formel

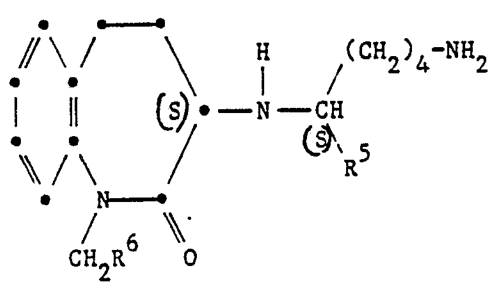

(XIV)

oder eine Mischung von Isomeren, die diese Verbindung enthält, worin $R^5$ oder $R^6$ eine in $COR^2$ bzw. $COR^3$ umwandelbare Gruppe bedeutet, und die andere $COR^2$ bzw. $COR^3$ bedeutet, oder worin $R^5$ und $R^6$ in $COR^2$ und $COR^3$ umwandelbare Gruppen bedeuten, und worin S die vorstehend angegebene Bedeutung hat, in eine Verbindung der Formel I umwandelt oder

k) in einer Verbindung der Formel

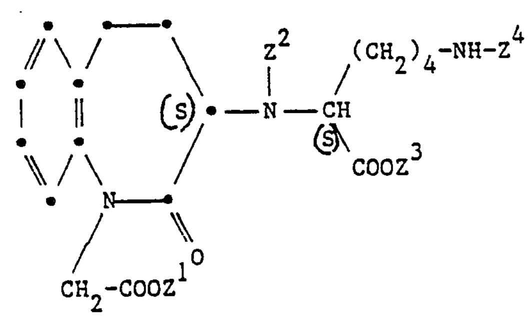

(XV)

oder einer Mischung von Stereoisomeren, die diese Verbindung enthält, worin zumindest eine der Gruppen $Z^1$ bis $Z^4$ eine Schutzgruppe, verbleibende Gruppen $Z^2$ und $Z^4$ Wasserstoff und verbleibende Gruppen $Z^1$ und $Z^3$ Wasserstoff oder Niederalkyl bedeuten, mit der Maßgabe, daß mindestens eine

Gruppe von verbleibenden Gruppen $Z^1$ und $Z^3$ Wasserstoff bedeutet, und worin S die vorstehend angegebene Bedeutung hat, um eine Verbindung der Formel I zu erhalten, worin einer oder beide Reste $R^2$ und $R^3$ Hydroxy bedeuten, die Schutzgruppe(n) entfernt, und gewünschtenfalls eine erhaltene Verbindung der Formel I in eine andere Verbindung der Formel I umwandelt und/oder gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt und/oder gewünschtenfalls das optische Isomere der Formel I, das die S,S-Konfiguration im Hinblick auf die beiden Chiralitätszentren besitzt, aus einer Mischung der stereoisomeren Formen, die eine Verbindung der Formel I enthält, isoliert.

2. Verfahren nach Anspruch 1, dadurch gekennzeichnet, dass man eine Verbindung der Formel I, worin $R^1$ 4-Aminobutyl bedeutet, und $R^2$ und $R^3$ unabhängig voneinander Hydroxy, Methoxy, Ethoxy oder tert.-Butoxy bedeuten, oder ein Salz davon herstellt.

3. Verfahren nach Anspruch 1, dadurch gekennzeichnet, dass man eine Verbindung der Formel I, worin $R^1$ 4-Aminobutyl bedeutet, $R^2$ Hydroxy, Methoxy oder tert.-Butoxy und $R^3$ Hydroxy oder Ethoxy bedeuten, oder ein Salz davon herstellt.

4. Verfahren nach .Anspruch 1, dadurch gekennzeichnet, dass man 3-[(5-Amino-1-carboxy)-(1S)-pentylamino]-1-carboxymethyl-2,3,4,5-tetrahydro-1H-1-(3S)-benzazepin-2-on oder ein Salz davon herstellt.

5. Verfahren nach Anspruch 1, dadurch gekennzeichnet, dass man das Hydrochlorid oder das Dihydrochlorid von 3-[(5-Amino-1-carboxy)-(1S)-pentylamino]-1-carboxymethyl-2,3,4,5-tetrahydro-1H-1-(3S)-benzazepin-2-on herstellt.

6. Verfahren zur Herstellung einer der in Anspruch 1 genannten Verbindungen der Formel I, in der alle Symbole die in Anspruch 1 angegebenen Bedeutungen haben, oder eines Salzes davon, dadurch gekennzeichnet, dass man

a) eine Verbindung der Formel

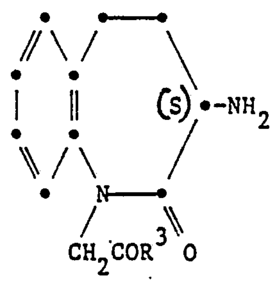

(II)

als S-Enantiomer oder Mischung von Stereoisomeren, die dieses Enantiomer enthält, worin $R^3$ die vorstehend angegebenen Bedeutungen besitzt, mit einer Verbindung der Formel

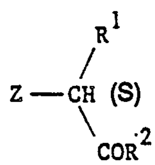

(IIIA)

als S-Enantiomer oder Mischung von Stereoisomeren, die dieses Enantiomer enthält, worin Z eine reaktive veresterte Hydroxygruppe bedeutet und $R^1$ und $R^2$ die vorstehend angegebenen Bedeutungen besitzen, oder mit einer Verbindung der Formel

$$R^1—CO—COR^2 \quad (IV)$$

worin $R^1$ und $R^2$ die oben angegebenen Bedeutungen haben, in Gegenwart eines Reduktionsmittels, unter vorübergehendem Schutz der Aminogruppe und/oder von Hydroxygruppen, die in irgendeinem der Reste $R^1$, $R^2$ und $R^3$ anwesend sein können, alkyliert oder

b) eine Verbindung der Formel

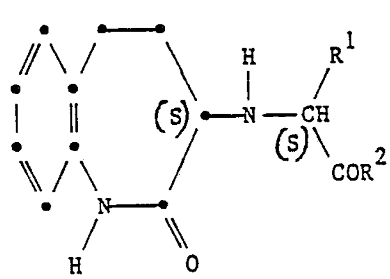

(V)

oder eine Mischung von Stereoisomeren, die diese Verbindung enthält, worin S, $R^1$ und $R^2$ die vorstehend angegebenen Bedeutungen besitzen, mit einer Verbindung der Formel

$$Z{-}CH_2COR^3 \qquad \text{(IIIB)},$$

worin Z eine reaktive veresterte Hydroxygruppe bedeutet und $R^3$ die vorstehend angegebenen Bedeutungen besitzt, alkyliert, wobei man vorzugsweise vorübergehend die Aminogruppe und/oder Hydroxygruppen, die in irgendeinem der Reaktanten anwesend sein können, schützt, oder

c) eine Verbindung der Formel

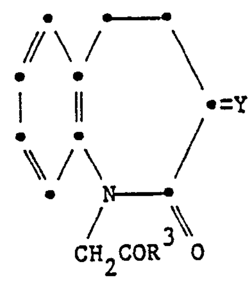

(VI)

worin Y Oxo, eine reaktive veresterte oder veretherte Hydroxygruppe Z gemeinsam mit Wasserstoff oder zwei reaktive veresterte oder veretherte Hydroxygruppen Z bedeutet und $R^3$ die vorstehend angegebenen Bedeutungen besitzt, mit einem Amin der Formel

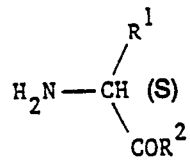

(VII)

als S-Enantiomer oder Mischung von Stereoisomeren, die dieses Enantiomer enthält, worin $R^1$ und $R^2$ die vorstehend angegebenen Bedeutungen besitzen, kondensiert, wobei, wenn Y Oxo oder zwei veresterte oder veretherte Hydroxygruppen bedeutet, die Kondensation in Gegenwart eines Reduktionsmittels durchgeführt wird, oder

d) in einer Verbindung der Formel

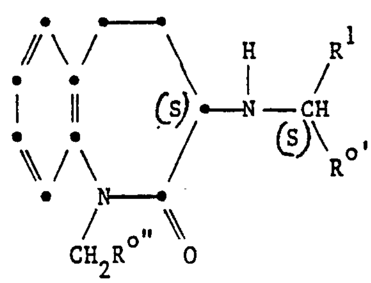

(VIII)

oder in einer Mischung von Stereoisomeren, die diese Verbindung enthält, worin S und $R^1$ die vorstehend angegebenen Bedeutungen besitzen, eines der Symbole $R^{0'}$ und $R^{0''}$ Cyano ist und das andere Cyano oder $COR^2$ bzw. $COR^3$ wie vorstehend definiert ist, die Cyanogruppe(n) einer Solvolyse unterzieht oder

e) eine Verbindung der Formel

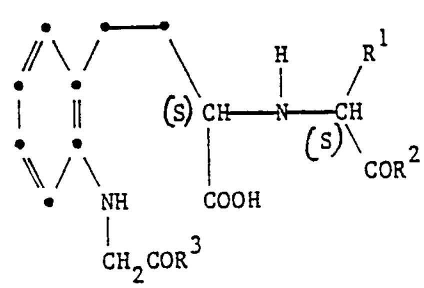

(IX)

oder eine Mischung von Stereoisomeren, die diese Verbindung enthält, worin S, $R^1$, $R^2$ und $R^3$ die vorstehend angegebenen Bedeutungen besitzen, oder einen reaktiven Ester hiervon cyclisiert oder

f) eine Verbindung, die strukturell identisch mit einer Verbindung der Formel I ist, ausser dass sie eine zusätzliche Doppelbindung am Kohlenstoffatom C-3 oder in der Seitenkette zwischen dem Stickstoffatom und dem benachbarten exocyclischen Kohlenstoffatom aufweist, mit einem Reduktionsmittel behandelt, um diese Doppelbindung zu sättigen, und gewünschtenfalls eine erhaltene Verbindung der Formel I in eine andere Verbindung der Formel I umwandelt und/oder gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt und/oder gewünschtenfalls das optische Isomere der Formel I, das die S,S-Konfiguration im Hinblick auf die beiden Chiralitätszentren besitzt, aus einer Mischung der stereoisomeren Formen, die eine Verbindung der Formel I enthält, isoliert.

7. Verfahren nach Anspruch 6, dadurch gekennzeichnet, dass man eine Verbindung des Anspruchs 2 oder ein Salz davon herstellt.

8. Verfahren nach Anspruch 6, dadurch gekennzeichnet, dass man eine Verbindung des Anspruchs 3 oder ein Salz davon herstellt.

9. Verfahren nach Anspruch 6, dadurch gekennzeichnet, dass man 3-[(5-Amino-1-carboxy)-(1S)-pentylamino]-1-carboxymethyl-2,3,4,5-tetrahydro-1H-1-(3S)-benzazepin-2-on oder ein Salz davon herstellt.

10. Verfahren zur Herstellung eines pharmazeutischen Präparates, gekennzeichnet durch die Verarbeitung eines nach einem der Ansprüche 1 bis 5 erfindungsgemäß hergestellten Wirkstoffs mit einem pharmazeutischen Trägermaterial.

11. Verfahren zur Herstellung eines pharmazeutischen Präparates, gekennzeichnet durch die Verarbeitung eines nach einem der Ansprüche 6 bis 9 erfindungsgemäß hergestellten Wirkstoffs mit einem pharmazeutischen Trägermaterial.

**Claims** (for the Contracting States : BE, CH, DE, FR, GB, IT, LI, LU, NL, SE)

1. Compounds of the general formula

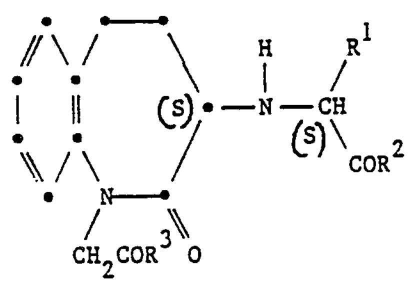

(I)

wherein $R^1$ represents 4-aminobutyl, $R^2$ and $R^3$ represent, independently of each other, hydroxy or lower alkoxy, and S represents the chirality, or salts thereof.

2. Compounds of the formula I shown in claim 1, wherein $R^1$ represents 4-aminobutyl, and $R^2$ and $R^3$ represent, independently of each other, hydroxy, methoxy, ethoxy or tert.-butoxy, or salts thereof.

3. Compounds of the formula I shown in claim 1, wherein $R^1$ represents 4-aminobutyl, $R^2$ represents hydroxy, methoxy or tert.-butoxy, and $R^3$ represents hydroxy or ethoxy, or salts thereof.

4. 3-[(5-amino-1-carboxy)-(1S)-pentylamino]-1-carboxymethyl-2,3,4,5-tetrahydro-1H-1-(3S)-benzazepin-2-on or the salts thereof.

5. The hydrochloride and the dihydrochloride of 3-[(5-amino-1-carboxy)-(1S)-pentylamino]-1-carboxymethyl-2,3,4,5-tetrahydro-1H-1-(3S)-benzazepin-2-one.

6. Pharmaceutical preparations containing a compound according to any one of claims 1 to 5 or a pharmaceutically acceptable salt thereof, together with a pharmaceutically suitable carrier.

7. A compound mentioned in any one of claims 1 to 5 or a pharmaceutically acceptable salt thereof for use in a method for the therapeutic treatment of the human or animal body.

8. A compound mentioned in any one of claims 1 to 5 or a pharmaceutically acceptable salt thereof as an inhibitor of the angiotensin-converting enzyme.

9. The use of one of the compounds mentioned in claims 1 to 5 or a pharmaceutically acceptable salt thereof for the production of pharmaceutical preparations.

10. A process for the preparation of a compound of the formula I mentioned in claim 1, in which all the symbols have the meanings given in claim 1, or a salt thereof, characterised in that

a) a compound of the formula

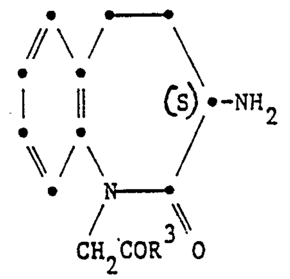

(II)

as the S-enantiomer or mixture of stereoisomers containing said enantiomer, wherein $R^3$ has the meanings given above, is alkylated with a compound of the formula

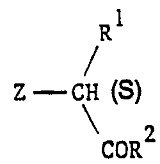

(IIIA)

as the S-enantiomer or mixture of stereoisomers containing said enantiomer, wherein Z is a reactive esterified hydroxy group and $R^1$ and $R^2$ have the meanings given above, or with a compound of the formula

$$R^1—CO—COR^2 \quad (IV),$$

wherein $R^1$ and $R^2$ have the meanings given above, in the presence of a reducing agent, with the temporary protection of the amino group and/or of hydroxy groups that may be present in any one of the radicals $R^1$, $R^2$ and $R^3$, or

b) a compound of the formula

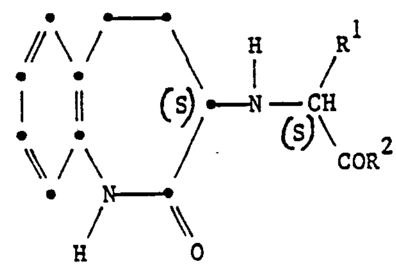

(V)

or a mixture of stereoisomers containing said compound, wherein S, $R^1$ and $R^2$ have the meanings given above, is alkylated with a compound of the formula

$$Z—CH_2COR^3 \quad (IIIB),$$

wherein Z is a reactive esterified hydroxy group and $R^3$ has the meanings given above, preferably with the temporary protection of the amino group and/or of hydroxy groups that may be present in any one of the reactants, or

c) a compound of the formula

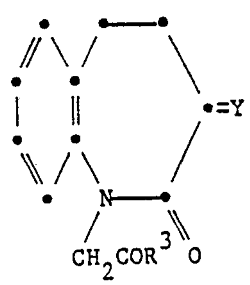

(VI)

wherein Y is oxo, a reactive esterified or etherified hydroxy group Z together with hydrogen or two reactive esterified or etherified hydroxy groups Z, and $R^3$ has the meanings given above, is condensed with an amine of the formula

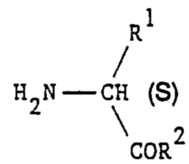

(VII)

as the S-enantiomer or mixture of stereoisomers containing said enantiomer, wherein $R^1$ and $R^2$ have the meanings given above, with the proviso that when Y is oxo or two esterified or etherified hydroxy groups, the condensation is carried out in the presence of a reducing agent, or

d) in a compound of the formula

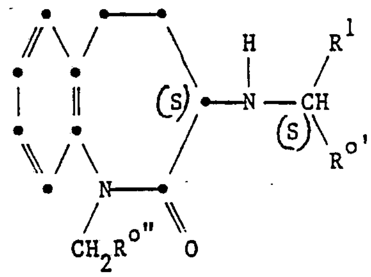

(VIII)

or in a mixture of stereoisomers containing said compound, wherein S and $R^1$ have the meanings given above, one of the symbols $R^{0'}$ and $R^{0''}$ is cyano and the other is cyano or $COR^2$ or, as appropriate, $COR^3$ as defined above, the cyano group(s) is(are) subjected to solvolysis, or

e) a compound of the formula

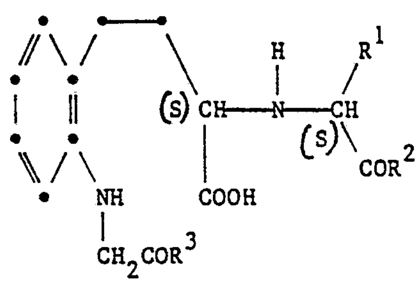

(IX)

or a mixture of stereoisomers containing said compound, wherein S, $R^1$, $R^2$ and $R^3$ have the meanings given above, or a reactive ester thereof, is cyclised, or

f) one or two double bonds in a compound of the formula

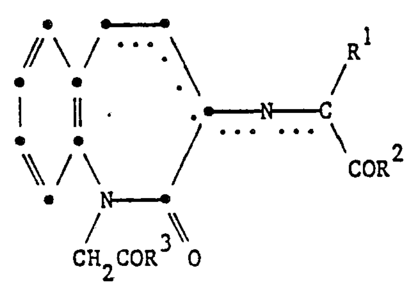

(X)

wherein said double bond(s) is(are) located between C3 and C4, C4 and C5 and/or in the side-chain between the nitrogen atom and an adjacent carbon atom, and wherein $R^1$, $R^2$ and $R^3$ have the meanings given above, is(are) satured by treatment with a reducing agent, or

g) the monocyclic lactam in a compound of the formula

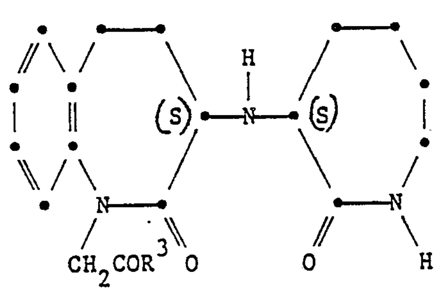

(XI)

or in a mixture of stereoisomers containing said compound, wherein S and $R^3$ have the meanings given above, is ringopened to give a compound of formula I wherein $R^2$ and $R^3$ are hydroxy, or

h) a compound of the formula

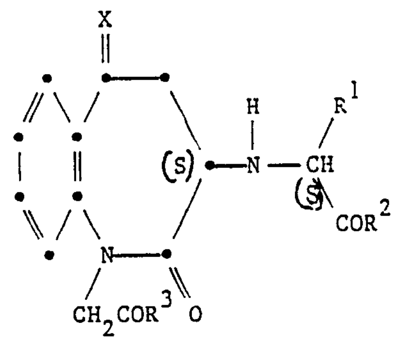

(XII)

or a mixture of stereoisomers containing said compound, wherein X is oxo, protected oxo or two esterified or etherified hydroxy groups, or wherein X is hydroxy or esterified or etherified hydroxy together with a hydrogen atom, and wherein S, $R^1$, $R^2$ and $R^3$ have the meanings given above, is hydrogenolysed or reduced, or

i) a compound of the formula

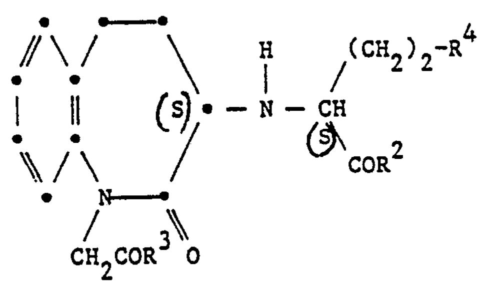

(XIII)

or a mixture of stereoisomers containing said compound, wherein $R^4$ is a group convertible into 2-aminoethyl, and $R^2$ and $R^3$ have the meanings given above, is converted into a compound of formula I, or

j) a compound of the formula

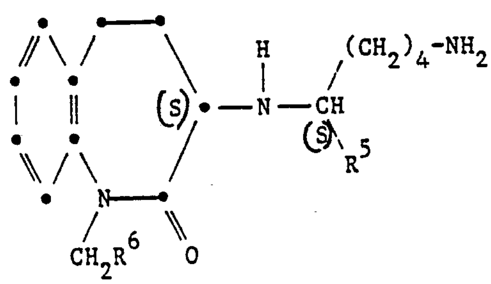

(XIV)

or a mixture of isomers containing said compound, wherein $R^5$ or $R^6$ is a group convertible into $COR^2$ or $COR^3$, respectively, and the other is $COR^2$ or $COR^3$ as appropriate, or wherein $R^5$ and $R^6$ are groups convertible into $COR^2$ and $COR^3$, and wherein S has the meaning given above, is converted into a compound of the formula I, or

k) in a compound of the formula

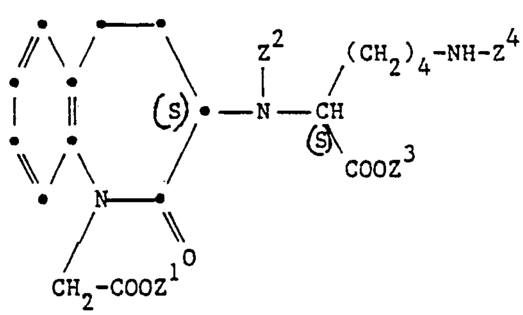

(XV)

or in a mixture of stereoisomers containing said compound, wherein at least one of the groups $Z^1$ to $Z^4$ is a protective group, remaining groups $Z^2$ and $Z^4$ are hydrogen and remaining groups $Z^1$ and $Z^3$ are hydrogen or lower alkyl, with the proviso that at least one group of the remaining groups $Z^1$ and $Z^3$ is hydrogen, and wherein S has the meaning given above, in order to obtain a compound of formula I wherein one or both of the radicals $R^2$ and $R^3$ is(are) hydroxy, the protective group(s) is(are) removed and, if desired, a resulting compound of formula I is converted into another compound of formula I and/or, if desired, a resulting compound of formula I having salt-forming properties is converted into a salt thereof or a resulting salt is converted into another salt, or a free compound is liberated from such a salt, and/or, if desired, the optical isomer of formula I that has the S,S configuration with respect to the two centres of chirality is isolated from a mixture of stereoisomeric forms containing a compound of formula I.

11. The compounds obtainable by the process of claim 10.

**Claims** (for Contracting State AT)

1. A process for the preparation of a 3-(5-aminopentyl)-amino-1-benzazepin-2-one-1-alkanoic acid of the general formula

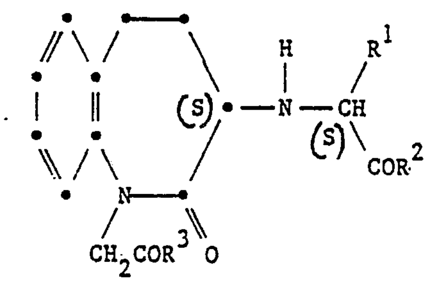

(I)

wherein $R^1$ represents 4-aminobutyl, $R^2$ and $R^3$ represent, independently of each other, hydroxy or lower alkoxy, and S represents the chirality, or a salt thereof, characterised in that

a) a compound of the formula

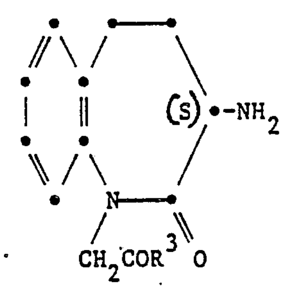

(II)

as the S-enantiomer or mixture of stereoisomers containing said enantiomer, wherein $R^3$ has the meanings given above, is alkylated with a compound of the formula

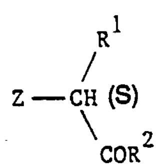

(IIIA)

as the S-enantiomer or mixture of stereoisomers containing said enantiomer, wherein Z is a reactive esterified hydroxy group and $R^1$ and $R^2$ have the meanings given above, or with a compound of the formula

$$R^1—CO—COR^2 \quad (IV),$$

wherein $R^1$ and $R^2$ have the meanings given above, in the presence of a reducing agent, with the temporary protection of the amino group and/or of hydroxy groups that may be present in any one of the radicals $R^1$, $R^2$ and $R^3$, or

b) a compound of the formula

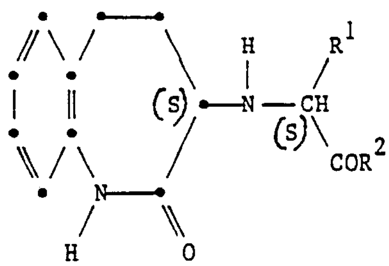

(V)

or a mixture of stereoisomers containing said compound, wherein S, $R^1$ and $R^2$ have the meanings given above, is alkylated with a compound of the formula

$$Z—CH_2COR^3 \qquad (IIIB),$$

wherein Z is a reactive esterified hydroxy group and $R^3$ has the meanings given above, preferably with the temporary protection of the amino group and/or of hydroxy groups that may be present in any one of the reactants, or

c) a compound of the formula

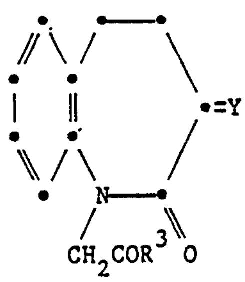

(VI)

wherein Y is oxo, a reactive esterified or etherified hydroxy group Z together with hydrogen or two reactive esterified or etherified hydroxy groups Z, and $R^3$ has the meanings given above, is condensed with an amine of the formula

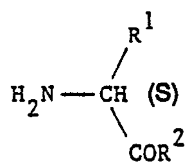

(VII)

as the S-enantiomer or mixture of stereoisomers containing said enantiomer, wherein $R^1$ and $R^2$ have the meanings given above, with the proviso that when Y is oxo or two esterified or etherified hydroxy groups, the condensation is carried out in the presence of a reducing agent, or

d) in a compound of the formula

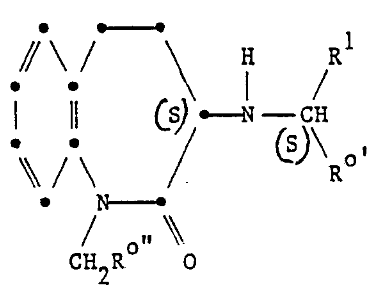

(VIII)

or in a mixture of stereoisomers containing said compound, wherein S and $R^1$ have the meanings given above, one of the symbols $R^{0'}$ and $R^{0''}$ is cyano and the other is cyano or $COR^2$ or, as appropriate, $COR^3$ as defined above, the cyano group(s) is(are) subjected to solvolysis, or

e) a compound of the formula

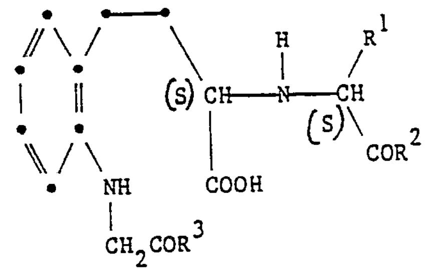

(IX)

or a mixture of stereoisomers containing said compound, wherein S, $R^1$, $R^2$ and $R^3$ have the meanings given above, or a reactive ester thereof, is cyclised, or

f) one or two double bonds in a compound of the formula

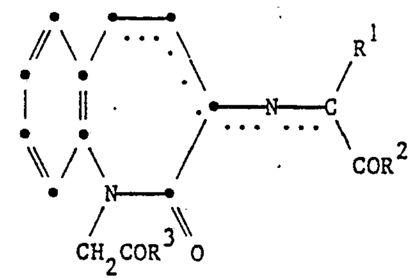

(X)

wherein said double bond(s) is(are) located between C3 and C4, C4 and C5 and/or in the side-chain between the nitrogen atom and an adjacent carbon atom, and wherein $R^1$, $R^2$ and $R^3$ have the meanings given above, is(are) saturated by treatment with a reducing agent, or

g) the monocyclic lactam in a compound of the formula

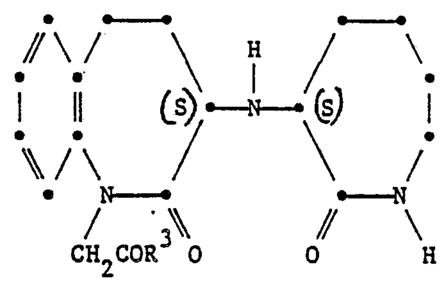

(XI)

or in a mixture of stereoisomers containing said compound, wherein S and $R^3$ have the meanings given above, is ringopened to give a compound of formula I wherein $R^2$ and $R^3$ are hydroxy, or

h) a compound of the formula

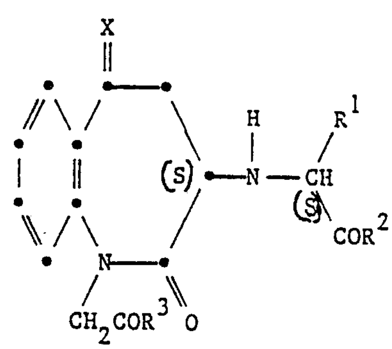

(XII)

or a mixture of stereoisomers containing said compound, wherein X is oxo, protected oxo or two esterified or etherified hydroxy groups, or wherein X is hydroxy or esterified or etherified hydroxy together with a hydrogen atom, and wherein S, $R^1$, $R^2$ and $R^3$ have the meanings given above, is hydrogenolysed or reduced, or

i) a compound of the formula

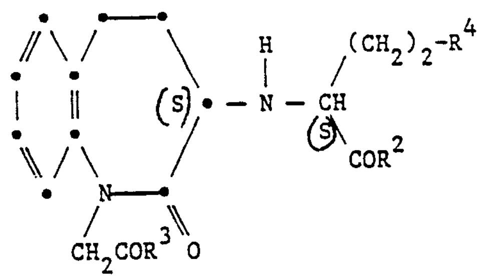

(XIII)

or a mixture of stereoisomers containing said compound, wherein $R^4$ is a group convertible into 2-aminoethyl, and $R^2$ and $R^3$ have the meanings given above, is converted into a compound of formula I, or

j) a compound of the formula

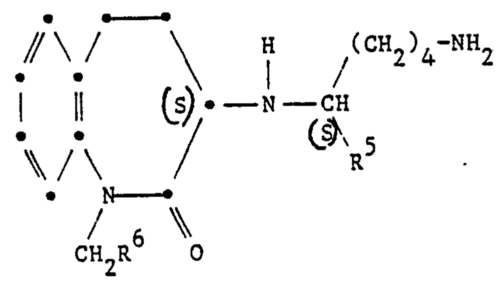

(XIV)

or a mixture of isomers containing said compound, wherein $R^5$ or $R^6$ is a group convertible into $COR^2$ or $COR^3$, respectively, and the other is $COR^2$ or $COR^3$ as appropriate, or wherein $R^5$ and $R^6$ are groups convertible into $COR^2$ and $COR^3$, and wherein S has the meaning given above, is converted into a compound of the formula I, or

k) in a compound of the formula

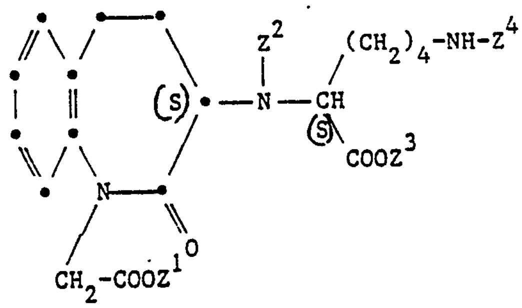

(XV)

or in a mixture of stereoisomers containing said compound, wherein at least one of the groups $Z^1$ to $Z^4$ is a protective group, remaining groups $Z^2$ and $Z^4$ are hydrogen and remaining groups $Z^1$ and $Z^3$ are hydrogen or lower alkyl, with the proviso that at least one group of the remaining groups $Z^1$ and $Z^3$ is hydrogen, and wherein S has the meaning given above, in order to obtain a compound of formula I wherein one or both of the radicals $R^2$ and $R^3$ is(are) hydroxy, the protective group(s) is(are) removed and, if desired, a resulting compound of formula I is converted into another compound of formula I and/or, if desired, a resulting compound of formula I having salt-forming properties is converted into a salt thereof or a resulting salt is converted into another salt, or a free compound is liberated from such a salt, and/or, if desired, the optical isomer of formula I that has the S,S configuration with respect to the two centres of chirality is isolated from a mixture of stereoisomeric forms containing a compound of formula I.

2. A process according to claim 1, characterised in that a compound of formula I wherein $R^1$ represents 4-aminobutyl, and $R^2$ and $R^3$ represent, independently of each other, hydroxy, methoxy, ethoxy or tert.-butoxy, or a salt thereof, is prepared.

3. A process according to claim 1, characterised in that a compound of formula I wherein $R^1$ represents 4-aminobutyl, $R^2$ represents hydroxy, methoxy or tert.-butoxy, and $R^3$ represents hydroxy or ethoxy, or a salt thereof, is prepared.

4. A process according to claim 1, characterised in that 3-[(5-amino-1-carboxy)-(1S)-pentylamino]-1-carboxymethyl-2,3,4,5-tetrahydro-1H-1-(3S)-benzazepin-2-one or a salt thereof is prepared.

5. A process according to claim 1, characterised in that the hydrochloride or the dihydrochloride of

3-[(5-amino-1-carboxy)-(1S)-pentylamino]-1-carboxymethyl-2,3,4,5-tetrahydro-1H-1-(3S)-benzazepin-2-one is prepared.

6. A process for the preparation of one of the compounds of formula I mentioned in claim 1, in which all the symbols have the meanings given in claim 1, or a salt thereof, characterised in that

a) a compound of the formula

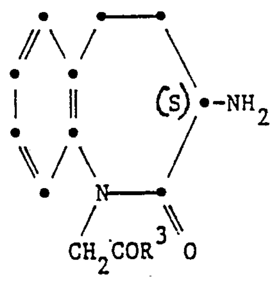

(II)

as the S-enantiomer or mixture of stereoisomers containing said enantiomer, wherein $R^3$ has the meanings given above, is alkylated with a compound of the formula

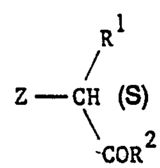

(IIIA)

as the S-enantiomer or mixture of stereoisomers containing said enantiomer, wherein Z is a reactive esterified hydroxy group and $R^1$ and $R^2$ have the meanings given above, or with a compound of the formula

$$R^1—CO—COR^2 \quad \text{(IV)},$$

wherein $R^1$ and $R^2$ have the meanings given above, in the presence of a reducing agent, with the temporary protection of the amino group and/or of hydroxy that may be present in any one of the radicals $R^1$, $R^2$ and $R^3$, or

b) a compound of the formula

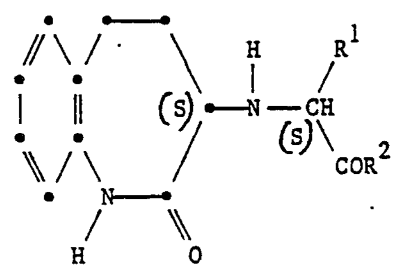

(V)

or a mixture of stereoisomers containing said compound, wherein S, $R^1$ and $R^2$ have the meanings given above, is alkylated with a compound of the formula

$$Z—CH_2COR^3 \quad \text{(IIIB)},$$

wherein Z is a reactive esterified hydroxy group and $R^3$ has the meanings given above, preferably with the temporary protection of the amino group and/or of hydroxy groups that may be present in any one of the reactants, or

c) a compound of the formula

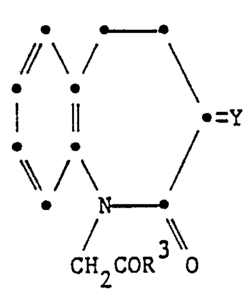

(VI)

wherein Y is oxo, a reactive esterified or etherified hydroxy group Z together with hydrogen or two reactive esterified or etherified hydroxy groups Z, and $R^3$ has the meanings given above, is condensed with an amine of the formula

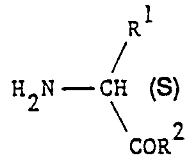

(VII)

as the S-enantiomer or mixture of stereoisomers containing said enantiomer, wherein $R^1$ and $R^2$ have the meanings given above, with the proviso that when Y is oxo or two esterified or etherified hydroxy groups, the condensation is carried out in the presence of a reducing agent, or

d) in a compound of the formula

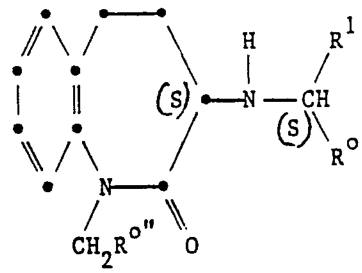

(VIII)

or in a mixture of stereoisomers containing said compound, wherein S and $R^1$ have the meanings given above, one of the symbols $R^{o'}$ and $R^{o''}$ is cyano and the other is cyano or $COR^2$ or, as appropriate, $COR^3$ as defined above, the cyano group(s) is(are) subjected to solvolysis, or

e) a compound of the formula

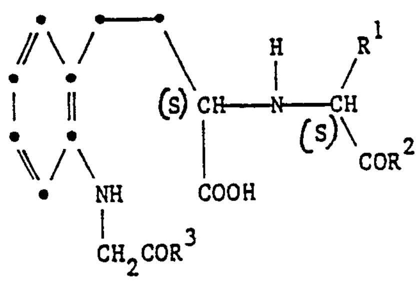

(IX)

or a mixture of stereoisomers containing said compound, wherein S, R1, $R^2$ and $R^3$ have the meanings given above, or a reactive ester thereof, is cyclised, or

f) a compound that is structurally identical with a compound of formula I, except that it contains an additional double bond at the C3 carbon atom or in the side-chain between the nitrogen atom and the adjacent exocyclic carbon atom, is treated with a reducing agent in order to saturate said double bond, and, if desired, a resulting compound of formula I is converted into another compound of formula I and/or, if desired, a resulting compound of formula I having salt-forming properties is converted into a salt thereof or a resulting salt is converted into another salt, or a free compound is liberated from such a salt, and/or, if desired, the optical isomer of formula I that has the S,S configuration with respect ot the two centres of chirality is isolated from a mixture of stereoisomeric forms containing a compound of formula I.

7. A process according to claim 6, characterised in that a compound of claim 2 or a salt thereof is prepared.

8. A process according to claim 6, characterised in that a compound of claim 3 or a salt thereof is prepared.

9. A process according to claim 6, characterised in that 3-[(5-amino-1-carboxy)-(1S)-pentylamino]-1-carboxymethyl-2,3,4,5-tetrahydro-1H-1-(3S)-benzazepin-2-one or a salt thereof is prepared.

10. A process for the production of a pharmaceutical preparation, characterised by processing an active ingredient prepared in accordance with the invention according to any one of claims 1 to 5 with a pharmaceutical carrier.

11. A process for the production of a pharmaceutical preparation, characterised by processing an active ingredient prepared in accordance with the invention according to any one of claims 6 to 9 with a pharmaceutical carrier.

**Revendications** (pour les Etats contractants : BE, CH, DE, FR, GB, IT, LI, LU, NL, SE)

1. Composés de formule générale

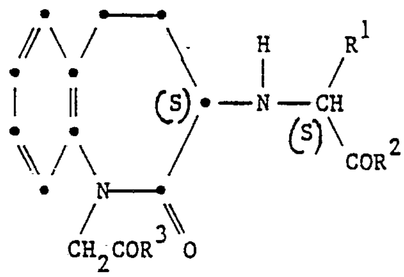

(I)

dans laquelle $R^1$ représente un groupe 4-aminobutyle, $R^2$ et $R^3$ représentent chacun, indépendamment l'un de l'autre, un groupe hydroxy ou alcoxy inférieur et S indique la chiralité, ou leurs sels.

2. Composés de formule I de la revendication 1, dans lesquels $R^1$ représente un groupe 4-aminobutyle et $R^2$ et $R^3$ représentent chacun, indépendamment l'un de l'autre, un groupe hydroxy, méthoxy, éthoxy ou tert-butoxy, ou leurs sels..

3. Composés de formule I de la revendication 1, dans lesquels $R^1$ représente un groupe 4-aminobutyle, $R^2$ représente un groupe hydroxy, méthoxy ou tert-butoxy et $R^3$ un groupe hydroxy ou éthoxy, ou leurs sels.

4. La 3-[(5-amino-1-carboxy)-(1S)-pentylamino]-1-carboxyméthyl-2,3,4,5-tétrahydro-1H-1-(3S)-benzazépine-2-one ou ses sels.

5. Le chlorhydrate et le dichlorhydrate de la 3-[(5-amino-1-carboxy)-(1S)-pentylamino]-1-carboxyméthyl-2,3,4,5-tétrahydro-1H-1-(3S)-benzazépine-2-one.

6. Compositions pharmaceutiques contenant un composé selon l'une des revendications 1 à 5, ou un sel acceptable pour l'usage pharmaceutique de ce composé avec un véhicule approprié à l'usage pharmaceutique.

7. Un composé mentionné dans l'une des revendications 1 à 5, ou un sel d'un tel composé acceptable pour l'usage pharmaceutique, pour l'utilisation dans un procédé pour le traitement thérapeutique de l'organisme humain ou animal.

8. Un composé mentionné dans l'une des revendications 1 à 5, ou un sel acceptable pour l'usage pharmaceutique d'un tel composé, en tant qu'inhibiteur de l'enzyme convertissant l'angiotensine.

9. Utilisation d'un des composés mentionnés dans les revendications 1 à 5, ou d'un sel acceptable pour l'usage pharmaceutique d'un tel composé, pour la préparation de compositions pharmaceutiques.

10. Procédé de préparation d'un composé mentionné dans la revendication 1, et répondant à la formule I dans laquelle tous les symboles ont les significations indiquées dans la revendication 1, ou d'un sel d'un tel composé, caractérisé en ce que

a) on alkyle un composé de formule

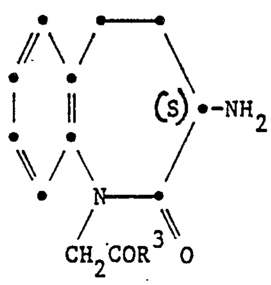

(II)

à l'état d'énantiomères S ou de mélange de stéréoisomères contenant cet énantiomère, $R^3$ ayant les significations indiquées ci-dessus, à l'aide d'un composé de formule

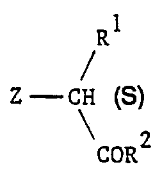

(IIIA)

à l'état d'énantiomère S ou de mélanges de stéréoisomères contenant cet énantiomère, Z représentant un groupe hydroxy estérifié réactif et $R^1$ et $R^2$ ayant les significations indiquées ci-dessus, ou à l'aide d'un composé de formule

$R^1—CO—COR^2$ (IV)

dans laquelle $R^1$ et $R^2$ ont les significations indiquées ci-dessus, en présence d'un agent réducteur et en protégeant transitoirement le groupe amino et/ou les groupes hydroxy éventuellement présents dans l'un quelconque des groupes $R^1$, $R^2$ et $R^3$, ou bien

b) on alkyle un composé de formule

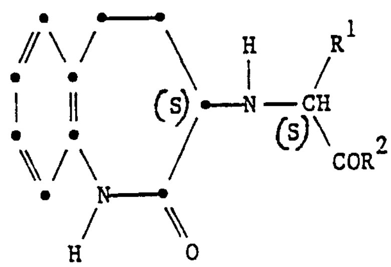

(V)

ou un mélange de stéréoisomères contenant ce composé, S, $R^1$ et $R^2$ ayant les significations indiquées ci-dessus, à l'aide d'un composé de formule

$Z—CH_2COR^3$ (IIIB)

Z représentant un groupe hydroxy estérifié réactif et $R^3$ ayant les significations indiquées ci-dessus, de préférence en protégeant provisoirement le groupe amino et/ou les groupes hydroxy éventuellement présents dans l'un quelconque des réactifs, ou bien

c) on condense un composé de formule

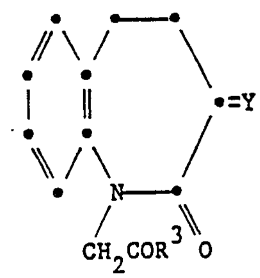

(VI)

dans laquelle Y représente un groupe oxo, un groupe hydroxy estérifié ou éthérifié réactif Z et l'hydrogène ou deux groupes hydroxy estérifiés ou éthérifiés réactifs Z et $R^3$ a les significations indiquées ci-dessus, avec une amine de formule

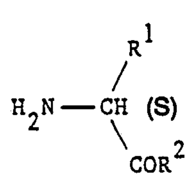

(VII)

à l'état d'énantiomère S ou de mélange de stéréoisomères contenant cet énantiomère, $R^1$ et $R^2$ ayant les significations indiquées ci-dessus, en effectuant la condensation en présence d'un agent réducteur lorsque Y représente un groupe oxo ou deux groupes hydroxy estérifiés ou éthérifiés, ou bien

d) dans un composé de formule

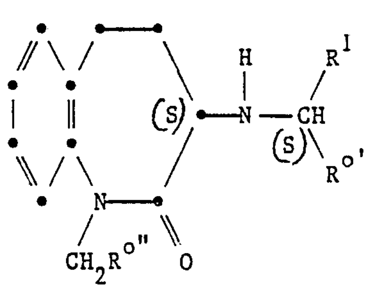

(VIII)

ou un mélange de stéréoisomères contenant ce composé, S et $R^1$ ayant les significations indiquées ci-dessus, l'un des symboles $R^{0'}$ et $R^{0''}$ représentant un groupe cyano et l'autre un groupe cyano ou $COR^2$ ou $COR^3$ tels que définis ci-dessus, on soumet le ou les groupes cyano à une solvolyse ; ou bien

e) on cyclise un composé de formule

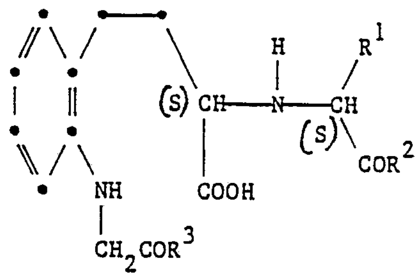

(IX)

ou un mélange de stéréoisomères contenant ce composé, S, $R^1$, $R^2$ et $R^3$ ayant les significations indiquées ci-dessus, ou un ester réactif d'un tel composé, ou bien

f) on sature une ou deux doubles liaisons d'un composé de formule

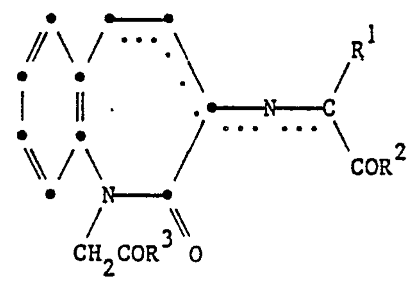

(X)

dans lequel cette ou ces doubles liaisons se trouvent entre les carbones C3 et C4, C4 et C5 et/ou dans la chaîne latérale entre l'atome d'azote et un atome de carbone voisin, $R^1$, $R^2$ et $R^3$ ayant les significations indiquées ci-dessus, par traitement à l'aide d'un agent réducteur, ou bien

g) on ouvre le cycle lactame monocyclique d'un composé de formule

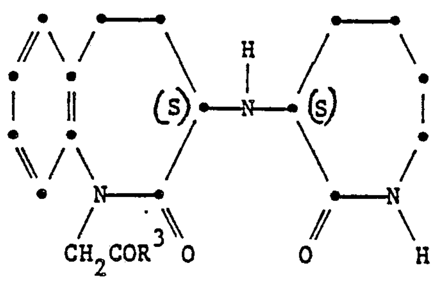

(XI)

ou d'un mélange de stéréoisomères contenant ce composé, S et $R^3$ ayant les significations indiquées ci-dessus, ce qui donne un composé de formule I dans laquelle $R^2$ et $R^3$ représentent des groupes hydroxy, ou bien

h) on hydrogénolyse ou on réduit un composé de formule

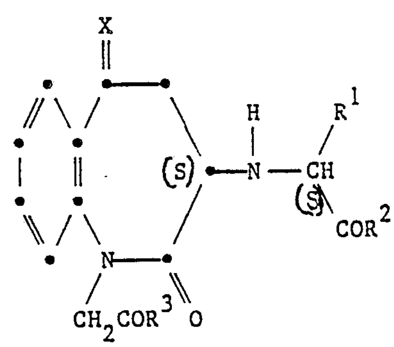

(XII)

ou un mélange de stéréoisomères contenant ce composé, X représentant un groupe oxo, un groupe oxo protégé ou deux groupes hydroxy estérifiés ou éthérifiés, ou bien X représente un groupe hydroxy, un groupe hydroxy estérifié ou éthérifié et un atome d'hydrogène, et S, $R^1$, $R^2$ et $R^3$ ayant les significations indiquées ci-dessus, ou bien

i) on convertit en un composé de formule I un composé de formule

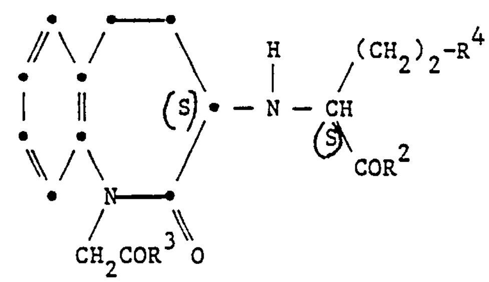

(XIII)

ou un mélange de stéréoisomères contenant ce composé, $R^4$ représentant un groupe convertible en un groupe 2-aminoéthyle et $R^2$ et $R^3$ ayant les significations indiquées ci-dessus, ou bien

j) on convertit en un composé de formule I un composé de formule

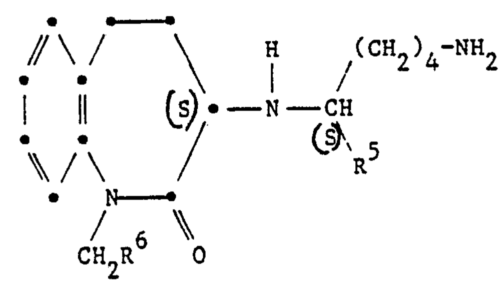

(XIV)

ou un mélange d'isomères contenant ce composé, $R^5$ ou $R^6$ représentant un groupe convertible en un groupe $COR^2$ ou $COR^3$ et l'autre de ces symboles représentant un groupe $COR^2$ ou $COR^3$, ou bien $R^5$ et $R^6$ représentant des groupes convertibles en $COR^2$ ou $COR^3$ et S a les significations indiquées ci-dessus, ou bien

k) dans un composé de formule

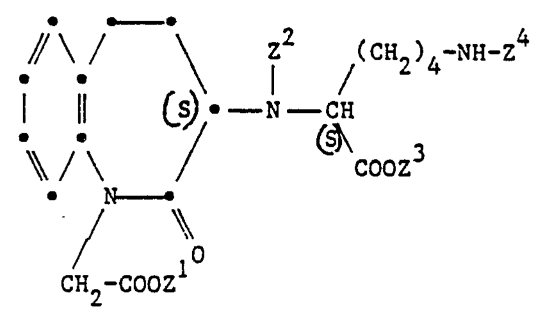

(XV)

ou dans un mélange de stéréoisomères contenant ce composé, dans lequel au moins un des symboles $Z^1$ à $Z^4$ représente un groupe protecteur, les groupes $Z^2$ et $Z^4$ restants représentant l'hydrogène et les groupes $Z^1$ et $Z^3$ restants représentant l'hydrogène ou des groupes alkyle inférieurs, étant spécifié que l'un au moins des symboles $Z^1$ et $Z^3$ restants représente l'hydrogène, et S ayant les significations indiquées ci-dessus, afin d'obtenir un composé de formule I dans lequel l'un des symboles $R^2$ et $R^3$ ou les deux représentent des groupes hydroxy, on élimine le ou les groupes protecteurs, et, si on le désire, on convertit un composé obtenu répondant à la formule I en un autre composé de formule I et/ou si on le désire, on convertit un composé obtenu répondant à la formule I et salifiable en un sel ou un sel obtenu en un autre sel ou on libère un composé à partir d'un tel sel et/ou si on le désire, on isole à partir d'un mélange de stéréoisomères contenant un composé de formule I l'isomère optique de formule I présentant la configuration S,S par rapport aux deux centres de chiralité.

11. Les composés obtenus par le procédé de la revendication 10.

**Revendications** (pour l'Etat contractant AT)

1. Procédé de préparation d'un acide 3-(5-aminopentyl)-amino-1-benzazépine-2-one-1-alcanoïque de formule générale

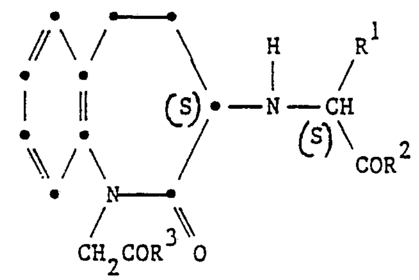

(I)

dans laquelle $R^1$ représente un groupe 4-aminobutyle, $R^2$ et $R^3$ représentent chacun, indépendamment l'un de l'autre, un groupe hydroxy ou alcoxy inférieur et S indique la chiralité, ou d'un sel d'un tel composé, caractérisé en ce que :

a) on alkyle un composé de formule

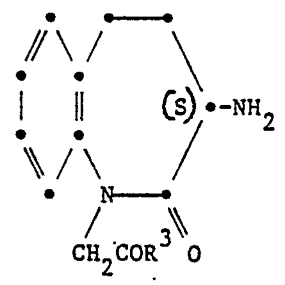

(II)

à l'état d'énantiomère S ou de mélange de stéréoisomères contenant cet énantiomère, $R^3$ ayant les significations indiquées ci-dessus, à l'aide d'un composé de formule

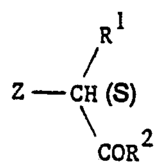

(IIIA)

à l'état d'énantiomère S ou de mélange de stéréoisomères contenant cet énantiomère, Z représentant un groupe hydroxy estérifié réactif et $R^1$ et $R^2$ ayant les significations indiquées ci-dessus, ou à l'aide d'un composé de formule

$$R^1—CO—COR^2 \quad (IV)$$

dans laquelle $R^1$ et $R^2$ ont les significations indiquées ci-dessus, en présence d'un agent réducteur, en protégeant transitoirement le groupe amino et/ou les groupes hydroxy éventuellement présents dans l'un quelconque des groupes $R^1$, $R^2$ et $R^3$, ou bien

b) on alkyle un composé de formule

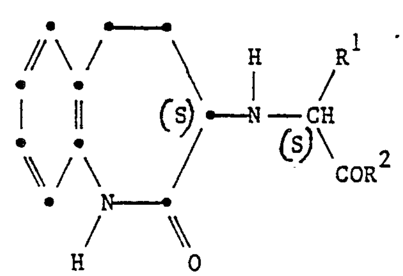

(V)

ou un mélange de stéréoisomères contenant ce composé, S, $R^1$ et $R^2$ ayant les significations indiquées ci-dessus, à l'aide d'un composé de formule

$$Z—CH_2COR^3 \qquad (IIIB),$$

Z représentant un groupe hydroxy estérifié réactif et $R^3$ ayant les significations indiquées ci-dessus, de préférence en protégeant transitoirement le groupe amino et/ou les groupes hydroxy éventuellement présents dans l'un quelconque des réactifs, ou bien

c) on condense un composé de formule

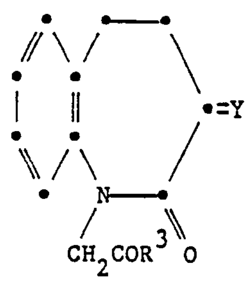

(VI)

dans laquelle Y représente un groupe oxo, un groupe hydroxy estérifié ou éthérifié réactif Z et un atome d'hydrogène ou deux groupes hydroxy estérifiés ou éthérifiés réactifs Z et $R^3$ a les significations indiquées ci-dessus, avec une amine de formule

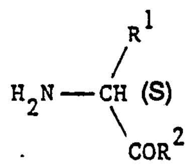

(VII)

à l'état d'énantiomères S ou de mélange de stéréoisomères contenant cet énantiomère, $R^1$ et $R^2$ ayant les significations indiquées ci-dessus, en opérant en présence d'un agent réducteur lorsque Y représente un groupe oxo ou deux groupes hydroxy estérifiés ou éthérifiés, ou bien

d) dans un composé de formule

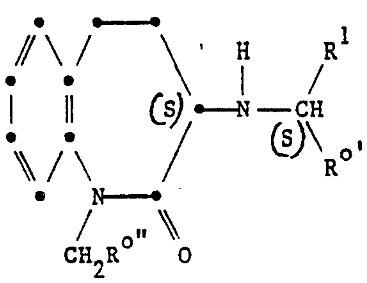

(VIII)

ou dans un mélange de stéréoisomères contenant ce composé, S et $R^1$ ayant les significations indiquées ci-dessus et l'un des symboles $R^{0'}$ et $R^{0''}$ représentant un groupe cyano et l'autre un groupe cyano ou $COR^2$ ou $COR^3$ tels que définis ci-dessus, on soumet le ou les groupes cyano à une solvolyse, ou bien

e) on cyclise un composé de formule

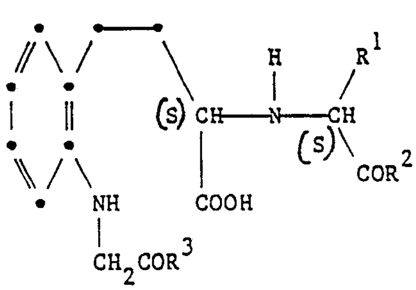

(IX)

ou un mélange de stéréoisomères contenant ce composé, dans lequel S, $R^1$, $R^2$ et $R^3$ ont les significations indiquées ci-dessus, ou un ester réactif d'un tel composé, ou bien

f) on sature une ou deux doubles liaisons d'un composé de formule

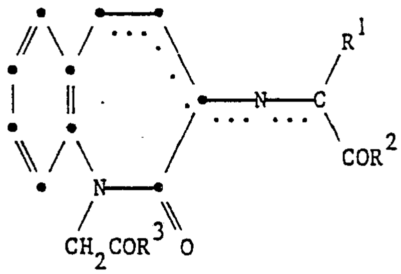

(X)

dans lequel cette ou ces doubles liaisons se trouvent entre les carbones C3 et C4, C4 et C5 et/ou dans la chaîne latérale entre l'atome d'azote et un atome de carbone voisin, $R^1$, $R^2$ et $R^3$ ayant les significations indiquées ci-dessus, par traitement à l'aide d'un agent réducteur, ou bien

g) on ouvre le cycle lactame monocyclique d'un composé de formule

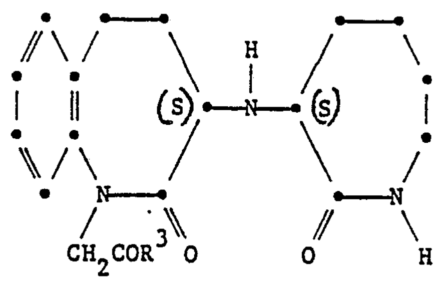

(XI)

ou d'un mélange de stéréoisomères contenant ce composé, S et $R^3$ ayant les significations indiquées ci-dessus, ce qui donne un composé de formule I dans laquelle $R^2$ et $R^3$ représentent des groupes hydroxy, ou bien

h) on hydrogénolyse ou on réduit un composé de formule

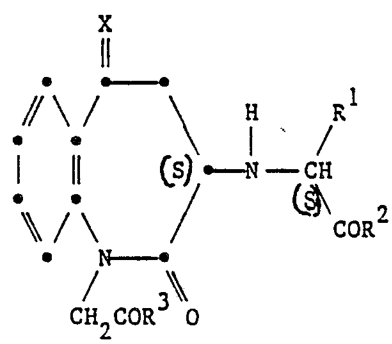

(XII)

ou un mélange de stéréoisomères contenant ce composé, dans lequel X représente un groupe oxo, un groupe oxo protégé ou deux groupes hydroxy estérifiés ou éthérifiés, ou bien X représente un groupe hydroxy ou un groupe hydroxy estérifié ou éthérifié et un atome d'hydrogène, et S, $R^1$, $R^2$ et $R^3$ ont les significations indiquées ci-dessus, ou bien

i) on convertit un composé de formule

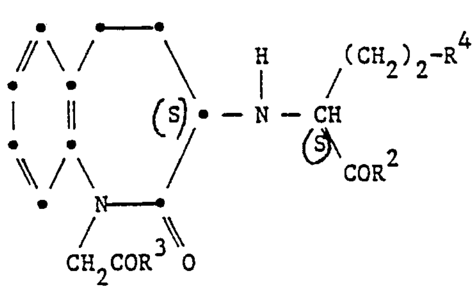

(XIII)

ou un mélange de stéréoisomères contenant ce composé, $R^4$ représentant un groupe convertible en groupe 2-aminoéthyle et $R^2$ et $R^3$ ayant les significations indiquées ci-dessus, en un composé de formule I, ou bien

j) on convertit un composé de formule

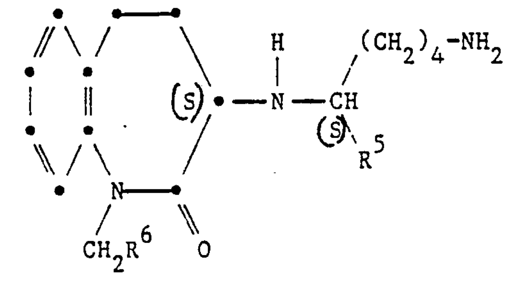

(XIV)

ou un mélange d'isomères contenant ce composé, $R^5$ ou $R^6$ représentant un groupe convertible en un groupe $COR^2$ ou $COR^3$ et l'autre de ces symboles représentant un groupe $COR^2$ ou $COR^3$, et $R^5$ et $R^6$ représentent des groupes convertibles en groupes $COR^2$ et $COR^3$, S ayant les significations indiquées ci-dessus, en un composé de formule I, ou bien

k) dans un composé de formule

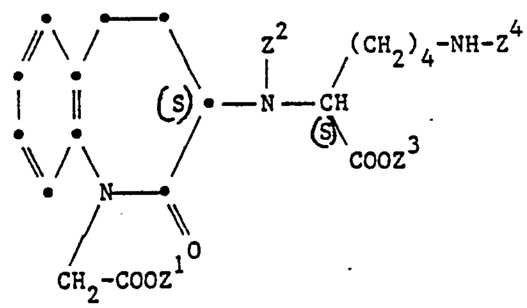

(XV)

ou un mélange de stéréoisomères contenant ce composé, dans lequel l'un au moins des symboles $Z^1$ à $Z^4$ représente un groupe protecteur, les symboles $Z^2$ et $Z^4$ restants représentant l'hydrogène et les symboles $Z^1$ et $Z^3$ restants représentant l'hydrogène ou des groupes alkyle inférieurs, étant spécifié que l'un au moins des symboles restants $Z^1$ et $Z^3$ représente l'hydrogène, S ayant les significations indiquées ci-dessus, en vue d'obtenir un composé de formule I dans lequel un ou deux des symboles $R^2$ et $R^3$ représentent un groupe hydroxy, on élimine le ou les groupes protecteurs et si on le désire, on convertit un composé obtenu répondant à la formule I en un autre composé de formule I et/ou si on le désire, on convertit un composé obtenu répondant à la formule I et salifiable en un sel ou un sel obtenu en un autre sel ou bien on libère un composé à partir d'un tel sel et/ou si on le désire, on isole l'isomère optique de formule I présentant la configuration S,S par rapport aux deux centres de chiralité à partir d'un mélange des formes stéréoisomères contenant un composé de formule I.

2. Procédé selon la revendication 1, caractérisé en ce que l'on prépare un composé de formule I dans laquelle $R^1$ représente un groupe 4-aminobutyle et $R^2$ et $R^3$ représentent chacun, indépendamment l'un de l'autre, un groupe hydroxy, méthoxy, éthoxy ou tert-butoxy, ou un sel d'un tel composé.

3. Procédé selon la revendication 1, caractérisé en ce que l'on prépare un composé de formule I dans laquelle $R^1$ représente un groupe 4-aminobutyle, $R^2$ représente un groupe hydroxy, méthoxy ou tert-butoxy et $R^3$ représente un groupe hydroxy ou éthoxy, ou un sel d'un tel composé.

4. Procédé selon la revendication 1, caractérisé en ce que l'on prépare la 3-[(5-amino-1-carboxy)-(1S)-pentylamino]-1-carboxyméthyl-2,3,4,5-tétrahydro-1H-1-(3S)-benzazépine-2-one, ou un sel de ce composé.

5. Procédé selon la revendication 1, caractérisé en ce que l'on prépare le chlorhydrate ou le dichlorhydrate de la 3-[(5-amino-1-carboxy)-(1S)-pentylamino]-1-carboxyméthyl-2,3,4,5-tétrahydro-1H-1-(3S)-benzazépine-2-one.

6. Procédé de préparation de l'un des composés mentionnés dans la revendication 1, répondant à la formule I dans laquelle tous les symboles ont les significations indiquées dans la revendication 1, ou d'un sel d'un tel composé, caractérisé en ce que :

a) on alkyle un composé de formule

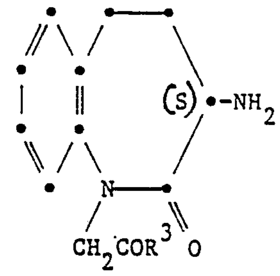

(II)

à l'état d'énantiomère S ou de mélange de stéréoisomères contenant cet énantiomère, $R^3$ ayant les significations indiquées ci-dessus, à l'aide d'un composé de formule

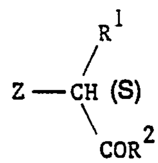

(IIIA)

à l'état d'énantiomère S ou de mélange de stéréoisomères contenant cet énantiomère, Z représentant un groupe hydroxy estérifié réactif et $R^1$ et $R^2$ ayant les significations indiquées ci-dessus, ou à l'aide d'un composé de formule

$$R^1—CO—COR^2 \qquad (IV)$$

dans laquelle $R^1$ et $R^2$ ont les significations indiquées ci-dessus, en présence d'un agent réducteur, en protégeant transitoirement le groupe amino et/ou les groupes hydroxy éventuellement présents dans l'un quelconque des groupes R1, $R^2$ et $R^3$, ou bien

b) on alkyle un composé de formule

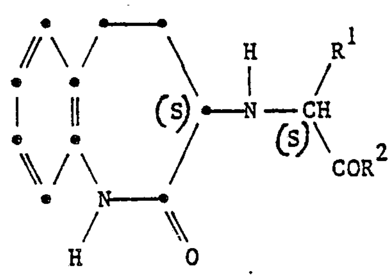

(V)

ou un mélange de stéréoisomères contenant ce composé, Z, $R^1$ et $R^2$ ayant les significations indiquées ci-dessus, à l'aide d'un composé de formule

$$Z—CH_2COR^3 \qquad (IIIB)$$

dans laquelle Z représente un groupe hydroxy estérifié réactif et $R^3$ a les significations indiquées ci-dessus, de préférence en protégeant transitoirement le groupe amino et/ou les groupes hydroxy éventuellement présents dans l'un quelconque des réactifs, ou bien

c) on condense un composé de formule

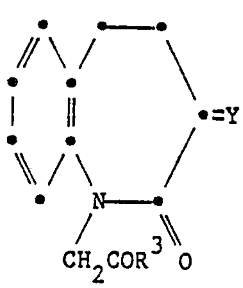

(VI)

dans laquelle Y représente un groupe oxo, un groupe hydroxy estérifié ou éthérifié réactif Z et un atome d'hydrogène ou deux groupes hydroxy estérifiés ou éthérifiés réactifs Z et $R^3$ a les significations indiquées ci-dessus, avec une amine de formule

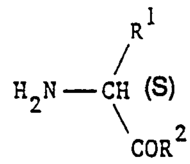

(VII)

à l'état d'énantiomère S ou de mélange de stéréoisomères contenant cet énantiomère, dans laquelle $R^1$ et $R^2$ ont les significations indiquées ci-dessus, en effectuant la condensation en présence d'un agent réducteur lorsque Y représente un groupe oxo ou deux groupes hydroxy estérifiés ou éthérifiés, ou bien

d) dans un composé de formule

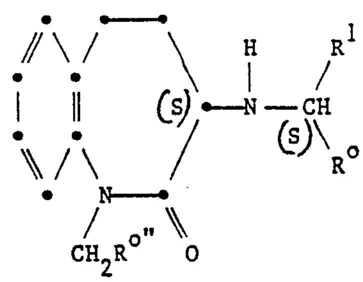

(VIII)

ou dans un mélange de stéréoisomères contenant ce composé, S et $R^1$ ayant les significations indiquées ci-dessus, l'un des symboles $R^{0'}$ et $R^{0''}$ représentant un groupe cyano et l'autre un groupe cyano ou $COR^2$ ou $COR^3$ tels que définis ci-dessus, on soumet le ou les groupes cyano à solvolyse, ou bien

e) on cyclise un composé de formule

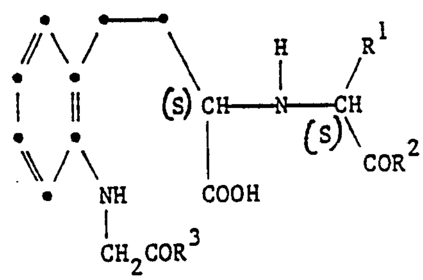

(IX)

ou un mélange de stéréoisomères contenant ce composé, S, $R^1$, $R^2$ et $R^3$ ayant les significations indiquées ci-dessus, ou un ester réactif d'un tel composé, ou bien

f) partant d'un composé dont la structure est identique à celle d'un composé de formule I sauf qu'il contient une double liaison supplémentaire sur l'atome de carbone C3 ou bien dans la chaîne latérale entre l'atome d'azote et l'atome de carbone exocyclique voisin, on traite ce composé par un agent réducteur afin de saturer la double liaison, et si on le désire, on convertit un composé obtenu répondant à la formule I en un autre composé de formule I et/ou si on le désire, on convertit un composé obtenu répondant à la formule I et salifiable en un sel ou un sel obtenu en un autre sel ou on libère un composé à partir d'un tel sel et/ou si on le désire, on isole l'isomère optique de formule I présentant la configuration S,S par rapport aux deux centres de chiralité à partir d'un mélange de formes stéréoisomères contenant un composé de formule I.

7. Procédé selon la revendication 6, caractérisé en ce que l'on prépare un composé de la revendication 2 ou un sel d'un tel composé.

8. Procédé selon la revendication 6, caractérisé en ce que l'on prépare un composé de la revendication 3 ou un sel d'un tel composé.

9. Procédé selon la revendication 6, caractérisé en ce que l'on prépare la 3-[(5-amino-1-carboxy)-(1S)-pentylamino]-1-carboxyméthyl-2,3,4,5-tétrahydro-1H-1-(3S)-benzazépine-2-one ou un sel de ce composé.

10. Procédé de préparation d'une composition pharmaceutique, caractérisé en ce que l'on combine une substance active préparée : conformément à l'invention selon l'une des revendications 1 à 5 avec un véhicule pharmaceutique.

11. Procédé de préparation d'une composition pharmaceutique, caractérisé en ce que l'on combine une substance active préparée conformément à l'invention selon l'une des revendications 6 à 9 avec un véhicule pharmaceutique.